(19) Europäisches Patentamt
European Patent Office
Office européen des brevets

(11) EP 2 930 238 A1

(12) EUROPEAN PATENT APPLICATION

(43) Date of publication:
**14.10.2015 Bulletin 2015/42**

(21) Application number: **14382136.1**

(22) Date of filing: **09.04.2014**

(51) Int Cl.:
***C12N 9/14*** *(2006.01)* ***C12N 15/55*** *(2006.01)*
***C12N 15/113*** *(2010.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Fundacio Centre de Regulacio Genomica**
**08003 Barcelona (ES)**

(72) Inventors:
- **Beato, Miguel**
  **E-08005 Barcelona (ES)**
- **Wright, Roni**
  **E-08003 Barcelona (ES)**

(74) Representative: **ABG Patentes, S.L.**
**Avenida de Burgos, 16D**
**Edificio Euromor**
**28036 Madrid (ES)**

(54) **Compositions for treating cancer**

(57) The present invention relates to a Nudix5 inhibitor for use in the treatment and/or prevention of cancer in a subject and pharmaceutical compositions comprising a therapeutically effective amount of a Nudix5 inhibitor and a pharmaceutically acceptable excipient or carrier, and to a pharmaceutical composition comprising a therapeutically effective amount of a composition comprising a Nudix5 inhibitor and at least a second antitumor agent and a pharmaceutically acceptable excipient or carrier. Furthermore, the invention relates to a method for assaying the activity of Nudix5 comprising assaying the formation of ATP, and to a method for identifying a compound potentially useful for treating and/or preventing cancer comprising assaying the activity of Nudix5 in the presence of said compound.

EP 2 930 238 A1

## Description

### TECHNICAL FIELD OF INVENTION

The present invention belongs to the field of cancer treatment and is related to compounds for their use in the treatment of cancer, in particular Nudix5 inhibitors.

### BACKGROUND OF INVENTION

Cancer is the second cause of death worldwide. In the case of breast cancer, epidemiological studies point to more than one million new cases diagnosed per year and an annual mortality rate close to 450,000 deaths.

Cancer cells proliferate at a high rate and accumulate a large number of DNA lesions, leading to extensive changes in gene expression that require a global modification of chromatin, involving several kinases, histone modifying enzymes and multiple ATP-dependent chromatin remodelers. Thus, cancer cells have a high requirement for ATP.

The cell nucleus requires an immense amount of energy to replicate or to repair their genome and to reprogram gene expression during differentiation or in response to external cues. Extensive changes in chromatin compaction and nucleosome organization are required to make genes accessible for regulatory proteins involved in these processes, including transcription factors and the basal transcriptional machinery. First the chromatin fiber must be decondensed by ill-defined mechanisms that include loosening the binding of linker histone H1 to DNA and core histones, a process that requires post-translational modifications and ATP-dependent remodeling enzymes. This is followed by mobilization of nucleosomes to allow transcription initiation and elongation. It has been calculated that to reposition by sliding a single nucleosome core particle would imply breaking and reforming hundreds of interactions between positively charged amino acids in core histones and negatively charged phosphates in the DNA backbone. A complete round of remodeling of nucleosome core particles assembled *in vitro* requires the hydrolysis of 1000 ATP molecules by the ATPases/DNA-translocase domains that fuel the activity of dedicated ATP dependent chromatin remodeling complexes. This is because not each remodeling step is effective in exposing the cleavage site for the restriction enzyme used in this assay. In the nucleus of living cells the energy cost may be different, as the chromatin fiber is folded and compacted linker histones.

Thus, reducing the levels of ATP has been suggested as a therapeutic approach for treating cancer. For example US2010/0190845A1 proposes the administration of aurovertin B, an ATP synthase inhibitor, for treating cancer. WZB 117 is an inhibitor of GLUT1 that decreases glucose uptake and intracellular ATP. Exogenous ATP rescues growth of WZB117-treated cancer cells, suggesting that reduction of ATP is an important mechanism of WZB117's anticancer effect (Cao X. Cancer Chemotherapy Pharmacol 2007; 59: 495-505).

Inhibition of LDHA (lactate dehydrogenase A) by siRNA or FX11 treatment reduces ATP levels and results in cancer cell death (Le A. et al., Proc Natl Acad Sci USA 2010; 107: 2037-2042).

Despite the promising results obtained with some drugs, the therapeutic targets mentioned above pose important therapeutic issues such as resistance, selectivity, toxicity and side effects. Among these side effects are oral mucosa function and integrity disorders. Consequences include serious ulceration (mucositis) and fungal superinfection of the mouth (candidiasis, thrush). These complications induced by the disease and its treatments involve pain in swallowing, dysphagia, malnutrition, delays in chemotherapy administration, interruptions in the radiotherapy scheme, loss of effectiveness of oncological treatments, prolonged hospital stays, elevated costs and in some patients, potentially deadly infections (sepsis). Some of the side effects may be due to the fact that these drugs blocked cellular ATP production in general, and are not selective for the nuclear ATP needs.

Therefore, there is still a need to develop new effective antitumoral agents focused on the energy requirements in the cell nucleus.

### SUMMARY OF THE INVENTION

In a first aspect, the invention relates to a Nudix5 inhibitor for use in the treatment and/or prevention of cancer in a subject.

In a second aspect, the invention relates to a pharmaceutical composition comprising a therapeutically effective amount of a Nudix5 inhibitor and a pharmaceutically acceptable excipient or carrier.

In a third aspect, the invention relates to a composition comprising a Nudix5 inhibitor and at least a second antitumor agent.

In a fourth aspect, the invention relates to a pharmaceutical composition comprising a therapeutically effective amount of a composition comprising a Nudix5 inhibitor and at least a second antitumor agent and a pharmaceutically acceptable excipient or carrier.

In a fifth aspect, the invention relates to a composition of the invention or a pharmaceutical composition of the invention for use in the treatment and/or prevention of cancer in a subject.

In a sixth aspect the invention relates to a method for assaying the activity of Nudix5 comprising assaying the formation of ATP.

In a seventh aspect, the invention relates to a method for identifying a compound potentially useful for treating and/or preventing cancer comprising assaying the activity of Nudix5 in the presence of a compound, wherein a compound that inhibits Nudix5 activity is a compound potentially useful for treating and/or preventing cancer.

In an eight aspect, the invention relates to a kit comprising a reagent for analyzing the ATP synthase activity of Nudix5.

In a ninth aspect, the invention relate to the use of the kit for analyzing the ATP synthesizing activity of Nudix5.

## BRIEF DESCRIPTION OF THE FIGURES

**Figure 1: ATP levels increase in the nucleus of cells treated with hormone.**

**A**. Representative immunoflurorescent images showing the localisation of Mitochondrial/Nuclear and Cytoplasmic ATeam ATP sensors in T47D cells.

**B.** Schematic detailing the experimental procedure used to record and measure ATP levels live in cells.

**C**. ATP/ADP ratio quantification of Mito-ATeam treated with glucose (10mM) at time 0 (T0). Data is presented as $\log_2^{YFP/CFP}$.

**D.** ATP/ADP ratio quantification of Mito-ATeam treated with glucose (10mM) at time zero (T0) followed by oligomycin addition 10 minutes afterwards (T10). Data is presented as $\log_2^{YFP/CFP}$.

**E.** T47Dwt cells transfected with nuclear/mitochondrial or cytoplasmic targeted luciferase constructs. The protein levels of each construct were detected by western blotting using luciferase specific antibody (* indicates non-specific antibody binding). **F.** T47Dwt cells transfected with nuclear/mitochondrial or cytoplasmic targeted luciferase constructs (schematic indicated). Representative images of the cellular localisation of each construct comparing DAPI, nuclear staining and an anti-luciferase antibody is shown. No phenotypic abnormalities were observed following expression of the luciferase constructs (bright field).

**Figure 2: ATP levels increase in the nucleus of cells treated with hormone.**

**A**. ATP/ADP ratio quantification of Nuc-ATeam expressing cells treated with hormone (R5020, left panel) or mock treated (EtOH alone, right panel). Data is the average of 18 nuclear ROI's +/-SEM, presented as $\log_2^{YFP/CFP}$.

**B.** Representative ratio images (5 minute intervals) of nuc-ATeam treated with hormone (T0). Scale bar indicates ratio YFP/CFP.

**C.** ATP/ADP ratio quantification of Mito-ATeam expressing cells treated with hormone (R5020) or mock treated (EtOH alone). Data is the average of 16 nuclear ROI's +/-SEM, presented as $\log_2^{YFP/CFP}$.

**D.** Bioluminescence image of cells transfected with Nuclear, Mitochondrial or Cytoplasmic targeted luciferase constructs and treated with hormone (R5020) for the time indicated. Scale bar represents the relative bio luminescence (RLU $x10^6$).

**E.** Quantification of the data presented in (D). Data is indicative of three independent experiments carried out in duplicate +/-SEM.

**Figure 3: Mitochondiral derived ATP is required initially following hormone.**

**A**. Bioluminescence image of cells transfected with Nuclear or Mitochondrial targeted luciferase constructs. Cells were treated with hormone for the time indicated either with (+) or without (-) prior treatment with oligomycin.

**B.** Quantification of the data presented in (A). Data is indicative of three independent experiments carried out in duplicate +/-SEM.

**C.** ATP/ADP ratio quantification of Nuc-ATeam treated with glucose (T0). Data is presented as $\log_2^{YFP/CFP}$. Data is the average of 14 nuclear ROI's +/-SEM, presented as $\log_2^{YFP/CFP}$.

**D.** ATP/ADP ratio quantification of Nuc-ATeam treated with glucose and oligomycin (T0). Data is presented as $\log_2^{YFP/CFP}$. Data is the average of 8 nuclear ROI's +/-SEM, presented as $\log_2^{YFP/CFP}$.

**E.** ATP/ADP ratio quantification of Nuc-ATeam treated with hormone (T0) and oligomycin (T10). Data is presented as $\log_2^{YFP/CFP}$. Data is the average of 12 nuclear ROI's +/-SEM, presented as $\log_2^{YFP/CFP}$.

**Figure 4: Transient Levels of PAR are mediated via the combined actions of PARP1 and PARG**

PAR levels were visualised by immunofluorescence using two different anti-PAR antibodies (monoclonal; *red*, polyclonal; *green*) in cells treated with hormone for the time points indicated following inhibition of PARP (PARPi; 3AB, top panel) and PARG (TA, PARGi, lower panels).

**Figure 5: PAR degradation in response to hormone and identification of PARP1 target proteins.**

**A**. Effect of hormone and inhibitors on PAR levels. PAR levels were visualised by immunofluorescence using anti-PAR antibody in untreated cells (T0) and in cells treated with hormone for 30 minutes (T30) in the absence of inhibitors (Control), or following inhibition for 30 minutes of PARP by 3AB (PARPi) or of PARG by TA (PARGi).
**B.** Quantitation of PAR levels in response to hormones and inhibitors. PAR levels were determined by PAR-capture ELISA in cells treated with hormone in the presence or absence of PARP or PARG inhibition (PARPi, PARGi).
**C.** Effect of hormone and inhibitors on NAD levels. NAD levels were determined by NAD colourimetric assay (Wright et al 2012, cited supra) in cells treated with hormone and inhibitors as in **B.**
**D**. Schematic representation of the experimental procedure used to identify PARylation target proteins in cells treated with hormone for 30 minutes. Right: Venn diagram showing the number of unique proteins identified by mass-spec.

**Figure 6: Novel PAR/PARG interactor NUDIX5 plays a role in hormone-induced nuclear ATP generation.**

A. Immunoprecipitation was performed in T47D cells treated with hormone (R5020, 30 minutes) using anti-PARP1, PARG, PAR or NUDIX5 antibodies. Protein complexes were separated by SDS-PAGE and probed using specific antibodies.
**B.** T47D cells were transfected with specific siRNA against NUDIX5 and the protein levels of NUDIX5 measured by western blot using specific antibodies.
**C.** Quantification of nuclear, mitochondrial and cytoplasmic targeted luciferase constructs treated with hormone (Time 0 minutes) in control (untreated) or in the presence of PARP inhibition (3AB, PARPi), PARG inhibition (TA, PARGi) or siRNA against Nudix5 (Nudix5i). Data represents the mean of three independent experiments carried out in duplicate +/- SEM
**D**. Bioluminescence image of cells transfected with Nuclear or Mitochondrial targeted luciferase constructs. Cells were treated with hormone for the time indicated in control (untreated) or in the presence of PARP inhibition (3AB, PARPi), PARG inhibition (TA, PARGi).
**E.** ATP assay in vitro, performed in the presence or absence of recombinant Nudix5 with varying concentrations of ADPR (as indicated).

**Figure 7: Nudix5 overexpression correlates with PARP1 and PARP family expression in breast and other cancers.**

**A.**qRT-PCR analysis showing the relative mRNA expression ofNudix family members Nudt12, Nudt6 and Nudt9 in the presence or absence of Nudt5 siRNA.
**B.** The expression of Nudix5, PARG along with other annotated nudix family members is shown for the 58 breast cancer cohorts (**A**) (n=2456 patients). The p value given indicates the significance of the difference obtained, note only the overexpression of Nudix5 and PARG but not other Nudix family members is significant.
**C.** The expression of Nudix5, in conjunction with annotated PARP1 family members is shown for normal breast and invasive ductal breast carcinoma (n=1056 patients). The p value given indicates the significance of the difference obtained. Note Nudix5 overexpression clearly correlated with PARP1 and DNA damage proteins in cancer versus normal tissue.

**Figure 8: Nuclear ATP is essential for hormone regulated gene expression and chromatin remodelling.**

**A**. Venn diagram showing the overlap of progesterone regulated genes (FC<-1.5>1.5, p<0.05) dependent on the actions of PARG and or Nudix. Below; Breakdown of up and down progesterone regulated genes, indicating their dependence on PARG, NUDIX or both.
**B.** Venn diagram showing the overlap of 2448 progesterone regulated genes (FC<-1.5>1.5, p<0.05) dependent on the actions of PARP, PARG and or Nudix.
**C.** Breakdown of both up (FC>1.5, p<0.05) and down (FC<1.5, p<0.05) regulated genes and the dependence on PARP, PARG or Nudix.
**D.** Cell proliferation was measured by BrdU incorporation in T47D cells at the time points indicated following in

the addition of hormone in the presence or absence of PARP, PARG, and Nudix. Data represents the mean of two independent experiments carried out in duplicate +/-SEM.

**E.** Cell proliferation was measured by BrdU incorporation in MCF7 cells at the time points indicated following the addition of hormone (estrogen) in the presence or absence of PARP, PARG, and Nudix. Data represents the mean of two independent experiments carried out in duplicate +/-SEM.

**F.** The expression of oestrogen target genes in the presence of PARP, PARG or Nudix5 inhibition was analysed by qRT-PCR. RNA was extracted 6 hours post hormone induction and the data is represents the mean of three independent experiments carried out in duplicate +/-SEM.

The enrichment of H1 **(G)** or H2A **(H)** was measured by ChIP using H2A or H1 specific antibodies in T47D cells treated with hormone for the time points indicated in the presence or absence of PARP (3AB, PARPi), PARG (TA, PARGi) or Nudix5 inhibition (siNudix5). Data represents the mean 5 different amplified regions previously demonstrated to be depleted of both H2A and H1 following hormone (Vicent et al 2012, Wright et al 2012) and two independent experiments carried out in duplicate +/-SEM.

**Figure 9: Cell growth and gene expression in response to oestrogen is dependent on the activities of PARP, PARGand Nudix5.**

**A**. MCF7 cells were treated with oestrogen in the presence or absence of the PARP (3AB) or PARG (TA) inhibitors; cells proliferation was visualized 48 hours later by bright field microscopy.

**B**.Immunofluroescence images showing the levels of PAR and phospho-ER (Serine 118) in MCF7 cells treated with oestrogen for 30 minutes.

**C.**MCF7 cells were transfected with nuclear-luciferase constructs as described and the levels of ATP measured by bioluminescence following addition of oestrogen for the time points indicated; top panel shows the bioluminescence image, below quantification of the bioluminescence (ATP) signal in the presence of PARP, PARG or NUDIX inhibition (PARGi; TA, PARPi; 3AB and NUDIXi; siNUDIX5).

**D**. Global Chip-seq analysis indicating the relative depletion or enrichment of the ChIP-seq profile indicated. Data is represented as fold change (FC) in normalised reads (for histone marks, right panel) or peaks (transcription factors, left panel). The signal was measured across the promoters (+/-2kbp), of hormone response genes (up □ or down □ regulated) the activity of which is dependent (D) or independent (I) of PARG and Nudix activity (Figure 5B overlap).

**Figure 10: Model/Mechanism of Action.**

**A**. Model of ATP economy in the nucleus in response to hormone. ATP dynamics can be separated into three distinct phases. One; energetic demands of the nucleus are met via mitochondrial dependent mechanisms, resulting in an increase in nuclear ATP and chromatin remodelling. Two; the transient increase in PARylation via the actions of PARP1 and PARG result in a depletion of cellular NAD and thus ATP, in concert the increase in energetic demand due to chromatin remodelling in the nucleus, results in the activation Nudix5. Three; in the final stage the combined action of PARG and Nudix5 result in an increase in nuclear ATP independent of the mitochondria, in order to ensure the vast energetic demands of the chromatin are met and cell death via PARthanatos is avoided.

**B**. Phosphorylation of Threonine 45 in Nudix5 decreases following hormone, enrichment/TO log2.

**C**. Far view with the two interactions that will be fixed by the phosphorylation (left) in volume or ribbon when the bond is broken the green monomer leaves and allows access to the ADPR (specifically to the two phosphates that are farther in this view). Lower panel zoom active site indicating the phosphorylated residues; ADPR and Mg ion are visualised.

**D**. Comparison between the dimer stability/interface energies (using zrank), between 500 decoys of Nudix5 (PDB: 2DSC), non-phosphorylated (left) or phosphorylated (right). Both Wilcoxon ($2.2E^{-4}$), and t-distributions ($4.14E^{-4}$) support the statistical significance of this difference.

**E**. Overexpression of the phosphomimetic Nudix mutant T45D in T47D cells acts as a dominant negative as tested by both cell proliferation and progesterone induced gene expression analysis **(F),** data represents the mean relative mRNA abundance (FC T6hrs/T0hrs) of three independent experiments carried out in duplicate +/-SEM.

**Figure 11: Nudix5 active domain structure**

**A**. View of the dimer from the active site. From the red chain we can see to GLU (93 - far- and 112 -right-) and

the LYS27 that would stabilize the dimer when THR45 is phosphorylated and, thus, has more negative charge. The distance between both residues is 8 Angstroms here a distance significantly decreased when T45 is phosphorylated. ADPR and the Mg ion are visualised

**B**. Western blot showing the levels of recombinant GST-Nudix5 prepared by in vitro translation system and subsequent GST purification. These extracts were used in the ATP assays in vitro to test the ability of Nudix5 to generate ATP from ADPR and PPi as discussed later.

**Figure 12 Cell Survival following DNA Damage is dependent on PARP1, PARG and Nudix functionality**

**A**. Western blot showing the protein levels of PAR and transfected myc-Nudix5 mutant constructs in T47D cells following hormone treatment for the times indicated.

**B**. T47D cells were transfected with myc-Nudix5 mutant expression plasmids as described and the relative mRNA levels of hormone induced genes, MMTV and EGFR were detected following hormone for 6 hours.

**C.** T47D cells were transfected with myc-Nudix5 mutant expression plasmids as described and cell proliferation was determined by performing a BrdU incorporation assay as described following hormone addition for the times indicated.

**D.** ATP levels following hormone were detected by bioluminescence as described in T47D wt cells. Prior to ATP detection, cells were transfected both myc-Nudix5 mutant or wild type expression plasmids and nuclear-luciferase contructs

E. Quantification of the bioluminescence data presented in Figure 12D.

F. Schematic representation of the levels of nuclear ATP and PAR in cells treated with hormone, initially nuclear ATP levels peak following hormone, the subsequent decrease is in conjunction with rising PAR and depleting NAD and ATP levels. Following the rise in PAR levels, PAR levels drop dramatically in direct correlation with a second peak in nuclear

**Figure 13: Nudix5 as a target for cancer therapy.**

**A**. MCF7 cells were transfected with Nudix5 si RNA or the activites of PARP and PARG inhibited via the addition of either 3AB or TA. Cells were then submitted to DNA damage via the addition of 0.2mM $H_2O_2$. Cells were then grown for 14 days, and the surviving colonies visualised by crystal violet staining as described. The image represented here shows and example.

**B**. Quantification of the data presented in Figure 13A, including titration of $H_2O_2$ concentration. Data is represented as a percentage surviving fraction as compared to undamaged controls in the presence of the inhibitor (ie PARPi control v PARPi Damage).

**C**. Probability survival curves of ER and PR positive breast cancer patients, Patient were separated according to Nudix5 levels; High or low Nudix5 levels being characterised as >75 or <25 percentile from the sample data.

**D**. Probability survival curves of ER and PR negative breast cancer patients, Patient were separated according to Nudix5 levels; High or low Nudix5 levels being characterised as >75 or <25 percentile from the sample data.

**E.** Graph indicates the levels of Nudix5 (log2 median centered intensity) across 19 multi-cancer types. The cancer type and number of patient samples are indicated in the legend. Data was obtained from oncominer database.

**F.** Graph indicates the levels of Nudix5 and PARP1 family members in acute lymphoblastic leukaemia versus normal blood mononuclear cells. The p value is given adjacent to the gene name. Data was obtained from oncominer database.

## DETAILED DESCRIPTION OF THE INVENTION

The economy of nuclear ATP has not attracted much attention. The general assumption is that nuclear energetic demands are met via the diffusion of ATP from the mitochondria. Although this may be the case in steady state situations, the question remains about the source of the energy when extensive or sudden changes in chromatin demand it. It has been suggested that ATP can indeed be generated within the nucleus via a mitochondria independent mechanism. Nearly 60 years ago Allfrey and Mirsky proposed that ATP could be generated in isolated nuclei. Later this was confirmed and it was proposed that the endogenous substrate for nuclear oxidative phosphorylation was in part generated by the ribose pathway. In 1989 an enzyme activity named ADP-ribose pyrophosphorylase (ARPP) was identified in HeLa cell nuclei that catalysed the formation of ATP and Ribose-5-phosphate from ADP-ribose and PPi. ATP generation by ARPP via ADPR catabolism has been proposed to be involved in DNA repair and replication. But whether nuclear ATP is also required for changes in gene expression in response to various signals is not known.

The authors of the present invention have previously shown that in response to progestins (Pg) breast cancer

cells undergo extensive changes in gene expression that require a global modification of chromatin, involving several kinases, histone modifying enzymes and multiple ATP-dependent chromatin remodelers. One key event in this response is the rapid and transient generation of poly-ADP-ribose (PAR) by PARP1 that consumes about 50% of the cellular NAD. They considered the possibility that the transient nature of PAR accumulation reflects the need for the degradation of PAR as an essential step that may indeed play a role in the energetic balance of the cell nucleus. Therefore the authors set out to measure the levels of nuclear ATP in cells treated with hormone and identify the enzymes involved in its generation.

The authors of the present invention have found that degradation of PAR by PARG is essential for hormonal gene regulation and cell proliferation of breast cancer cells and that it correlated with the generation of nuclear ATP catalyzed by a form of NUDIX5/NUDT5 that is generated a few minutes after hormone addition by dephosphorylation at T45. Blocking PAR degradation, depleting NUDIX5, or using a T45D phosphomimetic mutant of NUDIX5 compromises hormonal induced cell proliferation, gene regulation and chromatin remodeling.

<u>Medical uses</u>

In an aspect, the invention relates to a Nudix5 inhibitor for use in the treatment and/or prevention of cancer in a subject.

Alternatively the invention relates to the use of a Nudix5 inhibitor for the preparation of a medicament for the treatment and/or prevention of cancer in a subject.

"Nudix5" or NUDT5, belongs to the Nudix (nucleoside diphosphate linked moiety X) hydrolase superfamily. The encoded enzyme catalyzes the hydrolysis of modified nucleoside diphosphates, including ADP-ribose (ADPR) and 8-oxoGua-containing 8-oxo-dADP and 8-oxo-dGDP. The sequence of Nudix5 protein in humans corresponds to the sequence Q9UKK9 in the Uniprot database 27 March 2014.

<u>"Nudix5 inhibitor</u>", as used herein, relates to any compound capable of causing a decrease in the activity of Nudix5, particularly in the ATP synthesizing activity, including those compounds which prevent expression of Nudix5 gene and compounds which lead to reduced Nudix5 mRNA or protein levels, as well as any compound that stabilizes the inactive form of Nudix5.

The expression of a protein or nucleic acid is considered reduced when its levels decrease with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

The reference value refers to the level of protein or nucleic acid in control subject, which may be a subject who does not suffer a specific disease.

Suitable methods for determining whether an inhibitor is capable of decreasing Nudix5 mRNA levels include, without limitation, standard assays for determining mRNA expression levels such as qPCR, RT-PCR, RNA protection analysis, Northern blot, RNA dot blot, *in situ* hybridization, microarray technology, tag based methods such as serial analysis of gene expression (SAGE) including variants such as LongSAGE and SuperSAGE, microarrays, fluorescence *in situ* hybridization (FISH), including variants such as Flow-FISH, qFiSH and double fusion FISH (D-FISH), and the like. Preferably quantitative or semi-quantitative RT-PCR is preferred. Real-time quantitative or semi-quantitative RT-PCR is particularly advantageous.

The nucleic acid contained in the sample (e.g., cell or tissue prepared from the subject) is first extracted according to standard methods, for example using lytic enzymes or chemical solutions or extracted by nucleic-acid-binding resins following the manufacturer's instructions.

In case the mRNA is measured in a biological sample, said biological sample may be treated to physically, mechanically or chemically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor. In a particular embodiment samples can be obtained

from fresh tumor tissue or from OCT embedded frozen tissue.

In order to normalize the values of mRNA expression among the different samples, it is possible to compare the expression levels of the mRNA of interest in the test samples with the expression of a control RNA. A "control RNA" as used herein, relates to RNA whose expression levels do not change or change only in limited amounts in tumor cells with respect to non-tumorigenic cells. Preferably, the control RNA is mRNA derived from housekeeping genes and which code for proteins which are constitutively expressed and carry out essential cellular functions. Preferred housekeeping genes for use in the present invention include β-2-microglobulin, ubiquitin, 18-S ribosomal protein, cyclophilin, IPO8, HPRT, GAPDH, PSMB4, tubulin and β-actin.

The relative gene expression quantification may be calculated according to the comparative threshold cycle (Ct) method using a housekeeping gene as an endogenous control and commercial RNA controls as calibrators. Final results are determined according to the formula $2^{-(\Delta Ct\ sample - \Delta Ct\ calibrator)}$, where ΔCt values of the calibrator and sample are determined by subtracting the Ct value of the target gene from the value of the control gene.

Suitable methods for determining whether an inhibitor acts by decreasing the Nudix5 protein levels include the quantification by means of conventional methods, for example, using antibodies with a capacity to specifically bind to the proteins encoded by Nudix5 gene (or to fragments thereof containing antigenic determinants) and subsequent quantification of the resulting antibody-antigen complexes.

The antibodies to be employed in these assays can be, for example, polyclonal sera, hybridoma supernatants or monoclonal antibodies, antibody fragments, Fv, Fab, Fab' and F(ab')2, ScFv, diabodies, triabodies, tetrabodies and humanized antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc. There are a wide variety of well-known assays that can be used in the present invention, which use non-labeled antibodies (primary antibody) and labeled antibodies (secondary antibodies); among these techniques are included Western blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as dipsticks. Other ways of detecting and quantifying the levels of the protein of interest include techniques of affinity chromatography, binding-ligand assays, etc.

On the other hand, the determination of the levels of the Nudix5 protein can be carried out by constructing a tissue microarray (TMA) containing the subject samples assembled, and determining the expression levels of the corresponding protein by immunohistochemistry techniques. Immunostaining intensity can be evaluated by two or more different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumor cells and the specific cut-off for each marker. As a general criterion, the cut-offs are selected in order to facilitate reproducibility, and when possible, to translate biological events. Alternatively, the immunostaining intensity can be evaluated by using imaging techniques and automated methods such as those disclosed in Rojo, M.G. et al. (Folia Histochem. Cytobiol. 2009; 47: 349-54) or Mulrane, L. et al. (Expert Rev. Mol. Diagn. 2008; 8: 707-25).

Alternatively, in another particular embodiment, the levels of the Nudix5 protein are determined by Western blot. Western blot is based on the detection of proteins previously resolved by gel electrophoreses under denaturing conditions and immobilized on a membrane, generally nitrocellulose, by the incubation with an antibody specific and a developing system (e.g. chemoluminiscent).

A Nudix5 inhibitor may inhibit Nudix5 activity by at least 5%, at least 10%, at least 25%, at least 50%, at least 75%, or at least 90%, and all ranges between 5% and 100%. Suitable methods for determining whether an inhibitor acts by decreasing the Nudix5 activity include any method which allows determining the ATP formation. Assays to determine the activity of an enzyme are known by the skilled person and include, without limitation, initial rate assays, progress curve assays, transient kinetics assays and relaxation assays. Continuous assays of enzymatic activity include, without limitation, spectrophotometric, fluorometric, calorimetric, chemiluminiscent, light scattering and microscale thermopheresis assays. Discontinuous assays of enzymatic activity include, without limitation, radiometric and chromatographic assays. As the skilled person understands, factors that may influence enzymatic activity comprise salt concentration, temperature, pH, and substrate concentration.

The determination of the inhibitory capacity on the biological activity of Nudix5 can be detected by an *in vitro* assay based on measurement of ATP generation via ADPR + PPi, using for example a bioluminescence reagent. In a preferred embodiment, the biological activity of Nudix5 is measure using the method for assaying the Nudix5 activity of the invention.

In a preferred embodiment, Nudix5 inhibitors useful in the present invention are selected from Table 1.

| TABLE 1: Nudix5 INHIBITORS SUITABLE FOR USE ACCORDING TO THE INVENTION | |
|---|---|
| I | Small chemical compounds that inhibit ATP synthesizing activity of Nudix5. |
| II | Phosphomimetic T45D mutant of Nudix5 |
| III | Phosphonull T45A mutant of Nudix5 |
| IV | An interference RNA specific for the Nudix5 gene sequence |
| V | An antisense oligonucleotide specific for the Nudix5 gene sequence |
| VI | A ribozyme or DNA enzyme specific for the Nudix5 gene sequence |
| VII | Inhibitory antibodies that specifically bind to Nudix5 and inhibit ATP synthesizing activity of Nudix5. |
| VIII | Aptamers and spiegelmers. |

"Small chemical compound", as used herein relates to a molecule that regulate a biological process, in the present case inhibits ATP synthesizing activity of Nudix5. This compound can be natural or artificial.

"Phosphomimetic T45D mutant of Nudix5" refers to a Nudix5 protein wherein the threonine at position 45 is substituted by aspartic acid. Said mutant impairs progesterone induced gene expression and cell proliferation.

"Phosphonull T45A mutant of Nudix5" refers to a Nudix5 protein wherein the threonine at position 45 is substituted by alanine.

As used herein, the term "interference RNA" or "iRNA" refers to RNA molecules capable of silencing the expression of Nudix5 or of any gene needed for Nudix5 function. To that end, iRNA are typically double-stranded oligonucleotides having at least 30 base pairs in length, and they more preferably comprise about 25, 24, 23, 22, 21, 20, 19, 18 or 17 ribonucleic acid base pairs. Several different types of molecules have been used effectively in iRNA technology including small interfering RNA (siRNA) sometimes known as short interference RNA or silencer RNA, micro RNA (miRNA) which normally differ from siRNA because they are processed from single-stranded RNA precursors and they are shown to be only partially complementary to the target mRNA and short hairpin RNA (shRNA).

Small interfering RNA (siRNA) agents are capable of inhibiting target gene expression by interfering RNA. siRNAs may be chemically synthesized, or may be obtained by *in vitro* transcription, o may be synthesized *in vivo* in target cell. Typically, siRNAs consist of a double-stranded RNA from 15 to 40 nucleotides in length and may contain a protuberant region 3' and/or 5' from 1 to 6 nucleotides in length. Length of protuberant region is independent from total length of siRNA molecule. siRNAs act by post-transcriptional degradation or silencing of target messenger.

siRNA may be denominated shRNA (short hairpin RNA) characterized because the antiparallel strands that form siRNA are connected by a loop or hairpin region. siRNAs are constituted by a short antisense sequence (19 to 25 nucleotides) followed by a loop of 5-9 nucleotides, and the sense strand. shRNAs may be encoded by plasmids or virus, particularly retrovirus and, more particularly, retrovirus and under the control of promoters such as U6 promoter for RNA polymerase III.

The siRNAs for use within the context of the invention are substantially homologous to Nudix5 mRNA or this protein-coding genome sequence. The term "substantially honomogous" is understood to mean that siRNAs have a sequence sufficiently complementary or similar to target mRNA so that siRNA may be able to provoke mRNA degradation by RNA interference. Suitable siRNAs to provoke interference include siRNAs formed by RNA, as well as siRNAs containing chemically different modifications such as:

- siRNAs in which the links between nucleotides are different from those appearing in nature, such as phosphorothioate links;
- stranded-RNA conjugates with a functional reagent, such as a fluorophoro;
- modification of the ends of RNA strands, particularly the end 3' end by the combination with different functional hydroxyl groups at 2'-position;
- sugar-modified nucleotides such as O-alkylated radicals at 2'-position such as 2'-O-methylribose or 2'-O-fluororibose;
- base-modified nucleotides such as halogenated bases (for example 5-bromouracil and 5-iodouracil), alkylated bases (for example 7-methyl-guanosine).

The siRNAs and shRNAs for use within the context of the invention may be obtained using a series of techniques known to a person skilled in the art. For example, siRNA may be chemically synthesized from protected ribonucleoside phosphoramidites in a conventional DNA/RNA synthesizer. Alternatively, siRNA may be produced by recombinant dicer

from plasmid and viral vectors, where the coding region of siRNA strand or strands is under operative control of RNA polymerase III promoters. RNase Dicer processes shRNA into siRNA in cells.

The Nudix5 region which is taken as a basis for the design of siRNA is not limitative and may contain a region of coding sequence (between the initiation codon and the termination codon) or, alternatively, may contain sequences from the 5' or 3' untranslated region, preferably from 25 to 50 nucleotides in length and in any position in 3' position with regard to the initiation codon. A procedure for siRNA design involves the identification of sequence motive AA(N19)TT wherein N may be any nucleotide in Nudix5 sequence and the selection of those that exhibit a high content in G/C. If said sequence motive is not found, it is possible to identify sequence motive NA(N21) wherein N may be any nucleotide.

In a preferred embodiment the Nudix5 inhibitor is an siRNA. In a more preferred embodiment the siRNA is a commercial siRNA from Santa Cruz Biotechnology, particularly sc-75973.

In another embodiment, the inhibitor is an "antisense oligonucleotide" specific to Nudix5, i.e., molecules whose sequence is complementary to mRNA coding for Nudix5 i.e., complementary to cDNA coding chain. The antisense oligonucleotide may be complementary to a complete coding region or a region of same including both the coding region and the 5' and 3' untranslated regions. The antisense oligonucleotides may consist of 5, 10, 15, 20, 25, 30, 35, 40, 45, 50 or more nucleotides in length. The antisense oligonucleotides may be obtained by chemical synthesis or by enzymatic binding reactions widely known to a person skilled in the art. For example, an antisense oligonucleotide may further contain modified nucleotides which increase its biological stability or the stability of the bicatenary DNA-RNA complexes formed between the antisense oligonucleotide and the target polynucleotide, such as phosphorothioate derivatives, peptide nucleic acids and acridine-substituted oligonucleotides. Modified oligonucleotides that may be used for the preparation of antisense nucleic acid include 5-fluorouracil, 5-bromouracil, 5-chlorouracil, 5-iodouracil, hypoxanthine, xanthine, 4-acetyl-citosine, 5-(carboxyhydroxylmethyl) uracil, 5-carboxymethylaminomethyl-2-thiouridine, 5-carboxymethyl-aminomethyl uracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcitosine, 5-methylcitosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6- isopentenyladenine, uracil-5-oxyacetic acid, pseudouracil, queosine, 2- thiocitosine, 5-methyl-2-thiouracil, 2-thiouracil, 4-thiouracil, 5-methyluracil, uracil-5-oxyacetic acid methyl ester, 5-methyl-2-thiouracil, 3-(3-amino-3-N-2-carboxypropyl)uracil, and 2,6- diaminopurine. Alternatively, the antisense nucleic acid may be produced biologically using an expression vector in which the antisense-oriented nucleic acid has been cloned.

Another group of compounds that may form part of the present invention are catalytically active nucleic acids known as ribozimes. "Ribozimes" comprise a catalytic region and a second region whose sequence is complementary to target nucleic acid and confers substrate specificity on the ribozime. After the interaction between the ribozime and its substrate by hybridization and coupling between complementary regions of target nucleic acid and ribozime, an activation of the catalytic region is produced provoking the inter- or intramolecular rupture of target nucleic acid. Basic considerations for the design of ribozimes are widely known to a person skilled in the art (see, for example, Doherty and Doudna (Annu. Ref. Biophys. Biomolstruct. 2000; 30:457- 75).

Another type of compounds that may form part of the compositions of the invention includes inhibitory antibodies. The term "inhibitory antibody" is understood to mean, according to the present invention, an antibody that binds to Nudix5 provoking the inhibition of its ATP synthesizing activity. In a particular aspect, the inhibitory antibody against Nudix5 binds to Nudix5 and more particularly prevents the dephosphorylation of the threonine at position 45. In another particular aspect, the inhibitor antibody against Nudix5 binds to Nudix5 inhibiting its ATP synthesizing activity.

Antibodies may be prepared using any method known to a person skilled in the art. Thus, polyclonal antibodies are prepared by immunization of an animal with the protein aimed to be inhibited. Monoclonal antibodies can be prepared using the method described by Kohler, Milstein et al (Nature, 1975, 256: 495). Once antibodies capable of binding to Nudix5 are identified, those antibodies capable of inhibiting Nudix5 activity using the abovementioned assays for determination of Nudix5 activity will be selected. Suitable antibodies in the present invention include intact antibodies which comprise an antigen-binding variable region and a constant region, fragments "Fab", "F(ab')2" y "Fab"', Fv, scFv, diabodies and bispecific antibodies.

Other compounds capable of inhibiting Nudix5 expression that may form part of the compositions of the invention include aptamers and spiegelmers. "Aptamers and spiegelmers" are single-stranded or double-stranded D- or L-nucleic acids that specifically bind to the protein resulting in a modification of the biological activity of protein (Nudix5). Aptamers and spiegelmers are 15 to 80 nucleotides in length and, preferably, 20 to 50 nucleotides in length.

The term "treatment", as used herein comprises any type of therapy, which aims at terminating, preventing, ameliorating and/or reducing the susceptibility to a clinical condition as described herein. In a preferred embodiment, the term treatment relates to prophylactic treatment (i.e. a therapy to reduce the susceptibility of a clinical condition, a disorder or condition as defined herein). Thus, "treatment," "treating," and the like, as used herein, refer to obtaining a desired pharmacologic and/or physiologic effect, covering any treatment of a pathological condition or disorder in a mammal, including a human. The effect may be prophylactic in terms of completely or partially preventing a disorder or

symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, and/or immune deficiency.

As the skilled person acknowledges, effectiveness of a Nudix5 inhibitor in cancer therapy is demonstrated by reduced tumor volume, improved symptoms, and/or decreased serological tumor markers.

Various techniques have been developed in the art for monitoring tumor response to a therapy, wherein measuring tumor size and/or shrinkage on computed tomography (CT) or other anatomic imaging modalities represents a current standard. Imaging of tumor metabolism with PET and the glucose analog 18F-FDG is contemplated as well for assessing the effects of therapy objectively and quantitatively. In cases where the tumor can be easily accessed, biopsies may be taken sequentially before and during treatment and analyzed to monitor the effects of treatment on molecular processes, including development of tumor resistance. Methods for obtaining a biopsy sample are known by the skilled person and include, without limitation, surgical resection of a tissue mass or microdissection or other known cell separation methods, the cells can additionally be obtained by aspiration cytology by means of the pricking with a thin needle connected to a syringe.

The term "subject", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the subject is a male or female human of any age or race.

The term "cancer" or "tumour" or "tumour disease", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. Cancers usually share some of the following characteristics: sustaining proliferative signalling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumour cells resemble, which is therefore presumed to be the origin of the tumour.

These types include:

- Carcinoma: Cancers derived from epithelial cells. This group includes many of the most common cancers, particularly in the aged, and include nearly all those developing in the breast, prostate, lung, pancreas, and colon.
- Sarcoma: Cancers arising from connective tissue (i.e. bone, cartilage, fat, nerve), each of which develop from cells originating in mesenchymal cells outside the bone marrow.
- Lymphoma and leukaemia: These two classes of cancer arise from hematopoietic (blood-forming) cells that leave the marrow and tend to mature in the lymph nodes and blood, respectively.
- Germ cell tumour: Cancers derived from pluripotent cells, most often presenting in the testicle or the ovary (seminoma and dysgerminoma, respectively).
- Blastoma: Cancers derived from immature "precursor" cells or embryonic tissue. Blastomas are more common in children than in older adults.

In a preferred embodiment the cancer is selected from breast, ovarian, prostate, brain, pancreas, skin, bone, bone marrow, blood, thymus, uterus, testicles, hepatobiliary and liver tumors, adenoma, angiosarcoma, astrocytoma, epithelial carcinoma, germinoma, glioblastoma, glioma, hemangioendothelioma, hemangiosarcoma, hematoma, hepatoblastoma, leukaemia, lymphoma, medulloblastoma, melanoma, neuroblastoma, hepatobiliary cancer, osteosarcoma, retinoblastoma, rhabdomyosarcoma, sarcoma, and teratoma, acrallentiginous melanoma, actinic keratosis adenocarcinoma, adenoid cystic carcinoma, adenomas, adenosarcoma, adenosquamous carcinoma, astrocytictumors, bartholin gland carcinoma, basal cell carcinoma, bronchial gland carcinoma, capillary carcinoid, carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing’s sarcoma, focal nodular hyperplasia, germ cell tumors,

glioblastoma, glucagonoma, hemangioblastoma, hemangioendothelioma, hemangioma, hepatic adenoma, hepatic adenomatosis, hepatocellular carcinoma, hepatobiliary cancer, insulinoma, intraepithelial neoplasia, interepithelial squamous cell neoplasia, invasive squamous cell carcinoma, large cell carcinoma, leiomyosarcoma, melanoma, malignant melanoma, malignant mesothelialtumor, medulloblastoma, medulloepithelioma, mucoepidermoid carcinoma, neuroblastoma, neuroepithelial adenocarcinoma, nodular melanoma, osteosarcoma, papillary serous adenocarcinoma, pituitary tumors, plasmacytoma, pseudosarcoma, pulmonary blastoma, renal cell carcinoma, retinoblastoma, rhabdomyosarcoma, sarcoma, serous carcinoma, small cell carcinoma, soft tissue carcinoma, somatostatin-secreting tumor, squamous carcinoma, squamous cell carcinoma, undifferentiated carcinoma, uveal melanoma, verrucous carcinoma, vipoma, Wilm's tumor, intracerebral cancer, head and neck cancer, rectal cancer, astrocytoma, glioblastoma, small cell cancer, and non-small cell cancer.

In another preferred embodiment, the cancer to be treated according to the invention is characterised by expressing increased levels of Nudix5. Nudix5 levels are considered to be increased with respect to a reference value when the levels of Nudix5 in a sample of said cancer show an increase of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more. The reference value can be the value corresponding to the level of expression of Nudix5 in a non-cancer sample.

Types of cancer showing increased levels of Nudix5 are, for example acute myeloid leukemia, breast carcinoma, colorectal adenocarcinoma, diffuse large B-cell lymphoma, endometrial adenocarcinoma, follicular lymphoma, lung adenocarcinoma, melanoma, ovarian adenocarcinoma, pancreatic adenocarcinoma, pleural mesothelioma, B-cell acute lymphoblastic Leukemia, T-cell Acute Lymphoblastic Leukemia, prostate carcinoma and renal cell carcinoma.

In another preferred embodiment the cancer is a hormone-dependent cancer. A hormone-dependent cancer refers to a cancer that has hormonal sensitivity. Examples of said cancers are, without limitation, breast, endometrium, ovary, prostate, testis, thyroid and osteosarcoma cancer. In a preferred embodiment the cancer is a steroid-dependent cancer, more preferred estrogen and progestin cancer. In a more preferred embodiment the progestin dependent cancer is a progestin-dependent breast cancer.

In another more preferred embodiment, the cancer is an androgen dependent cancer, more preferably androgen-dependent prostate cancer.

*Pharmaceutical compositions [1]*

For their medicinal use, the Nudix5 inhibitor may be found in a pharmaceutical composition.

Thus, in a further aspect, the invention relates to a pharmaceutical composition comprising a therapeutically effective amount of a Nudix5 inhibitor and a pharmaceutically acceptable excipient or carrier, pharmaceutical composition [1].

The term "pharmaceutical composition", as used herein, refers to a composition comprising a therapeutical effective amount of the agent according to the present invention and at least one pharmaceutically acceptable excipient or carrier.

The term "therapeutically effective amount" as used herein in relation to the agent of the invention, or in relation to the agent, excipient and/or carrier comprised by the pharmaceutical composition of the invention, relates to the sufficient amount of said agent, excipient and/or carrier to provide the desired effect, i.e. to achieve an appreciable prevention, cure, delay, reduction of severity or amelioration of one or more symptoms derived from a disease, and will generally be determined by, among other causes, the characteristics of the agent itself and the therapeutic effect to be achieved. It will also depend on the subject to be treated, the severity of the disease suffered by said subject, the chosen dosage form, etc. For this reason, the doses mentioned in this invention must be considered only as guides for the person skilled in the art, who must adjust the doses depending on the aforementioned variables. In an embodiment, the effective amount produces the amelioration of one or more symptoms of the disease that is being treated.

The combination of compounds of the pharmaceutical compositions of the invention may be found as a prodrug, salt, solvate or clatrate, whether in an isolated dosage form or in combination with additional active agents.

The terms "pharmaceutically acceptable excipient", or "pharmaceutically acceptable carrier", refer to a non-toxic solid, semisolid or liquid filler, diluent, encapsulating material or formulation auxiliary of any conventional type. A pharmaceutically acceptable carrier is essentially non-toxic to recipients at the dosages and concentrations employed, and is compatible with other ingredients of the formulation. Suitable carriers include, but are not limited to water, dextrose, glycerol, saline, ethanol, and combinations thereof. The carrier can contain additional agents such as wetting or emulsifying agents, pH buffering agents, or adjuvants which enhance the effectiveness of the formulation. Adjuvants could be selected from the group consisting of sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used

as vehicles. Suitable pharmaceutical vehicles are described in "Remington's Pharmaceutical Sciences" by E.W. Martin, 21st Edition, 2005.

In a preferred embodiment of the present invention, the compounds of the pharmaceutical composition of the invention are formulated in accordance with standard procedure as a pharmaceutical composition adapted for delivered administration to human beings and other mammals. Typically, pharmaceutical compositions for intravenous or intraventricular administration are solutions in sterile isotonic aqueous buffer.

Where necessary, the pharmaceutical composition of the invention also includes a solubilizing agent and a local anesthetic to ameliorate any pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the pharmaceutical composition is to be administered by infusion, it can be dispensed with an infusion bottle containing sterile pharmaceutical grade water or saline. Where the pharmaceutical composition is administered by injection, an ampule of sterile water for injection or saline can be provided so that the ingredients may be mixed prior to administration.

In cases other than intravenous administration, the pharmaceutical composition can contain minor amounts of wetting or emulsifying agents, or pH buffering agents. The pharmaceutical composition can be a liquid solution, suspension, emulsion, gel, polymer, or sustained release formulation. The pharmaceutical composition can be formulated with traditional binders and carriers, as would be known in the art. Formulations can include standard carriers such as pharmaceutical grades of mannitol, lactose, starch, magnesium stearate, sodium saccharide, cellulose, magnesium carbonate, etc., inert carriers having well established functionality in the manufacture of pharmaceuticals. Various delivery systems are known and can be used to administer a therapeutic of the present invention including encapsulation in liposomes, microparticles, microcapsules and the like.

In yet another preferred embodiment, therapeutics containing the pharmaceutical composition of the invention can be formulated as neutral or salt forms. Pharmaceutically acceptable salts include those formed with free amino groups such as those derived from hydrochloric, phosphoric, acetic, oxalic, tartaric acids and the like, and those formed with free carboxyl groups such as those derived from sodium, potassium, ammonium, calcium, ferric hydroxides, isopropylamine, thriethylamine, 2-ethylamino ethanol, histidine, procaine or similar.

Preferred excipients to be used in the present invention include sugars, starches, celluloses, gums and proteins. In a particular embodiment, the pharmaceutical composition of the invention will be formulated as a solid pharmaceutical dosage form (for example tablets, capsules, coated tablets, granules, suppositories, sterile crystalline or amorphous solids that can be reconstituted to provide liquid forms, etc.), liquid dosage form (for example solutions, suspensions, emulsions, elixirs, lotions, ointments, etc.) or semisolid dosage form (gels, pomades, creams and the like). Examples of pharmaceutically acceptable carriers are known in prior art and include phosphate buffered saline solutions, water, emulsions, such as oil/water emulsions, different types of humectants, sterile solutions, etc.

The pharmaceutical compositions containing the compounds according to the invention can occur at any pharmaceutical form of administration considered appropriate for the selected administration route, for example, by systemic, oral, parenteral or topical administration, for which it will include the pharmaceutically acceptable excipients necessary for formulation of the desired method of administration.

The effective quantity of the compounds of the invention can vary within a wide range and, in general, will vary depending on the particular circumstances of application, duration of the exposure and other considerations.

Solid dosage forms for oral administration may include conventional capsules, sustained release capsules, conventional tablets, sustained-release tablets, chewable tablets, sublingual tablets, effervescent tablets, pills, suspensions, powders, granules and gels. At these solid dosage forms, the active compounds can be mixed with at least one inert excipient such as sucrose, lactose or starch. Such dosage forms can also comprise, as in normal practice, additional substances other than inert diluents, e.g. lubricating agents such as magnesium stearate. In the case of capsules, tablets, effervescent tablets and pills, the dosage forms may also comprise buffering agents. Tablets and pills can be prepared with enteric coatings.

Liquid dosage forms for oral administration may include emulsions, solutions, suspensions, syrups and elixirs pharmaceutically acceptable containing inert diluents commonly used in the technique, such as water. Those compositions may also comprise adjuvants such as wetting agents, emulsifying and suspending agents, and sweetening agents, flavoring and perfuming agents.

Injectable preparations, for example, aqueous or oleaginous suspensions, sterile injectable may be formulated according with the technique known using suitable dispersing agents, wetting agents and/or suspending agents. Among the acceptable vehicles and solvents that can be used are water, Ringer's solution and isotonic sodium chloride solution. Sterile oils are also conventionally used as solvents or suspending media.

For topical administration, the pharmaceutical compositions of the invention can be formulated as creams, gels, lotions, liquids, pomades, spray solutions, dispersions, solid bars, emulsions, microemulsions and similars which may be formulated according to conventional methods that use suitable excipients, such as, for example, emulsifiers, surfactants, thickening agents, coloring agents and combinations of two or more thereof.

Additionally, pharmaceutical compositions of the invention may be administered in the form of transdermal patches or iontophoresis devices. In one embodiment, the compounds of the invention are administered as a transdermal patch, for example, in the form of sustained-release transdermal patch. Suitable transdermal patches are described in more detail in, for example, US5262165, US5948433, US6010715 and US6071531.

The pharmaceutical compositions of the invention can additionally include conventional excipients, i.e. pharmaceutically acceptable carriers suitable for parenteral application which do not react damaging with the active compounds. Suitable pharmaceutically acceptable vehicles include, for example, water, salt solutions, alcohol, vegetable oils, polyethylene glycols, gelatin, lactose, amylose, magnesium stearate, talc, surfactants, silicic acid, viscous paraffin, perfume oil, monoglycerides and diglycerides of fatty acids, fatty acid esters petroetrals, hydroxymethyl cellulose, polyvinylpyrrolidone and similars.

Several drug delivery systems are known and can be used to administer the compounds or compositions of the invention, including, for example, encapsulation in liposomes, microbubbles, emulsions, microparticles, microcapsules and similars. The required dosage can be administered as a single unit or in a sustained release form.

Sustainable-release forms and appropriate materials and methods for their preparation are described in, for example, "Modified-Release Drug Delivery Technology", Rathbone, M. J. Hadgraft, J. and Roberts, M. S. (eds.), Marcel Dekker, Inc., New York (2002), "Handbook of Pharmaceutical Controlled Release Technology", Wise, D. L. (ed.), Marcel Dekker, Inc. New York, (2000). In one embodiment of the invention, the orally administrable form of a pharmaceutical composition of the invention is in a sustained release form further comprises at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of the them.

Enteric coatings may be applied using conventional processes known to experts in the art, as described in, for example, Johnson, J. L., "Pharmaceutical tablet coating", Coatings Technology Handbook (Second Edition), Satas, D. and Tracton, A. A. (eds), Marcel Dekker, Inc. New York, (2001), Carstensen, T., "Coating Tablets in Advanced Pharmaceutical Solids", Swarbrick, J. (ed.), Marcel Dekker, Inc. New York (2001), 455-468.

In the particular case that the inhibitor is a nucleic acid (siRNA, antisense oligonucleotides, ribozimes, aptamers and spiegelmers), the pharmaceutical compositions may be formulated as a composition intended for use in gene therapy; by way of illustration and not limitation, the pharmaceutical composition of the invention may contain a viral or nonviral vector, which comprises a polynucleotide of the invention or a gene construction of the invention. By way of illustration and not limitation, said vectors, may be viral, for example, based on retrovirus, adenovirus, etc., or nonviral such as ADN-liposome, ADN-polymer, ADN-polymer-liposome complexes, etc. [see "Nonviral Vectors for Gene Therapy", edited by Huang, Hung and Wagner, Academic Press (1999)]. Said vectors, which contain a polynucleotide or a gene construction of the invention, may be administered directly to humans or animals by conventional methods. Alternatively, said vectors may be used to transform, or transfect or infect cells, for example, mammal cells, including human, *ex vivo,* which subsequently will be implanted into a human body or an animal to obtain the desired therapeutic effect. For administration to a human body or an animal, said cells will be formulated in a suitable medium that will have no adverse influence on cell viability.

Those skilled in the art are familiar with the principles and procedures discussed in widely known and available sources as Remington's Pharmaceutical Science (17th Ed., Mack Publishing Co., Easton, Pa., 1985) and Goodman and Gilman's The Pharmaceutical Basis of Therapeutics (8th Ed., Pergamon Press, Elmsford, N.Y., 1990) both of which are incorporated herein by reference.

For treating cancer, the pharmaceutical compositions of the invention may be administered by any route, including, without limitation, oral, intravenous, intramuscular, intraarterial, intramedullary, intrathecal, intraventricular, transdermal, subcutanous, intraperitoneal, intranasal, enteric, topical, sublingual or rectal route. A review of the different dosage forms of active ingredients and excipients to be used and their manufacturing processes is provided in "Tratado de Farmacia Galénica", C. Faulí and Trillo, Luzán 5, S.A. de Ediciones, 1993 and in Remington's Pharmaceutical Sciences (A.R. Gennaro, Ed.), 20th edition, Williams & Wilkins PA, USA (2000).

Even though individual needs vary, determination of optimal ranges for effective amounts of the compositions of the invention belongs to the common experience of those experts in the art. In general, the dosage needed to provide an effective treatment, which can be adjusted by one expert in the art, will vary depending on age, health, fitness, sex, diet, weight, degree of alteration of the receptor, frequency of treatment and the nature and extent of impairment or illness, medical condition of the patient, route of administration, pharmacological considerations such as activity, efficacy, pharmacokinetic and toxicology profile of the particular compound used, if using a system drug delivery, and if the compound is administered as part of a combination of drugs.

The amount of the composition according to the invention that is effective in the treatment of a particular disorder or condition will depend on the nature of the disorder or condition, and can be determined by conventional clinical techniques, including reference to Goodman and Gilman, supra; The Physician's Desk Reference, Medical Economics Company, Inc., Oradell, NJ, 1995, and Drug Facts and Comparisons, Inc., St. Louis, MO, 1993. The precise dose to

use in the formulation will also depend on the route of administration, and severity of the disease or disorder, and should be decided in the physician's opinion and the patient's circumstances.

*Pharmaceutical Composition [2]*

In another aspect, the invention relates to a pharmaceutical composition comprising a therapeutically effective amount of a composition comprising a Nudix5 inhibitor and at least a second antitumor agent, pharmaceutical composition [2] of the invention.

The amount of Nudix5 inhibitor present in the pharmaceutical composition of the invention may vary within a wide range, however, in a particular embodiment, the weight percentage of Nudix5 inhibitor with respect to the total composition of the invention is of at least 0,10% 0,50%, 1%, 2%, 3%, 4%, 5%, 6%, 7%, 8%, 9%, 10%, 15% 20%, 25%, 30%, 40%, 50%, 60%, 70%, 80%, 90%, or, at least 95%.

The weight ratio of Nudix5 inhibitor and the at least second antitumor agent in the pharmaceutical composition of the invention may vary within a wide range, however, in general, the ratio is chosen, taking into account factors such as the condition being treated, the age, sex, weight, etc., of the subject who receives the inventive composition. Preferably, said weight ratio of Nudix5 inhibitor and the at least second antitumor agent is one that results in a beneficial effect, in particular, an increase in the therapeutic effect of the composition of the invention relative to each of its components so that it can reach the same result with lower doses of each of the components, thereby reducing the side effects on the subject receiving the composition of the invention.

The term "antitumor agent", as used herein, refers to any chemical or biological agent or compound with antiproliferative, antioncogenic and/or carcinostatic properties which can be used to inhibit tumor growth, proliferation and/or development Antitumor agents suitable in the present invention include, without limitation:

(i) a cytotoxic polypeptide,
(ii) an antiangiogenic polypeptide,
(iii) a polypeptide encoded by a tumor suppressor gene,
(iv) pro-apoptotic polypeptides
(v) polipeptides having anti-metastatic activity
(vi) a polypeptide encoded by a polynucleotide which is capable of activating the immune response towards a tumor,
(vii) a tumor suppressor gene,
(viii) a silencing agent,
(ix) a suicide gene,
(x) a polynucleotide which is capable of activating the immune response towards a tumor,
(xi) a chemotherapy agent and
(xii) an antiangiogenic molecule.

*(i)Cytotoxic polypeptides*

As used herein, the term "cytotoxic polypeptide" refers to an agent that is capable of inhibiting cell function. The agent may inhibit proliferation or may be toxic to cells. Any polypeptide that when internalized by a cell interfere with or detrimentally alter cellular metabolism or in any manner inhibit cell growth or proliferation are included within the ambit of this term, including, but are not limited to, agents whose toxic effects are mediated when transported into the cell and also those whose toxic effects are mediated at the cell surface. Useful cytoxic polypeptides include proteinaceous toxins and bacterial toxins.

Examples of proteinaceous cell toxins useful for incorporation into the conjugates according to the invention include, but are not limited to, type one and type two ribosome inactivating proteins (RIP). Useful type one plant RIPs include, but are not limited to, dianthin 30, dianthin 32, lychnin, saporins 1-9, pokeweed activated protein (PAP), PAP II, PAP-R, PAP-S, PAP-C, mapalmin, dodecandrin, bryodin-L, bryodin, Colicin 1 and 2, luffin-A, luffin-B, luffin-S, 19K-protein synthesis inhibitory protein (PSI), 15K-PSI, 9K-PSI, alpha-kirilowin, beta-kirilowin, gelonin, momordin, momordin-II, momordin-Ic, MAP-30, alpha-momorcharin, beta-momorcharin, trichosanthin, TAP-29, trichokirin; barley RIP; flax RIP, tritin, corn RIP, Asparin 1 and 2 (Stirpe et al., Bio/Technology 10:405-12, 1992). Useful type two RIPs include, but are not limited to, volkensin, ricin, nigrin-b, CIP-29, abrin, modeccin, ebulitin-[alpha], ebulitin-[beta], ebultin-[gamma], vircumin, porrectin, as well as the biologically active enzymatic subunits thereof (Stirpe et al., Bio/Technology 10:405-12, 1992; Pastan et al., Annu. Rev. Biochem. 61:331-54; Brinkmann and Pastan, Biochim. et Biophys. Acta 1198:27-45, 1994; and Sandvig and Van Deurs, Physiol. Rev. 76:949-66, 1996).

Examples of bacterial toxins useful as cell toxins include, but are not limited to, shiga toxin and shiga-like toxins (i.e., toxins that have the same activity or structure), as well as the catalytic subunits and biologically functional fragments thereof. These bacterial toxins are also type two RIPs (Sandvig and Van Deurs, Physiol. Rev. 76:949-66, 1996; Armstrong,

J. Infect. Dis., 171:1042-5, 1995; Kim et al., Microbiol. Immunol. 41:805-8, 1997, and Skinner et al., Microb. Pathog. 24:117-22, 1998). Additional examples of useful bacterial toxins include, but are not limited to, Pseudomonas exotoxin and Diphtheria toxin (Pastan et al., Annu. Rev. Biochem. 61:331-54; and Brinkmann and Pastan, Biochim. et Biophys. Acta 1198:27-45, 1994). Truncated forms and mutants of the toxin enzymatic subunits also can be used as a cell toxin moiety (Pastan et al., Annu. Rev. Biochem. 61:331-54; Brinkmann and Pastan, Biochim. et Biophys. Acta 1198:27-45, 1994; Mesri et al., J. Biol. Chem. 268:4852-62, 1993; Skinner et al., Microb. Pathog. 24:117-22, 1998; and U.S. Pat. No. 5,082,927). Other targeted agents include, but are not limited to the more then 34 described Colicin family of RNase toxins which include colicins A, B, D, E1-9, cloacin DF13 and the fungal RNase, [alpha]-sarcin (Ogawa et al. Science 283: 2097-100, 1999; Smarda et al., Folia Microbiol (Praha) 43:563-82, 1998; Wool et al., Trends Biochem. Sci., 17: 266-69, 1992).

*(ii) Antiangiogenic peptides and polypeptides*

Proliferation of tumors cells relies heavily on extensive tumor vascularization, which accompanies cancer progression. Thus, inhibition of new blood vessel formation with anti-angiogenic agents and targeted destruction of existing blood vessels have been introduced as an effective and relatively non-toxic approach to tumor treatment.

The term "antiangiogenic polypeptide", as used herein, denotes a polypeptide capable of inhibiting angiogenesis. Suitable antiangiogenic polypeptides include, without limitation, angiostatin, endostatin, anti-angiogenic antithrombin III, sFRP- 4 as described in WO2007115376, an anti-VEGF antibody such as anibizumab, bevacizumab (avastin), Fab IMC 1121 and F200 Fab.

*(iii) Polypeptides encoded by tumor suppressor genes,*

As used herein, a "tumor suppressor" is a gene or gene product that has a normal biological role of restraining unregulated growth of a cell. The functional counterpart to a tumor suppressor is an oncogene. Genes that promote normal cell growth may be known as "protooncogenes". A mutation that activates such a gene or gene product further converts it to an "oncogene", which continues the cell growth activity, but in a dysregulated manner Examples of tumor suppressor genes and gene products are well known in the literature and may include PTC, BRCA1, BRCA2, p16, APC, RB, WTI, EXT1, p53, NF1, TSC2, NF2, VHL,ST7, ST14, PTEN, APC, CD95 or SPARC.

*(iv) Pro-apoptotic polypeptides*

The term "pro-apoptotic polypeptides", as used herein, refers to a protein which is capable of inducing cell death in a cell or cell population. Suitable pro-apoptotic polypeptides include, without limitation, proapoptotic members of the BCL-2 family of proteins such as BAX, BAK, BOK/MTD, BID, BAD, BIK/NBK, BLK, HRK, BIM/BOD, BNIP3, NIX, NOXA, PUMA, BMF, EGL-I, and viral homologs, caspases sich as caspase-8, the adenovirus E4orf4 gene, p53 pathway genes, proapoptotic ligands such as TNF, FasL, TRAIL and/or their receptors, such as TNFR, Fas, TRAIL-R1 and

TRAIL-R2.

*(v) Polypeptides having anti-metastatic activity*

The term "metastasis suppressor" as used herein, refers to a protein that acts to slow or prevent metastases (secondary tumors) from spreading in the body of an organism with cancer. Suitable metastasis suppressor include, without limitation, proteins such as BRMS 1, CRSP3, DRG1, KAI1, KISS-1, NM23, a TIMP-family protein and uteroglobin.

*(vi) Immunostimulatory polypeptides*

As used herein, an immunostimulatory polypeptide agent is a polypeptide that stimulates an immune response (including enhancing a pre-existing immune response) in a subject to whom it is administered, whether alone or in combination with another agent, flagellin, muramyl dipeptide), cytokines including interleukins (e.g., IL-2, IL-7, IL- 15 (or superagonist/mutant forms of these cytokines), IL-12, IFN-gamma, IFN-alpha, GM-CSF, FLT3-ligand, etc.), immunostimulatory antibodies (e.g., anti-CTLA-4, anti-CD28, anti-CD3, or single chain/antibody fragments of these molecules), and the like.

*(vii) Tumor suppressor genes*

Suitable tumor suppressor genes include a gene or gene product that encodes any of the polypeptides defined above. Examples of tumor suppressor genes and gene products are well known in the literature and may include PTC,

BRCA1, BRCA2, p16, APC, RB, WTI, EXT1, p53, NF1, TSC2, NF2, VHL,ST7, ST14, PTEN, APC, CD95 or SPARC.

*(viii) Silencing agents*

Wherein the nucleic acid is a silencing agent, said silencing agent is aimed at blocking the expression of genes the over-expression of which leads to cell proliferation and tumor growth. Genes that can be inhibited by the conjugates according to the invention carrying silencing agent include, without limitation, HRAS (v-Ha-ras Harvey rat sarcoma viral oncogene homolog), NRAS (neuroblastoma RAS viral (v-ras) oncogene homolog), KRAS (v-Ki-ras2 Kirsten rat sarcoma viral oncogene homolog), MYC (v-myc myelocytomatosis viral oncogene homolog, avian), MYCN (v-myc myelocytomatosis viral related oncogene, neuroblastoma derived, avian), MYB (v-myb myeloblastosis viral oncogene homolog, avian), Jun-oncogene, FOS (v-fos FBJ murine osteosarcoma viral oncogene homolog), Oncogenic ABL1 (v-abl Abelson murine leukemia viral oncogene homolog 1 (this gene is an oncogene only if the SH3 domain is truncated, as happens regularity in certain leukemias), SRC (v-src sarcoma (Schmidt-Ruppin A-2) viral oncogene homolog, avian), FES (Feline sarcoma oncogene), RAF1(v-raf-1 murine leukemia viral oncogene homolog 1), REL (v-rel reticuloendotheliosis viral oncogene homolog, avian), RELA (v-rel reticuloendotheliosis viral oncogene homolog A, nuclear factor of kappa light polypeptide gene enhancer in B-cells 3, p65, avian), RELB (v-rel reticuloendotheliosis viral oncogene homolog B, nuclear factor of kappa light polypeptide gene enhancer in B-cells 3, avian), FGR (Gardner-Rasheed feline sarcoma viral (v-fgr) oncogene homolog), and KIT (v-kit Hardy-Zuckerman 4 feline sarcoma viral oncogene homolog).

Other silencing agents suitable for use in the present invention include those directed against cyclin Gl, cyclin Dl, Bcl-2 (B-cell chronic lymphatic leukemia associated protein 2), Bcl-XL (Bc1-2 related gene xl), HLA-G (Human leukocyte antigen class G), IGF-1 (Insulin-like growth factor-1), EGF (epidermal growth factor), FGF (fibroblast growth factor), VEGF (vascular endothelial growth factor), VEGFR (vascular endothelial growth factor receptor), IGFR (insulin-like growth factor receptor), EGFR (epidermal growth factor receptor), FGFR (fibroblast growth factor receptor), TGF-beta (transforming growth factor beta), Caspase 3, CEACAM6 (Carcinoembryonic antigen-related cell adhesion molecule 6), HPV-E6 (human papiloma virus protein E6), HPV-E7 (human papiloma virus protein E7), H-Ras gene, P100a gene, CREB (cAMP response element binding), BRAF gene, ATF2 (activating transcription factor 2), HER2 (Human EGF-like Receptor No. 2), and N-myc.

Examples of therapeutic molecules include, but are not limited to, cell cycle control agents; agents which inhibit cyclin proteins, such as antisense polynucleotides to the cyclin Gl and cyclin Dl genes.

*(ix) Suicide genes*

The term "<u>suicide gene</u>" refers to a product that causes cell death by itself or in the presence of other compounds. A representative example of a suicide gene is one, which codes for thymidine kinase of herpes simplex virus. Additional examples are thymidine kinase of varicella zoster virus and the bacterial gene cytosine deaminase, which can convert 5-fluorocytosine to the highly cytotoxic compound 5-fluorouracil.

Suicide genes may produce cytotoxicity by converting a prodrug to a product that is cytotoxic. In one embodiment, the term "prodrug" means any compound that can be converted to a toxic product for cells. Representative example of such a prodrug is gancyclovir which is converted in vivo to a toxic compound by HSV-thymidine kinase. The gancyclovir derivative subsequently is toxic to cells. Other representative examples of prodrugs include acyclovir, FIAU [1-(2-deoxy-2-fluoro-beta-D-arabinofuranosyl)-5-iodouracil], 6-methoxypurine arabinoside for VZV-TK, and 5-fluorocytosine for cytosine deaminase.

*(x) Polynucleotides capable of activating the immune response towards a tumor*

Suitable polynucleotides capable of activating the immune response towards a tumor include a gene that encodes any immunostimulatory polypeptide agent as defined above.

*(xi) Chemotherapy agents*

As used herein, an anti-cancer agent is an agent that at least partially inhibits the development or progression of a cancer, including inhibiting in whole or in part symptoms associated with the cancer even if only for the short term. Several anti-cancer agents can be categorized as DNA damaging agents and these include topoisomerase inhibitors (e.g., etoposide, ramptothecin, topotecan, teniposide, mitoxantrone), DNA alkylating agents (e.g., cisplatin, mechlorethamine, cyclophosphamide, ifosfamide, melphalan, chorambucil, busulfan, thiotepa, carmustine, lomustine, carboplatin, dacarbazine, procarbazine), DNA strand break inducing agents (e.g., bleomycin, doxorubicin, daunorubicin, idarubicin, mitomycin C), anti-microtubule agents (e.g., vincristine, vinblastine), anti-metabolic agents (e.g., cytarabine, methotrexate, hydroxyurea, 5-fluorouracil, floxuridine, 6-thioguanine, 6-mercaptopurine, fludarabine, pentostatin, chlorodeoxyadenosine), anthracyclines, vinca alkaloids, or epipodophyllotoxins.

Examples of anti-cancer agents include without limitation Acivicin; Aclarubicin; Acodazole Hydrochloride; Acronine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Bortezomib (VELCADE); Brequinar Sodium; Bropirimine; Busulfan; Cactinomycin; Calusterone; Caracemide; Carbetimer; Carboplatin (a platinum- containing regimen); Carmustine; Carubicin Hydrochloride; Carzelesin; Cedefingol; Chlorambucil; Cirolemycin; Cisplatin (a platinum-containing regimen); Cladribine; Crisnatol Mesylate; Cyclophosphamide; Cytarabine; Dacarbazine; Dactinomycin; Daunorubicin; Decitabine; Dexormaplatin; Dezaguanine; Diaziquone; Docetaxel (TAXOTERE); Doxorubicin; Droloxifene; Dromostanolone; Duazomycin; Edatrexate; Eflornithine; Elsamitrucin; Enloplatin; Enpromate; Epipropidine; Epirubicin; Erbulozole; Erlotinib (TARCEVA), Esorubicin; Estramustine; Etanidazole; Etoposide; Etoprine; Fadrozole; Fazarabine; Fenretinide; Floxuridine; Fludarabine; 5-Fluorouracil; Flurocitabine; Fosquidone; Fostriecin; Gefitinib (IRESSA), Gemcitabine; Hydroxyurea; Idarubicin; Ifosfamide; Ilmofosine; Imatinib mesylate (GLEEVAC); Interferon alpha-2a; Interferon alpha-2b; Interferon alpha-n1; Interferon alpha-n3; Interferon beta-I a; Interferon gamma-I b; Iproplatin; Irinotecan; Lanreotide; Lenalidomide (REVLLM1D, REVIMID); Letrozole; Leuprolide; Liarozole; Lometrexol; Lomustine; Losoxantrone; Masoprocol; Maytansine; Mechlorethamine; Megestrol; Melengestrol; Melphalan; Menogaril; Mercaptopurine; Methotrexate; Metoprine; Meturedepa; Mitindomide; Mitocarcin; Mitocromin; Mitogillin; Mitomalcin; Mitomycin; Mitosper; Mitotane; Mitoxantrone; Mycophenolic Acid; Nocodazole; Nogalamycin; Ormaplatin; Oxisuran; Paclitaxel; Pemetrexed (ALIMTA), Pegaspargase; Peliomycin; Pentamustine; Pentomone; Peplomycin; Perfosfamide; Pipobroman; Piposulfan; Piritrexim Isethionate; Piroxantrone; Plicamycin; Plomestane; Porfimer; Porfiromycin; Prednimustine; Procarbazine; Puromycin; Pyrazofurin; Riboprine; Rogletimide; Safingol; Semustine; Simtrazene; Sitogluside; Sparfosate; Sparsomycin; Spirogermanium; Spiromustine; Spiroplatin; Streptonigrin; Streptozocin; Sulofenur; Talisomycin; Tamsulosin; Taxol; Taxotere; Tecogalan; Tegafur; Teloxantrone; Temoporfin; Temozolomide (TEMODAR); Teniposide; Teroxirone; Testolactone; Thalidomide (THALOMID) and derivatives thereof; Thiamiprine; Thioguanine; Thiotepa; Tiazofurin; Tirapazamine; Topotecan; Toremifene; Trestolone; Triciribine; Trimetrexate; Triptorelin; Tubulozole; Uracil; Mustard; Uredepa; Vapreotide; Verteporfin; Vinblastine; Vincristine; Vindesine; Vinepidine; Vinglycinate; Vinleurosine; Vinorelbine; Vinrosidine; Vinzolidine; Vorozole; Zeniplatin; Zinostatin; Zorubicin.

The antitumor agent may be an enzyme inhibitor including without limitation tyrosine kinase inhibitor, a CDK inhibitor, a MAP kinase inhibitor, or an EGFR inhibitor. The tyrosine kinase inhibitor may be without limitation Genistein (4', 5, 7-trihydroxyisoflavone), Tyrphostin 25 (3,4,5-trihydroxyphenyl), methylene]-propanedinitrile, Herbimycin A, Daidzein (4',7-dihydroxyisoflavone), AG-126, trans-1-(3'-carboxy-4'-hydroxyphenyl)-2-(2'',5''-dihydroxy-phenyl)ethane, or HDBA (2-Hydroxy5-(2,5-Dihydroxybenzylamino)-2-hydroxybenzoic acid. The CDK inhibitor may be without limitation p21, p27, p57, p15, p16, p18, or pl9. The MAP kinase inhibitor may be without limitation KY12420 ($C_{23}H_{24}O_8$), CNI-1493, PD98059, or 4-(4-Fluorophenyl)-2-(4-methylsulfinyl phenyl)-5-(4-pyridyl) 1H-imidazole. The EGFR inhibitor may be without limitation erlotinib (TARCEVA), gefitinib (IRESSA), WHIP97 (quinazoline derivative), LFM-A12 (leflunomide metabolite analog), ABX-EGF, lapatinib, canertinib, ZD-6474 (ZACTIMA), AEE788, and AG1458.

The antitumor cancer agent may be a VEGF inhibitor including without limitation bevacizumab (AVASTIN), ranibizumab (LUCENTIS), pegaptanib (MACUGEN), sorafenib, sunitinib (SUTENT), vatalanib, ZD-6474 (ZACTIMA), anecortave (RETAANE), squalamine lactate, and semaphorin. The anti-cancer agent may be an antibody or an antibody fragment including without limitation an antibody or an antibody fragment including but not limited to bevacizumab (AVASTIN), trastuzumab (HERCEPTIN), alemtuzumab (CAMPATH, indicated for B cell chronic lymphocytic leukemia,), gemtuzumab (MYLOTARG, hP67.6, anti-CD33, indicated for leukemia such as acute myeloid leukemia), rituximab (RITUXAN), tositumomab (BEXXAR, anti-CD20, indicated for B cell malignancy), MDX-210 (bispecific antibody that binds simultaneously to HER-2/neu oncogene protein product and type I Fc receptors for immunoglobulin G (IgG) (Fc gamma RI)), oregovomab (OVAREX, indicated for ovarian cancer), edrecolomab (PANOREX), daclizumab (ZENAPAX), palivizumab (SYNAGIS, indicated for respiratory conditions such as RSV infection), ibritumomab tiuxetan (ZEVALIN, indicated for Non-Hodgkin's lymphoma), cetuximab (ERBITUX), MDX-447, MDX-22, MDX-220 (anti-TAG-72), I0R-C5, 10R-T6 (anti-CD 1), IOR EGF/R3, celogovab (ONCOSCINT OV 103), epratuzumab (LYMPHOCIDE), pemtumomab (THERAGYN), and Gliomab-H (indicated for brain cancer, melanoma).

Other suitable active agents are DNA cleaving agents. Examples of DNA cleaving agents suitable for inclusion in the compositions of the invention include, but are not limited to, anthraquinone-oligopyrrol-carboxamide, benzimidazole, leinamycin; dynemycin A; enediyne; as well as biologically active analogs or derivatives thereof (i.e., those having a substantially equivalent biological activity). Known analogs and derivatives are disclosed, for examples in Islam et al., J. Med. Chem. 34 2954-61, 1991; Skibo et al., J. Med. Chem. 37:78-92, 1994; Behroozi et al., Biochemistry 35:1568-74, 1996; Helissey et al., Anticancer Drug Res. 11:527-51, 1996; Unno et al., Chem. Pharm. Bull. 45:125-33, 1997; Unno et al., Bioorg. Med. Chem., 5:903-19, 1997; Unno et al., Bioorg. Med. Chem., 5: 883-901, 1997; and Xu et al., Biochemistry 37:1890-7, 1998). Other examples include, but are not limited to, endiyne quinone imines (U.S. Pat. No. 5,622,958); 2,2r-bis (2-aminoethyl)-4-4'-bithiazole (Lee et al., Biochem. Mol. Biol. Int. 40:151-7, 1996); epilliticine-salen.copper conjugates (Routier et al., Bioconjug. Chem., 8: 789-92, 1997).

*(xii) Antiangiogenic molecules*

These agents include, in addition to the anti-angiogenic polypeptides mentioned above, Marimastat; AG3340; COL-3, BMS-275291, Thalidomide, Endostatin, SU5416, SU6668, EMD121974, 2-methoxyoestradiol, carboxiamidotriazole, CM1O1, pentosan polysulphate, angiopoietin 2 (Regeneron), herbimycin A, PNU145156E, 16K prolactin fragment, Linomide, thalidomide, pentoxifylline, genistein, TNP470, endostatin, paclitaxel, accutin, angiostatin, cidofovir, vincristine, bleomycin, AGM- 1470, platelet factor 4 or minocycline.

In a preferred embodiment, the second antitumor agent is selected from a PARP1 inhibitor, a PARG inhibitor and a BRCA1 inhibitor.

"PARP1", as used herein refers to Poly [ADP-ribose] polymerase 1 also known as NAD+ ADP-ribosyltransferase 1 or poly [ADP-ribose] synthase 1. It has a role in repair of single-stranded DNA (ssDNA) breaks. The sequence of PARP1 protein in humans corresponds to the sequence P09874 in the Uniprot database 27 March 2014.

"PARP1 inhibitor", as used herein, relates to any compound capable of causing a decrease in the activity of PARP1, including those compounds which prevent expression of PARP1 gene and compounds which lead to reduced PARP1 mRNA or protein levels.

Methods for detecting reduced mRNA and protein levels have been previously disclosed in relation to Nudix5 and are equally applicable to PARP1.

A PARP1 inhibitor may inhibit PARP1 activity by at least 5%, at least 10%, at least 25%, at least 50%, at least 75%, or at least 90%, and all ranges between 5% and 100%. Suitable methods for determining whether an inhibitor acts by decreasing the activity of PARP1 include any method which allows detect the consumption of the substrate nicotinamide adenine dinucleotide (NAD+) or the formation of any of these three products, namely, polymer of ADP-ribose (pADPr or PAR), nicotinamide, and protons. Methods for detecting PARP1 activity are known in the art and include without limitations the methods disclosed in Shah G. et al., Methods in Molecular Biology. vol. 780, 2011,Pags 3-34, Putt KS et al., Anal Biochem. 2004 Mar 1;326(1):78-86

In a preferred embodiment the PARP1 inhibitor is selected from Table 2.

| TABLE 2: PARP1 INHIBITORS SUITABLE FOR USE ACCORDING TO THE INVENTION | |
|---|---|
| I | BMN 673 (Lt-673), Rucaparib (CO-338, AG014699, PF-0367338), Olaparib (AZD-2281), veliparib (ABT-888) MK-4827, BMN-673, CEP-9722, E7016 (GI 21016), INO-1001, LT-673, MP-124, NMS-P118, XAV939 as disclosed in Kummar et al., MNC Medicine 2012 10:25 and Davar D. et al Curr Med Chem 2012 1;19 (23):3907-3921 |
| II | An inhibitor that interacts with the DNA binding domain of PARP-1, acting as a non-competitive inhibitor of PARP-1, such as Iniparib (BSI-201) |
| III | nicotinamide; NU1025; 3-aminobenzamide; 4-amino-1,8-naphthalimide; 1,5-isoquinolinediol; 6(5H)-phenanthriddinone; 1,3,4,5,-tetrahydrobenzo(c)(1,6)- and (c)(1,7)-naphthyridin-6-ones; adenosine substituted 2,3-dihydro-1H-isoindol-1-ones; AG14361; AGO14699; 2-(4-chlorophenyl)-5-quinoxalinecarboxamide; 5-chloro-2-[3-(4-phenyl-3,6-dihydro-1(2H)-pyridinyl) propyl]-4(3H)-quinazolinone; isoindolinone derivative INO-1001; 4-hydroxyquinazo line; 2-[3-[4-(4-chlorophenyl)-1-piperazinyl]propyl]-4-3(4)-quinazolinone; 1,5-dihydroxyisoquinoline (DHIQ); 3,4-dihydro-5[4-(1-piperidinyl)(butoxy)-1(2H)-isoquinolone; CEP-6800; GB-15427; PJ34; DPQ; BS-201; AZD2281; BS401; CHP101; CHP102; INH2BP; BSI201; BSI401; TIQ-A; and imidazobenzodiazepines as disclosed in US 20090170860 A1 |
| IV | An interference RNA specific for the PARP1 sequence |
| V | An antisense oligonucleotide specific for the PARP1 sequence |
| VI | A ribozyme or DNA enzyme specific for the PARP1 sequence |
| VII | Inhibitory antibodies which bind specifically to PARP1 and inhibiting PARP1 activity. |
| VIII | Aptamers and spiegelmers. |

The terms "interference RNA specific for a sequence", "antisense oligonucleotide specific for a sequence", "a ribozyme or DNA enzyme specific for a sequence" and "inhibitory antibodies that bind specifically to a protein and inhibit the activity of a protein" , "aptamers and spiegelmers" and their embodiments have been explained previously in relation

to Nudix5 and equally apply to PARP1.

"PARPG" as used herein refers to Poly (ADP-ribose) glycohydrolase, the major enzyme responsible for the catabolism of poly (ADP-ribose). The sequence of PARG protein in humans corresponds to the sequence Q86W56 in the Uniprot database 27 March 2014.

"PARG inhibitor", as used herein, relates to any compound capable of causing a decrease in the activity of PARG, including those compounds which prevent expression of PARG gene and compounds which lead to reduced PARG mRNA or protein levels.

Method for detecting reduced mRNA and protein levels and its embodiments have been previously disclosed in relation to Nudix5 and are equally applicable to PARP1.

In a preferred embodiment the PARG inhibitor is selected from Table 3.

| TABLE 3: PARG INHIBITORS SUITABLE FOR USE ACCORDING TO THE INVENTION | |
|---|---|
| I | A compound having structure FX1 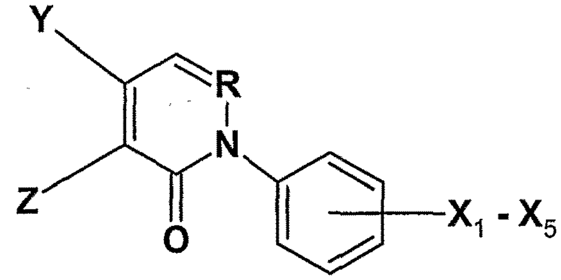 wherein R is C or N;<br>Y is NH2, NR1R2, halide or acyl, Ri and R2 are alkyl groups ranging from between one and about ten carbons inclusive, Ri and R2 can be alkyl groups where two alkyl groups form a cyclic moiety having 5, 6, 7 or 8 ring members where one ring carbon can be O, hydrogen, aryl, substituted alkyl where one or more substituents are halides, alkyl aryl;<br>Z is NH2, NR4R5, halide, a group that is converted under physiological<br>Of I<1 p conditions to NH2; R4 and/or R5 can have values of Ri and R2 , 5 , where R6 is H or small alkyl; and<br>Xi - X5 = hydrogen, halide, alkyl, alkylhalide, OH, OCH3, OR7, where R7 is alkyl; as disclosed in WO 2007014226 A2 |
| II | ADP-ribose analogues, such as ADP-(hydroxymethyl)pyrrolidinediol (ADP-HPD),N-bis-(3-phenyl-propyl)9-oxo-fluorene-2,7-diamide (GPI 16552) |
| III | DNA intercalators |
| IV | Tannins such as Gallotannin (GLTN), Fathers C. et al., Cell Cycle 11:5, 990-997; March 1, 2012 and galloyl-containing molecules, particularly 3-galloyl-glucose, 3-galloyl-O-methyl-glucose and 2-galloyl-glucose, as disclosed in Formentinin L. et al., Br J Pharmacol. Dec 2008; 155(8): 1235-1249 and ethacridine |
| V | PAR containing the etheno moiety, Shirato M et al., Biochem Biophys Res Commun. 2007 Apr 6;355(2):451-6 |
| VI | An interference RNA specific for the PARG sequence |
| VII | An antisense oligonucleotide specific for the PARG sequence |
| VIII | A ribozyme or DNA enzyme specific for the PARGsequence |
| IX | Inhibitory antibodies that bind specifically to PARG and inhibit PARG activity. |

The terms "interference RNA specific for a sequence", "antisense oligonucleotide specific for a sequence", "ribozyme or DNA enzyme specific for a sequence" and "inhibitory antibodies that bind specifically to a protein and inhibit the activity of a protein" and their embodiments have been explained previously in relation to Nudix5 and equally apply to PARG.

A PARG inhibitor may inhibit PARG activity by at least 5%, at least 10%, at least 25%, at least 50%, at least 75%, or at least 90%, and all ranges between 5% and 100%. Suitable methods for determining whether an inhibitor acts by decreasing the PARG activity include any method which allows determining the hydrolization of PAR (polyADP-ribose). Several methods are known in the art, such as the methods disclosed in Putt KS et al Analytical Biochemistry

326 (2004) 78-86, and commercial kits such as Universal PARG Assay Kits from Trevigen®.

"BRCA1", as used herein refers to breast cancer 1, early onset, and is part of a complex that repairs double-strand breaks in DNA. BRCA1 acts in the same relevant DNA repair pathway as PARP1/PARG. The sequence of BRCA1 protein in humans corresponds to the sequence P38398 in the Uniprot database 7 April 2014.

"BRCA1 inhibitor", as used herein , relates to any compound capable of causing a decrease in the activity of BRCA1, including those compounds which prevent expression of BRCA1 gene and compounds which lead to reduced BRCA1 mRNA or protein levels.

Methods for detecting reduced mRNA and protein levels have been previously disclosed in relation to Nudix5 and are equally applicable to BRCA1.

A BRCA1 inhibitor may inhibit BRCA1 activity by at least 5%, at least 10%, at least 25%, at least 50%, at least 75%, or at least 90%, and all ranges between 5% and 100%. Suitable methods for determining whether an inhibitor acts by decreasing the activity of BRCA1 include any method for assaying transcriptional regulation, mRNA polyadenylation, chromatin remodeling and ubiquitination. (Carvalho M. et al., Int J Biochem Cell Biol. 2007; 39(2): 298-310).

The transcription activation assay was derived from observations indicating that the carboxy-terminal region of BRCA1 had the ability to function as a transactivation domain when expressed as a fusion to a heterologous DNA binding domain (DBD. Quantitation of the reporter gene product or its activity permits an indirect assessment of the transcriptional activity mediated by the BRCA1 fusion protein

In a preferred embodiment, the BRCA1 inhibitor is selected from Table 4.

| TABLE 4: BRCA1 INHIBITORS SUITABLE FOR USE ACCORDING TO THE INVENTION | |
|---|---|
| I | BRCA1 inhibitors that target the BRCT(BRCA1)-phosphoprotein interaction and mimics the M177R/K BRCA1 mutation as disclosed in Pesseto Z.Y. et al., Breast Cancer Res Treat. Jul 2012; 134(2): 511-517. |
| | PI3K inhibitors that impairs BRCA1 expression and sensitize to PARP inhibition as disclosed in Ibrahim Y. et al., Cancer Discovery November 2012 2; 1036 |
| III | An interference RNA specific for the BRCA1 sequence |
| IV | An antisense oligonucleotide specific for the BRCA11 sequence |
| V | A ribozyme or DNA enzyme specific for the BRCA1 sequence |
| VI | Inhibitory antibodies which bind specifically to BRCAland inhibiting BRCA1 activity. |
| VII | Aptamers and spiegelmers. |

The terms "interference RNA specific for a sequence", "antisense oligonucleotide specific for a sequence", "a ribozyme or DNA enzyme specific for a sequence" and "inhibitory antibodies that bind specifically to a protein and inhibit the activity of a protein" , "aptamers and spiegelmers" and their embodiments have been explained previously in relation to Nudix5 and equally apply to BRCA1.

The combination therapy containing Nudix5 inhibitor and at least a second antitumor agent may be complemented with other antitumor agents. In a preferred embodiment, it is contemplated a Nudix5 inhibitor in combination with a PARP1 inhibitor and/or with a PARG1 inhibitor and/or with a BRCA1 inhibitor. In a preferred embodiment, the combination therapy is selected form a composition containing a Nudix5 inhibitor and PARP1 inhibitor; a Nudix5 inhibitor and a PARG inhibitor; a Nudix5 inhibitor and a BRCA1 inhibitor; a Nudix5 inhibitor, a PARP1 inhibitor and a PARG inhibitor; a Nudix5 inhibitor, a PARP1 inhibitor and a BRCA1 inhibitor; a Nudix5 inhibitor, a PARG inhibitor and a BRCA1 inhibitor; or a Nudix5 inhibitor, a PARP1 inhibitor, a PARG inhibitor and a BRCA1 inhibitor.

Thus, the invention contemplates the combined use of Nudix5 inhibitor as above described and one or more additional antitumor agents.

These additional antitumor agents may form part of the composition or, alternatively, may be formulated as a separate composition for its administration at the same time (simultaneous administration) as the compositions of the invention or at different times (sequential administration) with regard to the composition provided by this invention.

The terms "Nudix5 inhibitor", "second antitumor agent", "treatment", "cancer" and "subject" and their embodiments, have been previously described an are equally applicable to the pharmaceutical composition [2].

The particulars of the pharmaceutical composition [1] are equally applicable to the pharmaceutical composition [2] of the invention.

*Non-pharmaceutical compositions*

In an aspect, the invention relates to a composition comprising a Nudix5 inhibitor and at least a second antitumor agent (non-pharmaceutical composition of the invention).

The term "composition", as used herein, relates to a material composition that comprises at least two components, as well as any product resulting, directly or indirectly, from the combination of the different components in any quantity thereof. Those skilled in the art will observe that the composition may be formulated as a single formulation or may be presented as separate formulations of each of the components, which may be combined for joint use as a combined preparation. The composition may be a kit-of-parts wherein each of the components is individually formulated and packaged.

The amount of Nudix5 inhibitor present in the compositions of the invention may vary within a wide range, however, in a particular embodiment, the weight percentage of Nudix5 inhibitor with respect to the total composition of the invention is of at least 0,10% 0,50%, 1%, 2%, 3%, 4%, 5%, 6%, 7%, 8%, 9%, 10%, 15% 20%, 25%, 30%, 40%, 50%, 60%, 70%, 80%, 90%, or, at least 95%.

The weight ratio of Nudix5 inhibitor and the at least second antitumor agent in the composition of the invention may vary within a wide range, however, in general, the ratio is chosen, taking into account factors such as the condition being treated, the age, sex, weight, etc., of the subject who receives the inventive composition. Preferably, said weight ratio of Nudix5 inhibitor and the at least second antitumor agent is one that results in a beneficial effect, in particular, an increase in the therapeutic effect of the composition of the invention relative to each of its components so that it can reach the same result with lower doses of each of the components, thereby reducing the side effects on the subject receiving the composition of the invention.

The terms "Nudix5 inhibitor", "second antitumor agent", and their embodiments, have been previously described and are equally applicable to the non-pharmaceutical composition of the invention.

*Medical use of the non-pharmaceutical composition of the invention*

In another aspect, the invention relates to the use of a composition comprising a Nudix5 inhibitor and at least a second antitumor agent for use in the treatment and/or prevention of cancer in a subject.

The terms "Nudix5 inhibitor", "second antitumor agent", "treatment", "cancer" and "subject" and their embodiments, have been previously described an are equally applicable to the medical use of the non-pharmaceutical composition of the invention.

The particulars of the medical use previously describe are equally applicable to the medical use of the non-pharmaceutical composition of the invention.

*Methods for the treatment and/or prevention of cancer*

In another aspect the invention relates to a method for treating and/or preventing cancer in a subject comprising administering a Nudix5 inhibitor to a subject in need thereof.

In another aspect, the invention relates to a method for treating and/or preventing cancer in a subject comprising administering a pharmaceutical composition [1] of the invention, in a subject in need thereof. The pharmaceutical composition [1] of the invention comprises a therapeutically effective amount of a Nudix5 inhibitor and a pharmaceutically acceptable excipient or carrier

In another aspect, the invention relates to a method for treating and/or preventing cancer in a subject comprising administering a pharmaceutical composition [2] of the invention, in a subject in need thereof. The pharmaceutical composition [2] of the invention comprises a therapeutically effective amount of a composition comprising a Nudix5 inhibitor and at least a second antitumor agent and a pharmaceutically acceptable excipient or carrier.

The terms "Nudix5 inhibitor", "antitumor agent", "treatment", "cancer", "subject" and their embodiments have been previously described an are equally applicable to the method for the treatment and/or prevention of cancer.

The particulars of the pharmaceutical composition [1] and the pharmaceutical composition [2] of the invention are equally applicable to the methods of treatment and/or prevention cancer comprising administering said pharmaceutical compositions.

*Method for assaying the activity of Nudix5*

In another aspect, the invention relates to a method for assaying the activity of Nudix5 comprising assaying the formation of ATP.

In a particular aspect, the assay comprises the steps of

a) contacting a substrate with ADPR and PPi in the presence of Nudix5,
b) reacting the ADRP and PPi with a luminescence reagent under conditions such that the reagent in reduced state is converted to an oxidized state with fluorescence emission, and
c) determining the activity of the enzyme based on the fluorescence emitted by the reagent in the reduced state.

In some embodiments, the measurement of ATP generation via ADPR degradation can be measured base on Roche ATp Bioluminescen Assay kit II.

In a particular aspect, step b) is perfomed by incubating all the components at 37°C for 10-40 minutes, preferably 20 minutes.

As a person skilled in the art knows, the ATP concentration may be calculated using ATP standard curves.

In some embodiments, the ATP formed is detected with a detection system other than the luciferase-luciferin reaction. In a commercially available ATP kit (#366-A, Sigma, St. Louis, MO), a combination of two enzymes, phosphoglycerate kinase and glyceraldehyde phosphate dehydrogenase, are used to catalyze the formation of NAD from NADH in the presence of the ATP formed, which is detected as a decrease in NADH-dependent UV absorbance (340 nm) or fluorescence emission (460 nm), as described further in U.S. Patent No. 6,335,162,. Another detection system for the ATP formed is based on the use of a FAD synthetase-active system in conjunction with FMN to generate FAD. This detection system is fully described in U.S. Patent No. 4,806,415.

In preferred embodiments, the ATP formed is detected by the luciferase-luciferin reaction. In the presence of ATP and $O_2$, luciferase catalyzes the oxidation of luciferin, producing light:

$$ATP + O_2 + luciferin \rightarrow AMP + pyrophosphate + CO_2 + oxyluciferin + light.$$

Additional products of the reaction are AMP, pyrophosphate and oxyluciferin. The light can be detected by a luminometer or similar light-sensitive instrument, or more specifically, by a photomultiplier, a photodiode, a charged coupled device (CCD), or the like.

Preferred luciferase is recombinant firefly luciferase available for example from Promega (Madison, WI) and, as a kit component, from Molecular Probes (Eugene, OR).

A person skilled in the art knows that ATP and some reagents are sensitive to pH. Thus, in a preferred embodiment the reaction is carried out at a pH 7.3-7.8 more preferably around 7.75, and even more preferably at pH 7.6.

<u>Method for identifying a compound for treating or preventing cancer</u>

In another aspect, the invention relates to a method for identifying a compound potentially useful for treating and/or preventing cancer comprising assaying the activity of Nudix5 in the presence of a compound, wherein a compound that inhibits Nudix5 activity is a compound potentially useful for treating and/or preventing cancer.

In a preferred embodiment the Nudsx5 activity is assayed using a method according to the present invention.

<u>Kits</u>

In another aspect, the invention relates to a kit comprising a reagent for analyzing the ATP synthase activity of Nudix5

As used herein, the term <u>"kit"</u> is used in reference to a combination of articles that facilitate the methods of the present invention. These kits provide the materials necessary for carrying out the application described herein.

The term <u>"reagent for analyzing the ATP synthase</u>", as used herein, refers to a compound necessary for the formation of ATP and compounds necessary for detecting said ATP.

In a preferred embodiment, the compound necessary for the formation of ATP is ADRP and PPi. In another preferred embodiment, the compound for detecting ATP is luciferase.

In another preferred embodiment, the kit may comprise a recombinant Nudix5 protein. In a more preferred embodiment the kit comprises the recombinant Nudix5 linked to a support, by way of example a 96 well plate.

The kit of the invention may comprise a buffer needed for Nudix5 activity, by way of example $MgCl_2$, particularly 10 mM $MgCl_2$, Tris-HCl, particularly 10 mM Tris-HCl pH 7.6.

The kit may comprise, in addition, a packaging which allows maintaining the reagents within determined limits. Suitable materials for preparing such packings include glass, plastic (polyethylene, polypropylene, polycarbonate and the like), bottles, vials, paper, sachets and the like. The kit of the invention can additionally contain instructions for using the components contained therein. Said instructions can be found in the form of printed material or in the form of an electronic support which can store instructions such that they can be read by a subject, such as electronic storage media

(magnetic disks, tapes, USB flash drives, memory cards and the like), optical media (CD-ROM, DVD) and the like. The media can additionally or alternatively contain Internet websites providing said instructions.

In another aspect the invention relates to the use of the kit of the invention for assaying the ATP synthase activity of Nudix5.

The invention will be described by way of the following examples which are to be considered as merely illustrative and not limitative of the scope of the invention.

EXAMPLES

## Materials and methods

### Cell culture; hormone and inhibitor treatment

T47DM cells were used for all experiments unless otherwise stated. For hormone induction experiments, cells were grown in RPMI medium without Phenol Red, supplemented with 10% dextran-coated charcoal-treated FBS (DCC/FBS) after 24 h in serum-free conditions; cells were incubated with R5020 (10 nM) or vehicle (ethanol) as described (Vicent et al. 2011). For hormone induction experiments in MCF7 cells a similar procedure was performed; cells were grown in DMEM medium without Phenol Red, supplemented with 10% dextran-coated charcoal-treated FBS (DCC/FBS) after 24 h in serum-free conditions; cells were incubated with Estradiol (10 nM) or vehicle (ethanol). PARG and PARP inhibition were carried out via incubating cells with 5uM TA (tannic acid) or 10uM 3AB (3-amino-benzamide) respectively 1 hour prior to hormone treatment. All transfections were performed using Lipofectamine$^{2000}$ (Invitrogen) according to manufacturer's instructions.

### PAR-capture ELISA

Hormone and or inhibitor treatments were carried out as described, and sample preparation was carried out as follows: At the required time point, cells were washed twice with ice-cold PBS and scraped in lysis buffer (0.4 M NaCl, 1% Triton X-100) plus protease inhibitors. Cell suspensions were then incubated for 30 min on ice with periodic vortexing. The disrupted cell suspension was centrifuged at 10,000g for 10 min at 4°C, and the supernatant was recovered, snap-frozen, and stored at 80°C until required. Ninety-six-well black-walled plates were incubated with 2 ng/mL anti-PAR monoclonal antibody (Trevigen) in 50 mM sodium carbonate (pH 7.6) overnight at 4°C. The plates were washed once with PBS and blocked with blocking solution (5% semiskimmed milk powder, 0.1% Tween 20, 25 mM Tris at pH 7.4, 150 mM NaCl) for 1 h at room temperature. The plate was then washed four times in PBS and 0.1% Tween 20. One-hundred microliters of each sample (20 mg of total protein) or a PAR standard was applied and incubated for 1.5 h at room temperature. The plate was washed four times with PBS and 0.1% Tween 20 and incubated with anti-PAR rabbit polyclonal antibody (Trevigen) for 1.5 h at room temperature. The plate was washed four times with PBS and 0.1% Tween 20 and incubated with anti-rabbit HRP conjugate secondary antibody (5% semiskimmed dried milk) for 1.5 h at room temperature. The plate was washed six times with PBS and 0.1% Tween 20, and PAR was identified via incubation with TACs Sapphire (Trevigen) for 10 min in the dark. The reaction was terminated with 5% phosphoric acid, and the absorbance at 450 nm and 520 nm was recorded.

**NAD** depletion assay

T47D$^{M}$ cells were either left untreated or treated with the inhibitor indicated prior to R5020 addition as described. NAD concentration was determined as described (Wright et al 2012). At T = t, cells were washed with ice-cold PBS and incubated in 0.5 M PCA for 20 min on ice. An equal volume of KOH (1 M)/K2HPO4 (0.33 M) (pH 7.0) was added, and the samples were vortexed and incubated for 20 min on ice.

Samples were spun at 10,000 rpm for 2 min at 4°C, and the supernatant was stored at - 20°C. The quantification of NAD was carried out as follows: Samples were added to an equal volume of reaction buffer (200 mM Bicine at pH 7.8, 1 mg/mL BSA, 1MEtOH, 10mMEDTA at pH 8.0, 4mMphenazine). One tenth of the reaction volume of alcohol dehydrogenase was added and incubated for 30 min at 30°C. The reaction was terminated with a 0.33 sample volume of 12 mM iodoacetate, and the absorbance at 570 mM was measured.

**Modelling and evaluation of the phosphorylated and unphosphorylated T45 NUDIX5 homodimer.** Two phosphorylated versions of NUDIX5 were created from a crystal structure of NUDIX5 (Zha et al. 2006 DOI: 10.1016/j.jmb.2006.09.078) deposited in PDB (Berman et al. 2000 Nucleic Acids Res 28, 235-242 (2000)) with PDB identifier 2DSC using MODELLER Protein Sci 9, 1753-1773 (2000).

**Chromatin Immunoprecipitation (ChIP) in cultured cells.** ChIP assays were performed as described (Vicent et al PLoS Genet 5, e1000567 2009) using the H2A specific antibody (Cell Signaling Technologies), or the H1 antibody

(AE4 abcam). Quantification of chromatin immunoprecipitation was performed by qRT-PCR and the fold enrichment of target sequences in the immunoprecipitated (IP) compared to input (I) fractions were calculated using the comparative Ct method with ($2^{Ct(IP)-Ct\ (Ref).}$). Values were corrected by the human b-globin gene and referred as relative abundance over time zero. Primers sequences are available on request

**RNA interference experiments.** All siRNAs were transfected into the T47D$^M$ cells using Lipofectamine 2000 (Invitrogen) as described (Vicent et al 2009 PLoS Genet 5, e1000567). siRNA are sequences available on request.

**RNA extraction and RT-PCR.** Total RNA was prepared and cDNA generated as previously described (Wright et al. 2012 Genes Dev 26:1972-1983). Quantification of gene products was performed by qRT-PCR. Each value calculated using the standard curve method was corrected by the human GAPDH and expressed as relative RNA abundance over time zero. Primer sequences are available on request.

**Protein Extract Preparation, western blotting/Co-immunoprecipitation assay.** Cells were prepared as previously described (Wright et al 2012 Genes Dev 26:1972-1983), briefly cells were lysed (1% NP40, 150mM NaCl, 50mM Tris-HCl) and following total protein quantification, 2mg of cell extracts were incubated with protein G/A agarose beads previously coupled with 5μg of the corresponding antibodies or an unspecific control antibody as described (Vicent et al. 2009 PLoS Genet 5, e1000567). Inputs and immunoprecipitated material was analyzed by western blot using; PARP1 (Cell Signalling technologies), PARG (Abcam ab169639), PAR (trevigen) and NUDIX5 (abcam) specific antibodies.

**Immunofluroescence.** T47D$^M$ or MCF7 cells were grown on coverslips treated with hormone for the desired length of time as described in previous sections prior to fixation using 4% paraformaldehyde in PBS for 15 min and permeabilized with PBS 0.2% Triton X-100 at room temperature. Coverslips were blocked with 5% skim-milk for 1 h at room temperature and incubated 2 hours with primary antibodies diluted in PBS 1% skim-milk at 1/500 PAR monoclonal and or polylonal (trevigen)), 1/1000 (PARP1 (Cell Signalling technologies), or 1/500 anti-phospho Serine 118 ER, anti-luciferase (Sigma L0159). After washes with PBS Tween 20 0.05%, samples were incubated with secondary antibodies (AlexaFluor 555 anti-rabbit and AlexaFluor 488 anti-mouse, Invitrogen-Molecular Probes) for 1 h at room temperature. After washes with PBS Tween 0.05% and DNA staining with DAPI, samples were mounted with mowiol. Images were acquire with a Leica TCS SP5 CFS confocal microscope.

**Microarray Design and Procedure.** The levels of NUDIX5 were reduced in T47D$^M$ cells using siRNA as described or the activity of PARP and PARG were inhibited by treating T47D$^M$ cells with either treated with 3AB or TA respectively. As a control, T47D$^M$ cells were treated with solvent (EtOH alone) prior to R5020 induction for 0 or 6 hours as described above. Total RNA was prepared and cDNA generated as described. Independently generated biological replicates hybridised against the human whole genome 44K expression platform (Agilent). Significantly induced, or repressed genes were considered as those with a $\log_2$ fold of $\geq$ 1.5 and $\leq$-1.5 respectively and a q value of $\leq$5.00.

**Site directed mutagenesis and detection of Myc-NUDIX5 phosphomimetic constructs for expression in mammalian cells.** Site-directed mutagenesis was carried out on the wild-type human PARP1 fused to Myc (pCMV-Myc NUDT5; genescript) as directed in the Quick Change mutagenesis kit. The mutagenic primers were as follows 5'-3'; T45D Fw; TGTACGTTTCACTGATTCCCAATCTCTAGTTTTACCAGTAGGATCC (SEQ ID NO:1 , T47D Rv; GGATCCTACTGGTAAAACTAGAGATTGGGAATCAGTGAAACGTACA (SEQ ID NO:2. Following the mutagenesis procedure, mutations were confirmed by sequencing. Myc-wtNUDIX5 or Myc-NUDIX5-Thr/Asp mutant plasmids were transfected into T47D$^M$ cells using Lipofectamine 2000 (Invitrogen) as described. 48 hours later, cells were treated with hormone, cell proliferation, and gene or protein expression determined as described above.

**Cell proliferation assay**.T47D$^M$ / MCF7 cells transfected with control, NUDIX5 siRNAs or treated with the inhibitors 3AB/tannic acid as described above. Cells ($1 \times 10^4$) were plated in a 96-well plate in the presence or absence of R5020 (T47D$^M$) or estradiaol (MCF7). The cell proliferation ELISA BrdU Colorimetric assay (Roche) was performed according to the manufacturer's instructions. The experiments were performed in triplicate.

**Purification of Recombinant NUDT5 in vitro**.Recombinant NUDIXT5 was produced using 1-step Human high yield in vitro mammalian translation (IVT) system (Thermo scientific). Briefly, IVT reactions containing (Hela cell lysate, accessory proteins, reaction mix, 4ug NUDIX5 DNA (pANT7 GST-NUDT5 (DNASU, plasmid depository) in 100ul) was dialysed for 16 hours at 30C according to manufacturer's instructions. Recombinant NUDIX5 was then purified from the total reaction using GST beads (as described Wright et al 2012 Genes Dev 26:1972-1983).

**DNA damage; Cell Survival Assay**.MCF7 cells were transfected with control, NUDIX5 siRNAs or treated with the inhibitors 3AB/tannic acid as described above. Cells were then plated in a 100mm plates at varying concentrations and subjected to DNA damage (H2O2) at varying concentrations (0.1, 0.2, 0.5mM). The medium was replaced every 3 days and the resultant surviving colonies stained using crystal violet solution (0.5% crystal violet, 10% methanol). Data is represented as a percentage surviving fraction compared to control (undamaged).

**PAR Mass Spec Peptide Enrichment analysis**.T47D cells were treated with hormone or ethanol for 30 minutes and resultant lysates immunoprecipted using anti-PAR antibody. Immunoprecipiated peptides were digested trypsin. Following this 1ug of each sample were analyzed by LCMSMS using a MEDI_CID method in the LTQ Orbitrap XL. To avoid carry over, samples were injected in increasing amount and BSA controls were included both in the digestion and

LCMSMS analysis for quality control.

Samples were searched against SwissProt_Human (January 2013) using an internal version of the search algorithm Mascot (http://www.matrixscience.com/).

Data analysis was performed with Proteome Discoverer v1.3.

Peptides have been filtered based on the FDR (False Discovery rate) for peptides with FDR better (lower) than 5%.

Protein Discoverer gives an approximate estimation of protein amount with the parameter "Area" which is the average peak area of the 3 top peptides for a given protein. Immunoprecipitation was performed in two independent experiments for both control and hormone treated samples and the relative fold change compared to control determined using the average area for the two experiments.

**Real time ATP visualisation using ATeam probes following hormone.** T47D cells were transfected with Ateam constructs in 35mm diameter cell culture plates and starved prior to hormone treatment as described. Hormone was added directly prior to YFP and CFP emission detection at 1 minute intervals and the ratio for each region of interest calculated following background substraction.

## Results

### Example 1- Nuclear ATP levels increase in response to progestin

To measure ATP levels in living T47D$^{M}$ cells we utilised the ATeam constructs were used (Imamura et al., 2009 Proceedings of the National Academy of Sciences of the United States of America 106, 15651-15656.), a set of FRET-based ATP/ADP detectors targeted to cell nucleus, mitochondria or cytosol (Figure 1A). **ATP**$^{(YFP)}$/**ADP**$^{(CFP)}$ emissions were measured at 1 minute intervals after hormone stimulation, the images were processed and the ratio of ATP/ADP determined as described (Kardash et al., 2011 Nature protocols 6, 1835-1846 and Figure 1B). The specificity and sensitivity of the ATeams were demonstrated using glucose stimulated Mito-ATeam in the presence or absence of the ATP-synthase inhibitor oligomycin. Levels of ATP increased rapidly following the addition of glucose but the increase was inhibited by simultaneous treatment with oligomycin (Figure 1C and D, respectively.). Following addition of hormone to cells expressing the Nuclaer-ATeam the nuclear ratio **ATP**$^{(YFI)}$/**ADP** $^{(CFP)}$ increased rapidly to reached a low peak at 10-15 min followed by a much higher peak between 35 and 50 minutes compared to the mock treated cells. (Figure 2A, quantification of 18 nuclei for each condition; Figure 2B, snapshots at 5 minutes intervals of a cell treated with hormone). No significance changes were observed in cells expressing the Mitochondrial-ATeam treated with hormone or with solvent alone (Figure 2C).

To confirm the FRET based results, we used nuclear, cytoplasmic and mitochondrial luciferase-based reporter constructs (Hageman et al., 2007 The Journal of biological chemistry 282, 34334-34345.) to measure the levels of ATP by bioluminescence imaging (Figure 1E and F). We confirmed the specific increase in nuclear ATP levels in cells treated with hormone, with a low peak at 10-15 min and a high peak at 40-50 minutes, which was not observed with the cytoplasmic or mitochondrial targeted constructs (Figure 2D, representative bioluminescent image; Figure 2E quantification of three experiments).

The increase in nuclear ATP was compromised by inhibiting the production of mitochondrial ATP with oligomycin prior to hormone addition, indicating that mitochondrial ATP is required for initiation of nuclear ATP generation (Figure 3A and B). We therefore analysed the time kinetics of mitochondrial requirement. In response to glucose the ATP levels in the nucleus increase rapidly (Figure 3C), likely via diffusion of ATP from the mitochondria, as demonstrated by its inhibition by oligomycin (Figure 3D). The subsequent depletion of nuclear ATP demonstrate that the nucleus in steady state contains a finite pool of ATP which can withstand approximately 5-10 minutes before exhaustion under conditions where it is not replenished (Figure 3D dashed line). However, in cells treated with hormone followed after 10 min by oligomycin, the nuclear levels of ATP continue to increase up to 40-50 minutes via a mitochondrial independent mechanism (Figure 3E). We conclude that nuclear ATP generated after 15-20 minutes of hormone treatment, is independent of mitochondrial ATP synthesis.

### Example 2 PAR degradation, conversion to ATP

As previously shown, hormone induces a strong and transient increase in nuclear Poly-ADP-ribose (PAR) levels (Figure 5A top panels), due to the activation of PARP1 (Wright et al., 2012 Genes & Dev 26,:1972-1983.). Concomitantly with the PARP1 activation the degradation of PAR by PARG is also induced, as indicated by the opposite effect of PARP1 and PARG inhibition. While PARP1 inhibition prevents the accumulation of PAR, PARG inhibition results in persistence of elevated PAR levels (Figure 5A, bottom panels, and Figure 5B). In concert with PAR accumulation following hormone we observe a transient decrease in cellular NAD levels, which is dependent on PARP activity as demonstrated by the effect of PARP inhibition with 3AB (Figure 5C). In cells treated with hormone, both PAR and NAD

levels return to basal state after 60-80 minutes (Figure 5B and C, respectively). The rescue of NAD levels is dependent on PARG activity as shown in the presence of the selective inhibitor Tannic Acid, suggesting that ADP ribose may be required (Figure 5C).

**Example 3 Role of NUDIX5 in the Hormonal Response.**

To gain further insight into the role of PAR, we performed mass spec analysis of the PAR interactome in untreated control cells and in cells treated with hormone for 30 minutes (Figure 5D). Cell extracts were immunoprecipitated with an antibody against PAR and precipitated proteins were analysed by mass spectroscopy. Peptide enrichment and coverage led to the identification of 1654 proteins (Figure 5D), including both PARylated and PAR binding proteins. Many of those identified proteins have also been reported in other cell types. Network analysis performed based on GO biological function identified significant protein sub-networks.

One identified protein of particular interest with regards to the generation of ATP was NUDIX5 (also known as NUDT5), which belongs to the GO:molecular function ADP-ribose diphosphate activity. Members of the NUDIX (Nucleoside Diphosphate linked to X) superfamily of enzymes are found in all classes of organisms and catalyse the hydrolysis of a wide range of substrates including nucleotide sugars (review McLennan et al., 2006 Cell Mol ife Sci 63, 123-143.). NUDIX5 is in the top 10% of genes overexpressed in breast cancer in correlation with PARP1 and PARG (Figure 7A), a signature not shared by other members of the NUDIX family (Figure 7B). NUDIX5 overexpression correlates with known PARP1 interacting proteins in invasive breast carcinoma compared to normal breast (p=7.4E-30) in addition to other cancer types (Figure 13E).

This evidence led us to hypothesize a crosstalk between PARP1, PARG and NUDIX5. Indeed NUDIX5 interacts with both PAR and PARG in a hormone dependent manner (Figure 6A). Nudix5 catalyses the hydrolysis of modified nucleoside diphosphates including ADP-ribose yielding 5-ribose-phosphate and AMP. Therefore we hypothesised that ADPR generated by PARG could be used by NUDIX5 to generate ATP in nuclei of cells treated with hormone.

To test this hypothesis we measured nuclear ATP in the presence of inhibitors of PARP1 or PARG, or in the presence of a siRNA against NUDIX5 (Figure 6B). We observed that specifically the high increase in nuclear ATP around 40-50 minutes after hormone was dependent on PARP1 and PARG, as well as on NUDIX5 (Figure 6C). These findings indicate that both the formation of PAR and its degradation to ADPR are needed for nuclear ATP generation, and that the enzyme NUDIX5 belonging to the phosphohydrolase subfamily of Nudix enzymes is also essential for nuclear ATP synthesis.

It is important to note that the rapid peak of nuclear ATP at around 10 minutes following hormone (Figure 6C dash box) is independent of PAR formation, degradation or the action of NUDIX5, and is mitochondria dependent, as shown by the simultaneous depletion of ATP (Figure 6C and D). In contrast, the later peak at 40-50 minutes is mitochondria independent however PARP, PARG and NUDIX dependent.

To directly test this hypothesis, we synthesized recombinant human NUDIX5 in vitro (Figure 11B) and used it in an assay with ADPR and PPi in the presence of 4mM Mg2+. ATP production was determined by measuring luciferase activity. We found that ATP was generated at 37°C using 200uM PPi and a ADPR (0.5-500uM) as substrates (Figure 6E).

**Example 4 PAR derived ATP generation and chromatin remodelling**

To gain insight into the molecular process for which PAR-derived ATP is used, we analysed the effect of inhibiting ATP synthesis on chromatin dynamics and gene expression following hormone treatment. In expression microarray experiments, inhibition of PARG or NUDIX5 compromised regulation of 50% of hormone-responsive genes, with a significant overlap between genes dependent on PARG and on NUDIX5 (Figure 8A). 70% of both NUDIX5 and PARG dependent genes required both activities and majority of these genes also depended on PARylation (Figure 8B and C). Consistent with previous reports (Frizzel et al., 2009 BCR 11, 111., Wright *et al.,* 2012 cited supra), we found that genes dependent on PARP and PARG were enriched in processes such as cell cycle, metabolism and stress.

Since we have shown previously that progestin induced proliferation is abrogated by inhibition of PAR formation (Wright et al., 2012 Genes & Dev 26,:1972-1983.), we tested the effect of blocking PARG or NUDIX5. Similar to PARP1, both PARG and NUDIX5 and hence PAR-derived ATP are required for progestin induced cell proliferation (Figure 8D). This effect was not restricted to T47D cells and progestins. MCF7 induced proliferation and associated changes in gene expression in response to oestrogen are also dependent on these three enzymatic activities (Figure 8E and F, Figure 9A). Indeed although the reaction was slower than in response to progestin, estrogens induced increases in PAR and nuclear ATP that were dependent on the activities of PARP, PARG and NUDIX5 (Figure 9B, and C).

We have demonstrated previously the rapid and global changes that take place on chromatin and genome topology and gene expression following hormone treatment. Two consecutive chromatin remodelling events take place on the chromatin of hormone response genes; an early one (1-5 min) leads to histone H1 displacement, and a later one (5-60 min) leads to displacement of H2A/H2B dimers (Vicent et al., 2011 Genes & Dev 25: 845-862). The displacement

of histone H1 depends on the activities of CDK2, PARP1 and NURF, and is a prerequisite for the displacement of H2A/H2B dimers, which is catalysed by BAF and PCAF (Vicent et al., 2011 Genes & Dev 25: 845-862). Blocking this first step precludes the subsequent recruitment of factors that remove H2A/H2B, including kinases, histone acetyl transferases and the BAF complex (Figure 8G and H). We now found that the initial displacement of histone H1 does not depend upon the actions of either PARG or NUDIX5 (Figure 8G), while displacement of H2A depend on PARP1, and weakly on PARG and NUDIX5 (Figure 8H). In contrast late (30 and 60 min) displacement of H1 and H2A/H2B was dependent on all 3 activities PARP1, PARG and NUDIX5 (Figure 8G and H). This suggests that initial remodeling uses ATP coming form the mitochondria while the subsequent remodelling steps depend on nuclear ATP generation. As an indicator of chromatin accessibility we measure the sensitivity to DNase I of PR binding sites in a few genes, whose hormonal regulation is dependent on nuclear ATP (Di Stefano et al., 2014 Nature 506, 235-239). We found that in response to hormone these genes exhibited increased nuclease sensitivity than regulated genes, which are not dependent on nuclear ATP. Collectively these data support the idea that ATP is required for genes which regulation involves extensive chromatin remodelling.

To explore this notion we compared the chromatin landscape over genes, which regulation is dependent or independent of PARG and NUDIX5. Relative peak density for transcription factors or epigenetic marks was plotted as fold change following hormone compared to T0. As expected, polymerase II is enriched on up-regulated genes, compared to its displacement from down-regulated genes (Figure 9D left panel). Similarly, the progesterone receptor (PR), PARP1, p300, BPFT and FOXA1 show a preferential recruitment to up-regulated compared to down-regulated genes. In contrast to the profiles of Histone H1, H2A, LSD1 and HP1$\gamma$, which are more strongly displaced from up-regulated genes and enriched in down-regulated genes following hormone. (Figure 9). This method of visualising the chromatin environment was used to analyzed the role of ATP generated via the degradation of PAR. Hormone up-regulated genes are ATP dependent and have an increased recruitment of PARP1, BPTF, and CDK2 in conjunction with increased displacement of histone H1 H2A, LSD1 and HPl$\gamma$ compared to ATP-independent genes. Indicative of a higher requirement for ATP at genes dependent on PARP1, PARG and NUDIX5.

## Example 5 - Mechanism of Nudix5 activation

The authors have preliminary evidence for the mechanism of NUDIX5 activation in cells treated with hormone. Unlike all the known NUDIX hydrolase family members, NUDIX5 is known to form a homodimer in order to catalyse the hydrolysis of ADPR to 5-ribose-phosphate and AMP. Phosphoproteomic analysis in T47D$^M$ cells prior to hormone treatment identified NUDIX5 to be phosphorylated threonine 45 (T45), which was rapidly and completely lost following hormone (Figure 10C). Structural modelling leads us to the hypothesis that T45 phosphorylation is key to maintain NUDIX5 as a homodimer, resulting in a double lock of the monomers (Figure 11A). De-phosphorylation of T45, would result in a de-stabilisation of the homodimer (Figure 10D). We hypothesised that this change in conformation may be important for NUDIX5 substrate recognition or/and catalysis. Hydrolysis of ADP-ribose to AMP is energetically favourable with the homodimer, while the reaction energetically and structurally feasible by the monomer is the hydrolysis of ADP-ribose to 5-ribose phosphate and ATP in the presence of pyrophosphate. In order to test this hypothesis we generated NUDIX5 T45 phospho-null and phospho-mimetic mutants (T45A/D) and found that indeed NUDIX5 phosphomimetic, behaves as a dominant negative for progesterone induced gene regulation (Figure 10E and F).

## Conclusions

Nuclear processes; including chromatin remodelling, transcription and replication require immense amounts of energy in form of ATP. This essential molecule cannot be stored and hence is constantly used and replenished. Here we have described a novel mechanism of energy rescue in breast cancer cells treated with hormone whereby the energetic cost of global chromatin modification is guaranteed via the catalysis of ADPR to ATP, to ensure cell survival and that the local energy demands of chromatin are met.

In response to hormone changes nuclear ATP levels reflect two distinct processes; an initial mitochondrial dependent phase (5-10 minutes) followed by a later increase in ATP (30-40 minutes) independent of the mitochondria but dependent on nuclear ADPR (Figure 2 and 3). In the initial stages (5 minutes) histone H1 and H2A/H2B are locally displaced from the promoters of hormone responsive genes via the concerted actions of the chromatin remodelling factors NURF and BAF and on PARylation by PARP1. The energy requirements during these initial minutes are met via the pool of nuclear ATP present in the nucleus and are mitochondria dependent (Figure 3). Nevertheless the activity of PARP1 during these initial stages is essential for the displacement of histone H1 and H2A/H2B presumably via parylation of histone H1 or competition with its binding to chromatin (Krishnakumar et al 2008 Science 319, 819-821) . During these initial stages of hormone activation the levels of PAR steadily increase in concert with a decrease in NAD levels (Figure 5A-C). During the second phase; PAR levels sharply decrease due the direct actions of PARG (Figure 5B), a decrease accompanied by a rescue of NAD and an increase in nuclear ATP (Figure 2 and 5C). This energy store is as a direct

result of PAR degradation via PARG and ADPR catalysis to ATP via NUDIX5 (Figure 6) and it essential for late chromatin modification (30-60 min) required for the transcriptional gene regulation, cell proliferation and survival (Figure 8, model Figure 10A).

Previous work suggested that ATP generated via ADPR might play a role in DNA damage repair (Oei & Ziegler, 2000 The Journal of biological chemistry 275, 23234-23239). Cell survival and ATP assays performed in MCF7 cell lines confirmed that cell survival in response to DNA damage is dependent on PARP1, PARG and NUDIX5 (Figure 13A and B). It has been suggested that in response to hormone, and prior to transcription activation local DNA breaks occur that induce parylation and DNA repair (Ju et al 2006 Science 312, 1798-1802). NUDIX5 over expression in breast cancer patients correlates with a poor outcome in hormone receptor positive breast cancer compared to PR and ER negative cancer (Figure 13A and B). This in addition to the observation that NUDIX5 overexpression is significant in multiple cancer types and correlates with high expression of PARP1 (Figure 13C and D) suggests that perhaps this mechanism of energy rescue and ATP generation is a process exploited in cancer and hence a NUDIX5 provides a new target for PARP1 combinatorial drug therapy.

## Claims

1. A Nudix5 inhibitor for use in the treatment and/or prevention of cancer in a subject.

2. Pharmaceutical composition comprising a therapeutically effective amount of a Nudix5 inhibitor and a pharmaceutically acceptable excipient or carrier.

3. A composition comprising a Nudix5 inhibitor and at least a second antitumor agent.

4. Composition according to claim 3 wherein the antitumor agent is selected from PARP1 inhibitor, PARG inhibitor and BRCA1 inhibitor.

5. Nudix5 inhibitor for use according to claim 1, pharmaceutical composition according to claim 2 or composition according to any of claims 3 or 4 wherein the Nudix5 inhibitor is selected from Table 1.

6. A Nudix5 inhibitor for use, a pharmaceutical composition or a composition according to claim 5 wherein the Nudix5 inhibitor is selected from siRNA and a Nudix5 phosphomimetic mutant T45D.

7. Pharmaceutical composition comprising a therapeutically effective amount of a composition according to any of claims 3 to 6 and a pharmaceutically acceptable excipient or carrier.

8. Composition according to any of claims 3 to 6 or pharmaceutical composition according to any of claims 2, 5, 6 or 7 for use in the treatment and/or prevention of cancer in a subject.

9. Nudix5 inhibitor for use according to any of claims 1, 5 or 6, composition or pharmaceutical composition for use according to claim 8 wherein the cancer is selected from acute myeloid leukemia, breast carcinoma, colorectal adenocarcinoma, diffuse large B-cell lymphoma, endometrial adenocarcinoma, follicular lymphoma, lung adenocarcinoma, melanoma, ovarian adenocarcinoma, pancreatic adenocarcinoma, pleural mesothelioma, B-cell acute lymphoblastic Leukemia, T-cell Acute Lymphoblastic Leukemia, prostate carcinoma and renal cell carcinoma.

10. Nudix5 inhibitor for use according to any of claims 1, 5, 6 or 9, composition or pharmaceutical composition for use according to any of claims 8 or 9 wherein the cancer is a hormone-dependent cancer.

11. Nudix5 inhibitor for use, composition or pharmaceutical composition for use according to claim 15, wherein the hormone- dependent cancer is a progesterone-dependent breast cancer.

12. A method for assaying the activity of Nudix5 comprising assaying the formation of ATP.

13. A method for identifying a compound potentially useful for treating and/or preventing cancer comprising assaying the activity of Nudix5 in the presence of a compound, wherein a compound that inhibits Nudix5 activity is a compound potentially useful for treating and/or preventing cancer.

14. A kit comprising a reagent for analyzing the ATP synthasizing activity of Nudix5.

**15.** Use of the kit according to claim 14 for analyzing the ATP synthesizing activity of Nudix5.

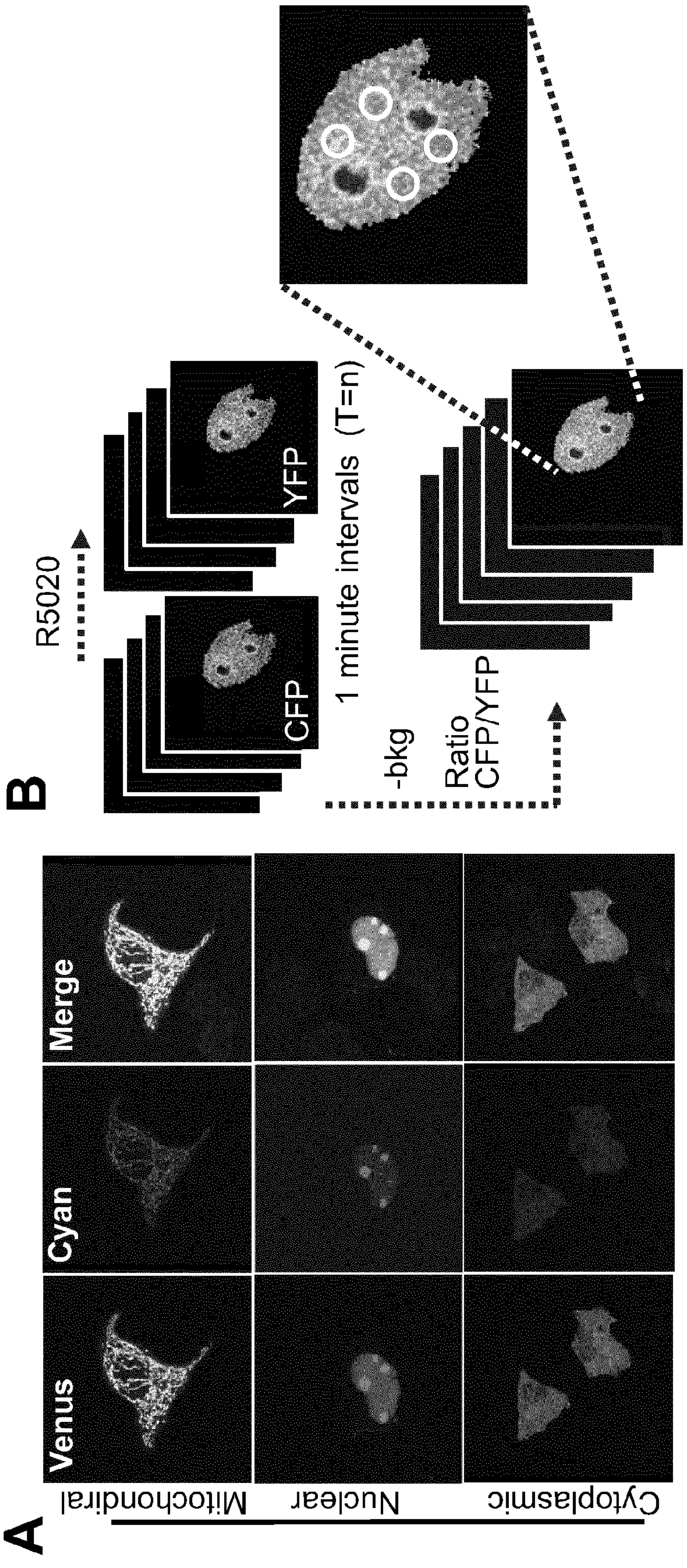
A
Venus
Cyan
Merge
Mitochondiral
Nuclear
Cytoplasmic
B
R5020
CFP
YFP
1 minute intervals (T=n)
-bkg
Ratio
CFP/YFP

FIG. 1

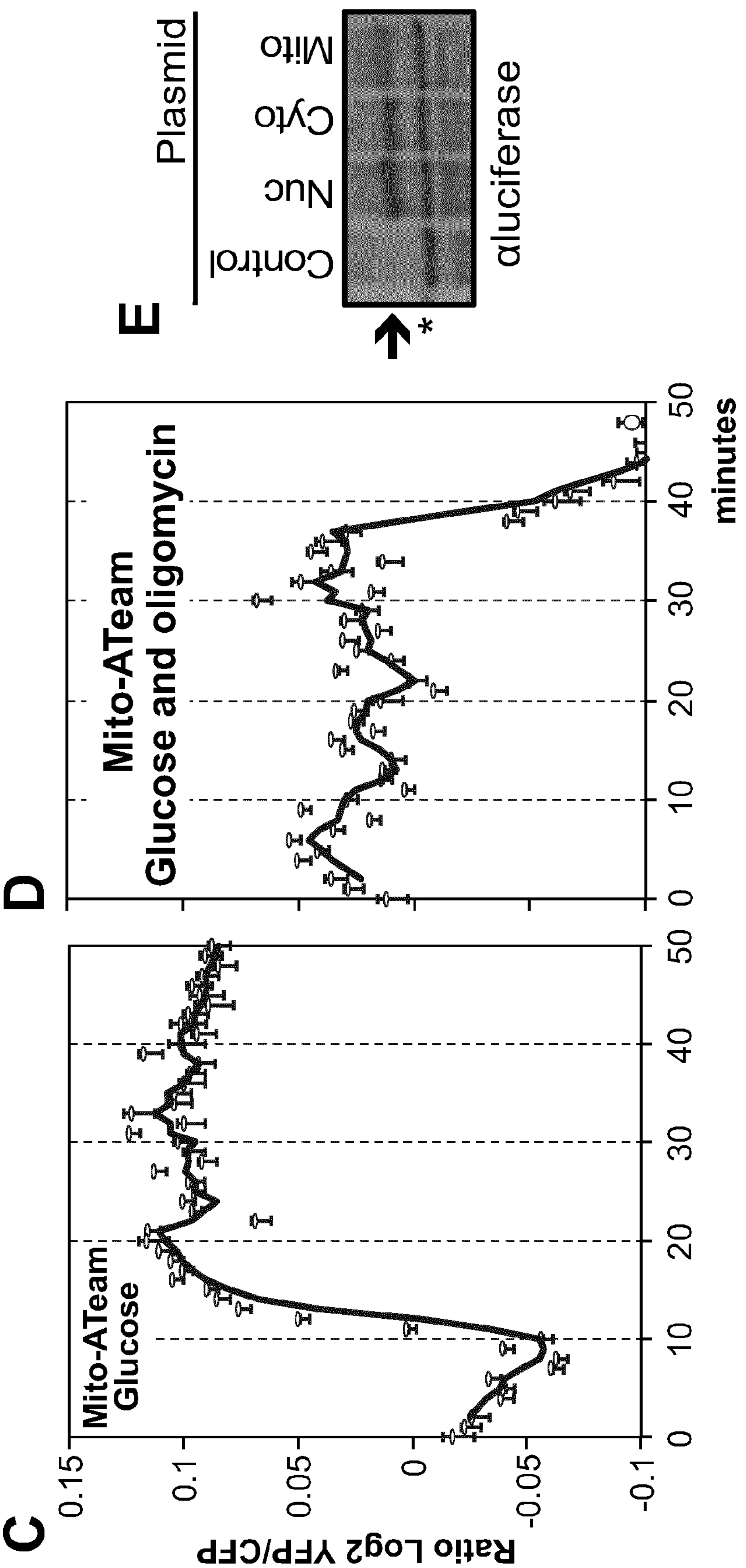
C
Ratio Log2 YFP/CFP
0.15
0.1
0.05
0
-0.05
-0.1
Mito-ATeam
Glucose
0
10
20
30
40
50
D
Mito-ATeam
Glucose and oligomycin
0
10
20
30
40
50
minutes
E
Plasmid
Control
Nuc
Cyto
Mito
*
αluciferase

FIG. 1 (cont. 1)

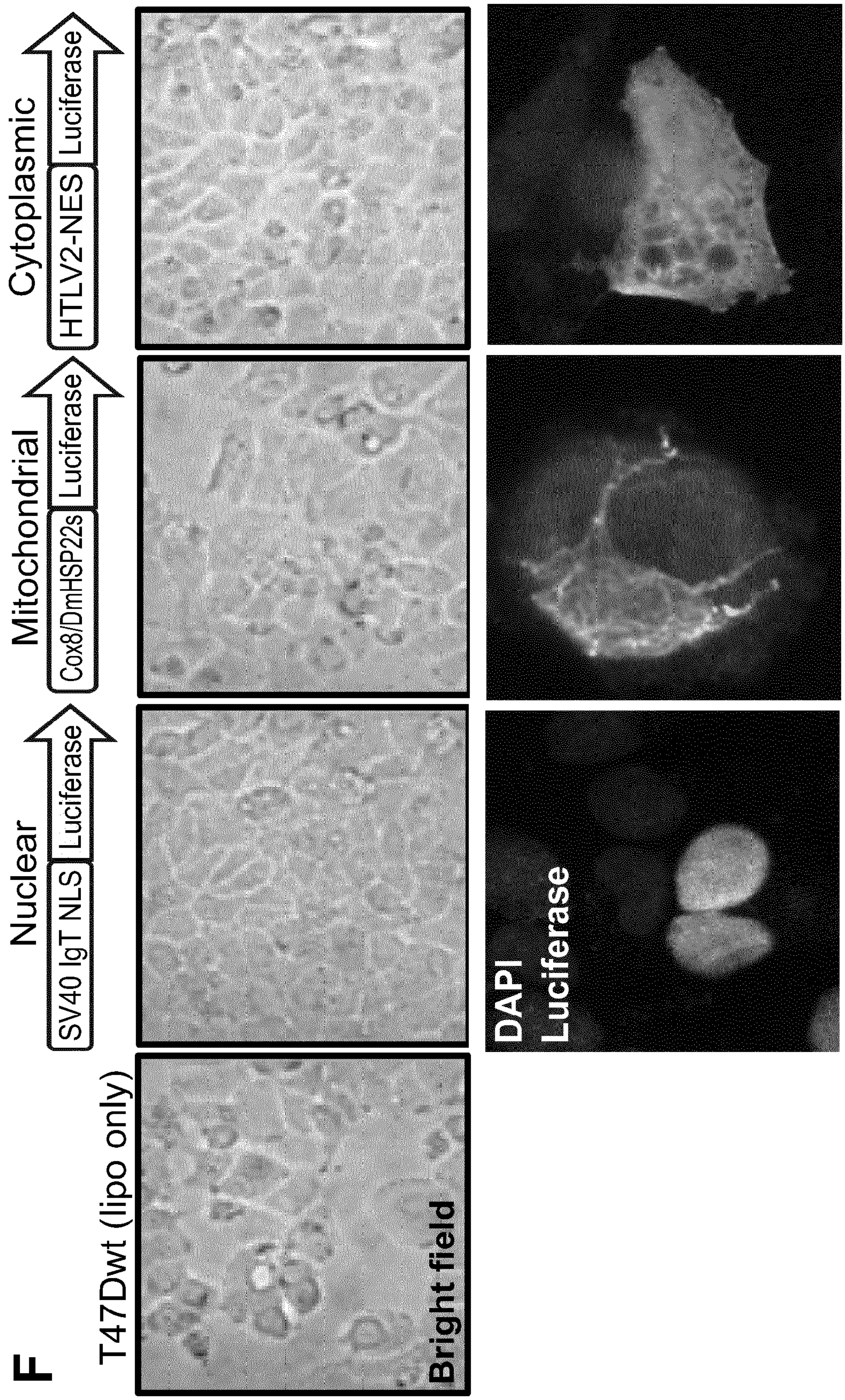
F
T47Dwt (lipo only)
Bright field
Nuclear
SV40 IgT NLS
Luciferase
Mitochondrial
Cox8/DmHSP22s
Luciferase
Cytoplasmic
HTLV2-NES
Luciferase
DAPI
Luciferase

FIG. 1 (cont. 2)

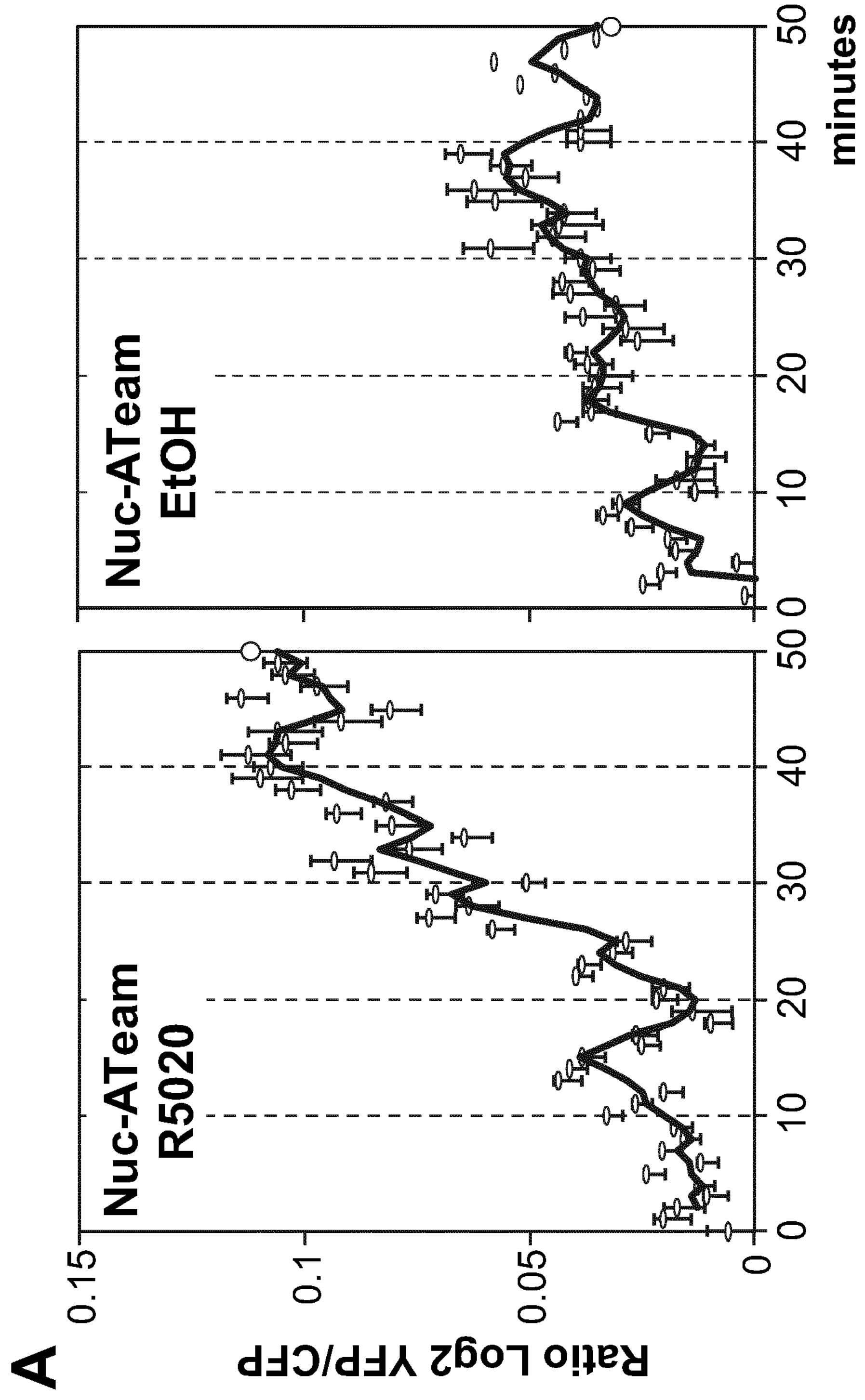
A
Ratio Log2 YFP/CFP
0.15
0.1
0.05
0
Nuc-ATeam
R5020
Nuc-ATeam
EtOH
0
10
20
30
40
50
minutes

FIG. 2

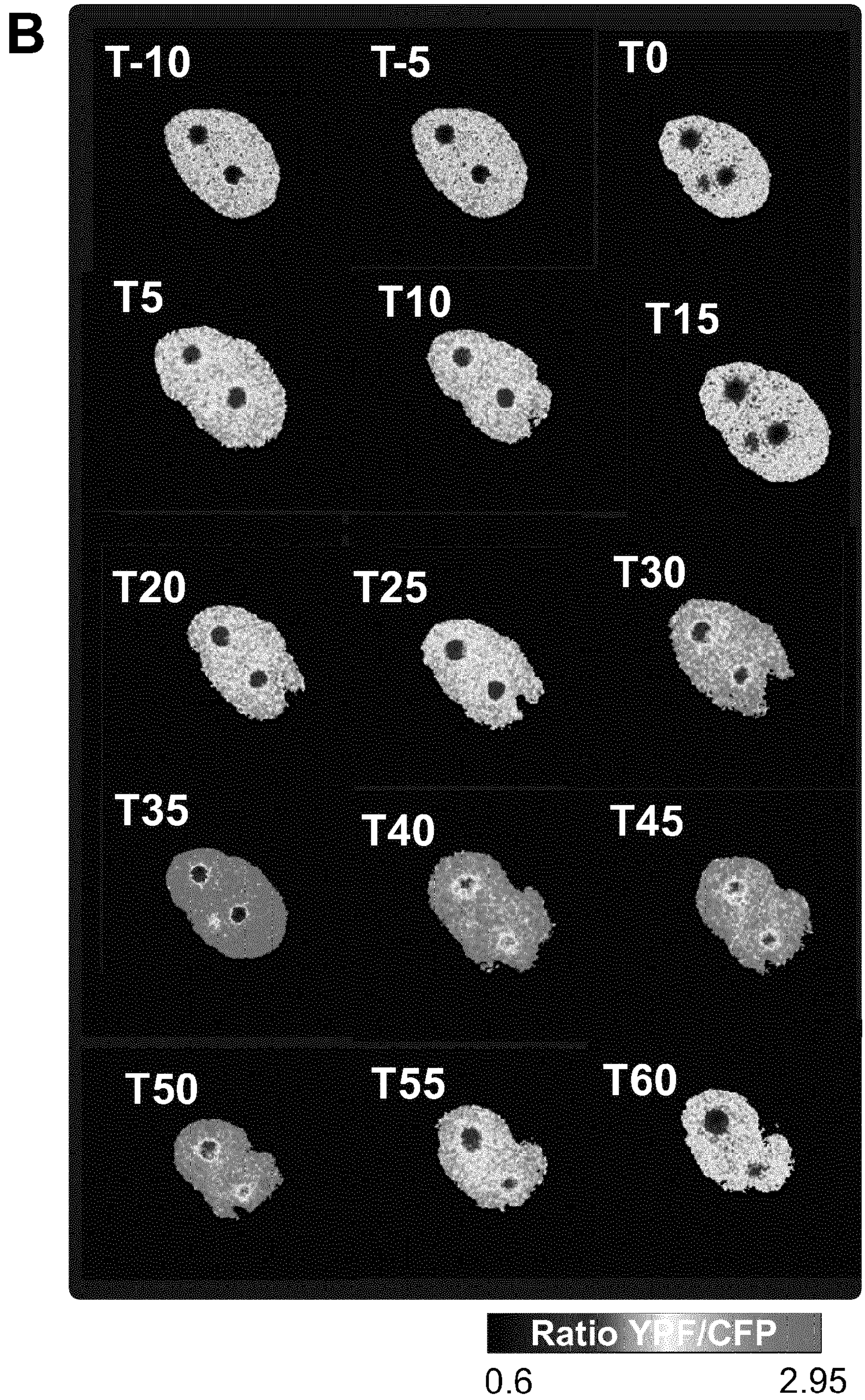
B
T-10
T-5
T0
T5
T10
T15
T20
T25
T30
T35
T40
T45
T50
T55
T60
Ratio YPF/CFP
0.6
2.95

FIG. 2 (cont. 1)

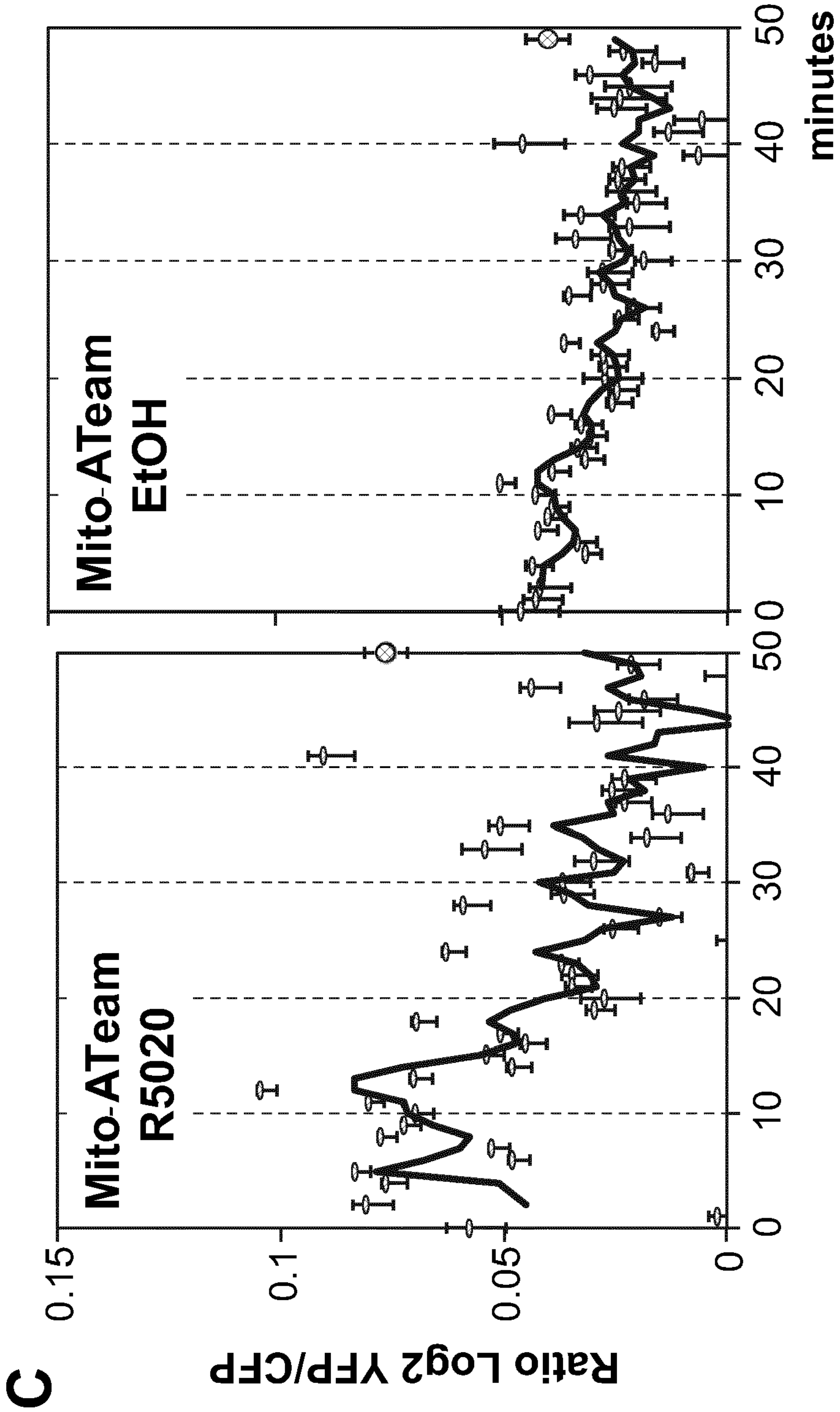
C
Ratio Log2 YFP/CFP
0.15
0.1
0.05
0
Mito-ATeam
R5020
Mito-ATeam
EtOH
0
10
20
30
40
50
minutes

FIG. 2 (cont. 2)

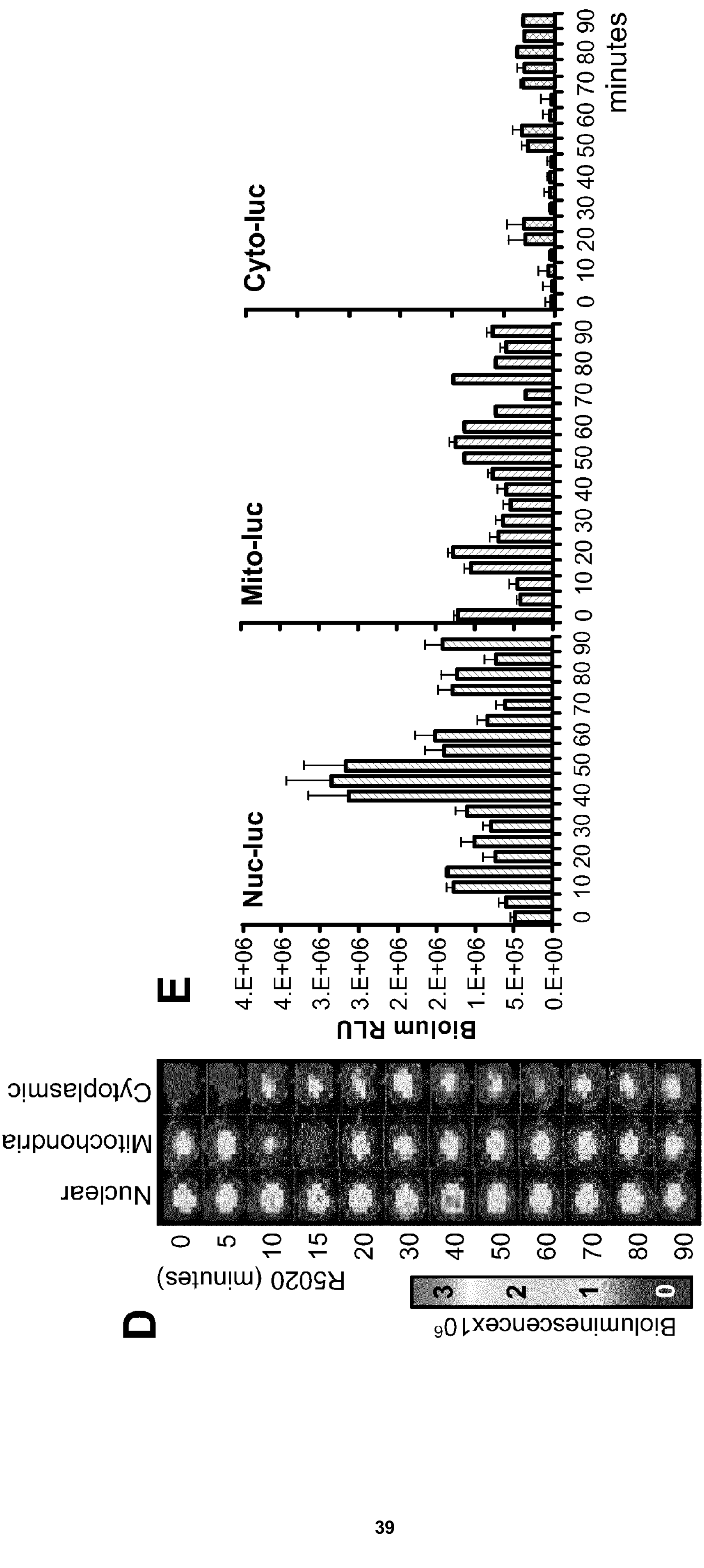
D
R5020 (minutes)
0
5
10
15
20
30
40
50
60
70
80
90
Nuclear
Mitochondrial
Cytoplasmic
Bioluminescencex10^6
3
2
1
0
E
Biolum RLU
4.E+06
4.E+06
3.E+06
3.E+06
2.E+06
2.E+06
1.E+06
5.E+05
0.E+00
Nuc-luc
Mito-luc
Cyto-luc
0 10 20 30 40 50 60 70 80 90
minutes

FIG. 2 (cont. 3)

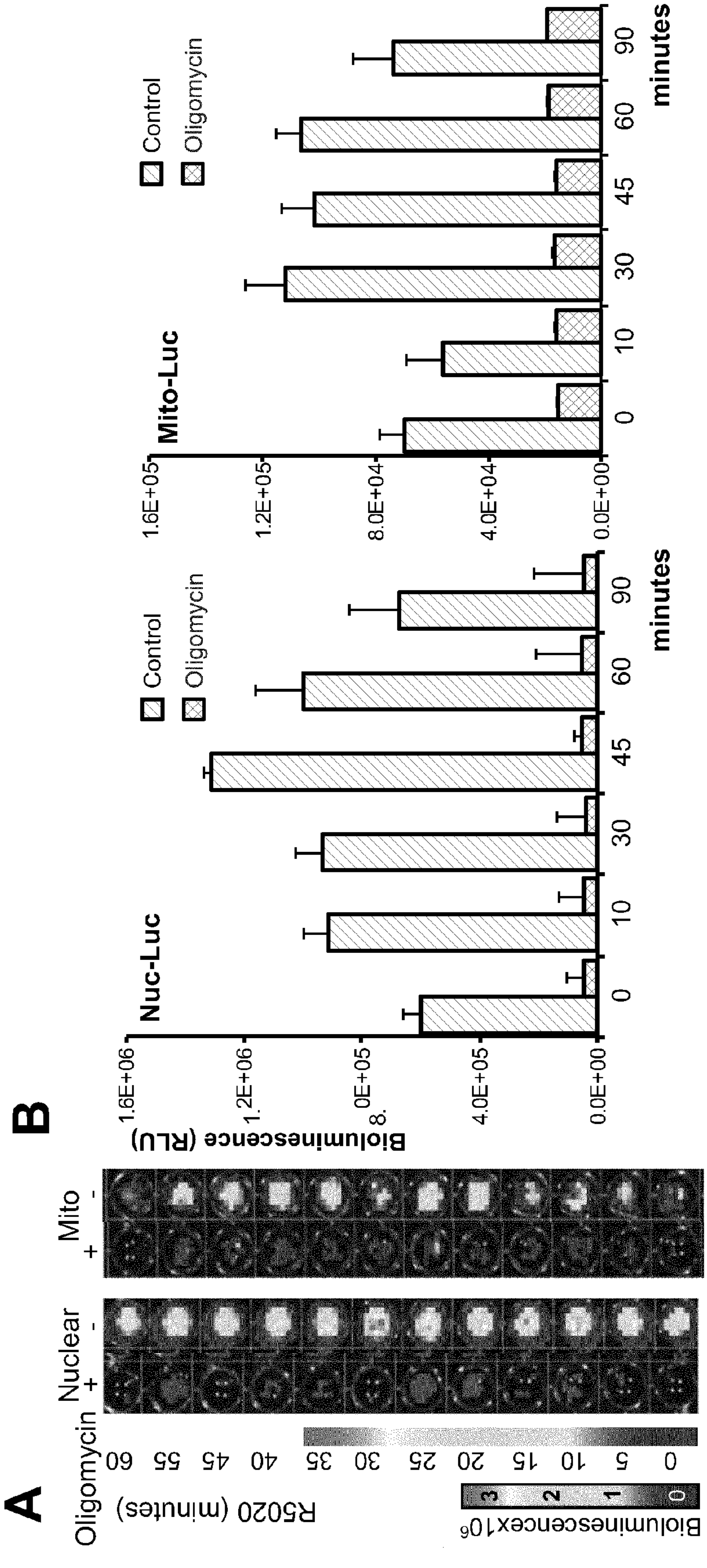

A
Oligomycin
R5020 (minutes)
Nuclear
Mito
+
-
0
5
10
15
20
25
30
35
40
45
55
60
Bioluminescencex10^6
0
1
2
3
B
Bioluminescence (RLU)
0.0E+00
4.0E+05
8.0E+05
1.2E+06
1.6E+06
Nuc-Luc
Control
Oligomycin
minutes
0.0E+00
4.0E+04
8.0E+04
1.2E+05
1.6E+05
Mito-Luc
0
10
30
45
60
90


FIG. 3

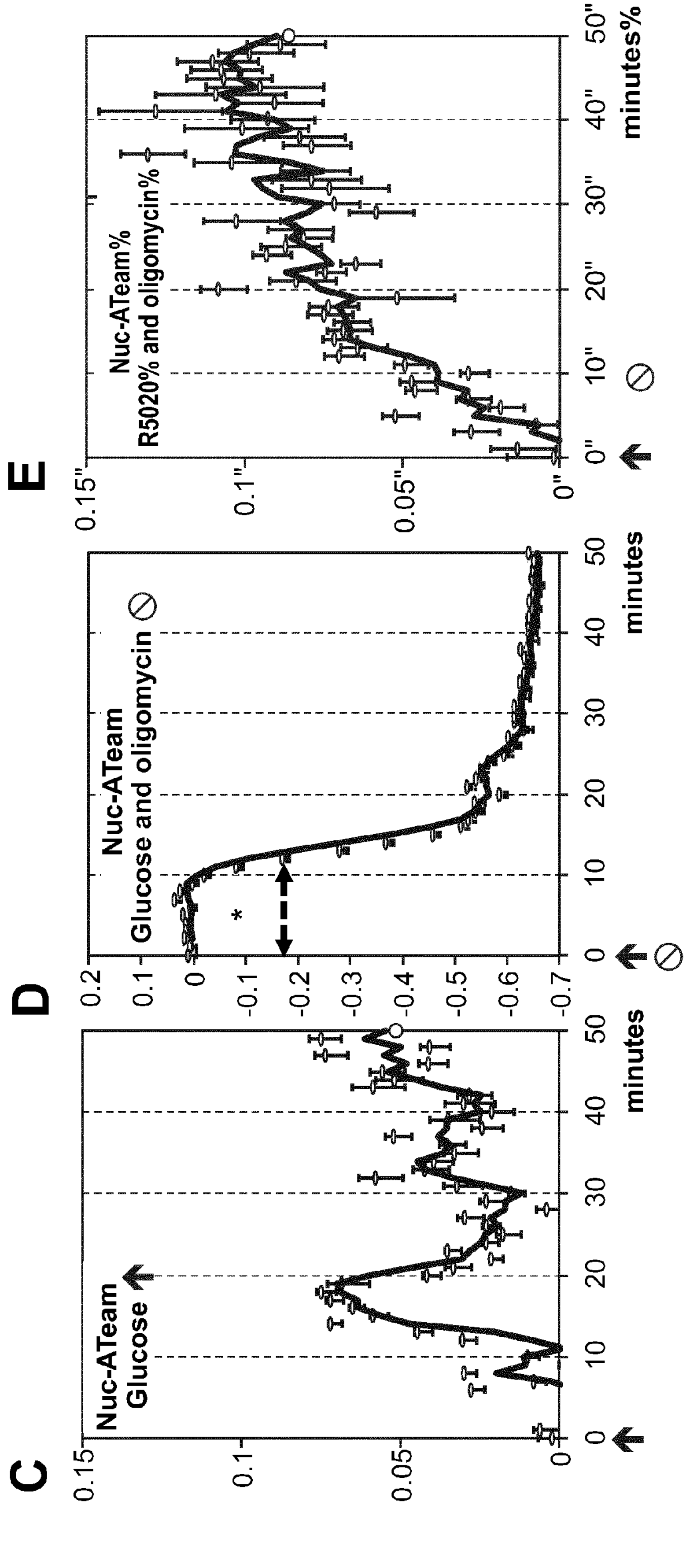
C
Nuc-ATeam
Glucose
minutes
D
Nuc-ATeam
Glucose and oligomycin
*
minutes
E
Nuc-ATeam%
R5020% and oligomycin%
minutes%

FIG. 3 (cont. 1)

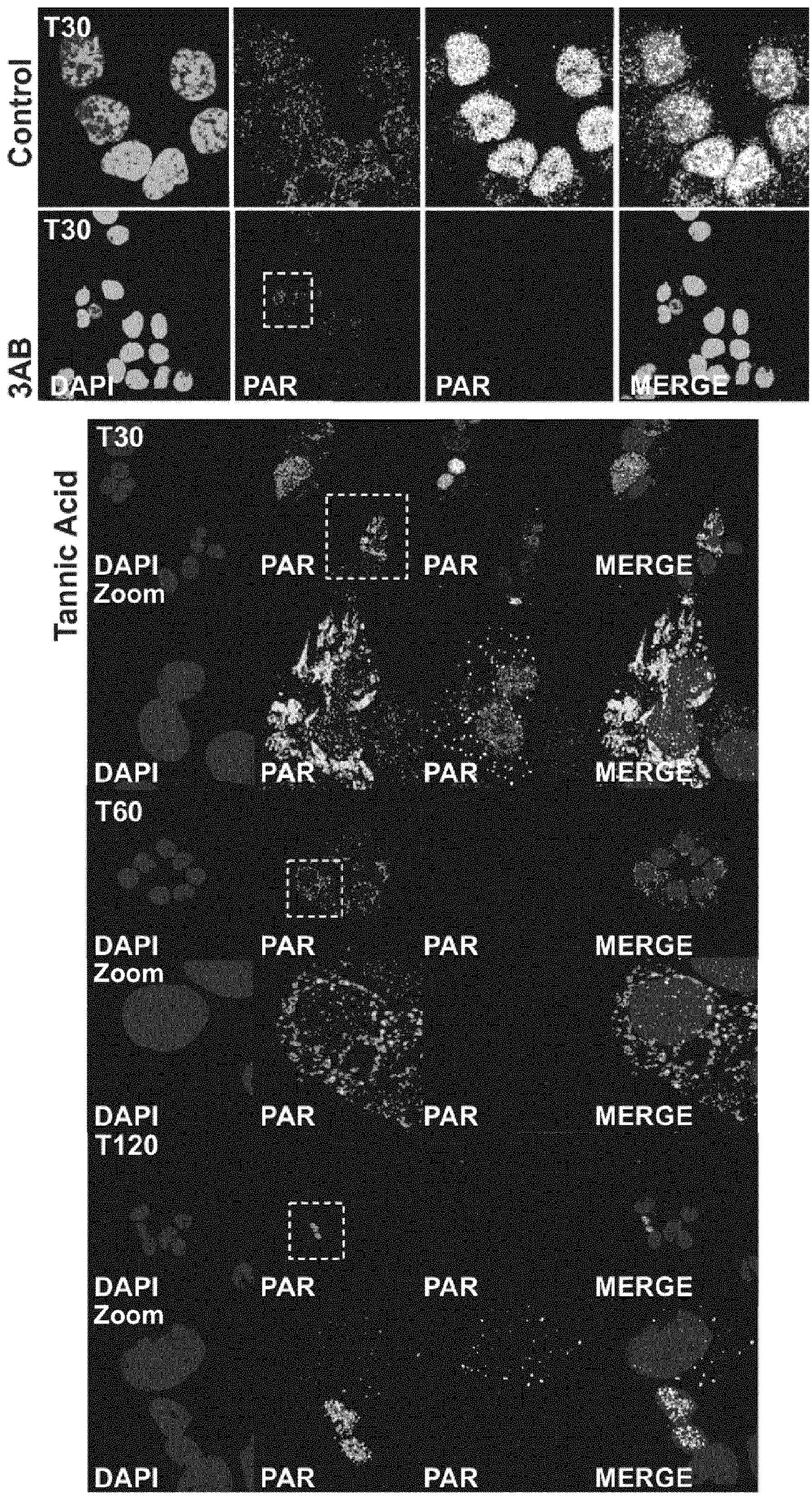
Control
3AB
Tannic Acid
T30
T30
T30
T60
T120
DAPI
PAR
PAR
MERGE
DAPI
Zoom
DAPI
PAR
PAR
MERGE
DAPI
Zoom
DAPI
PAR
PAR
MERGE
DAPI
Zoom
DAPI
PAR
PAR
MERGE

FIG. 4

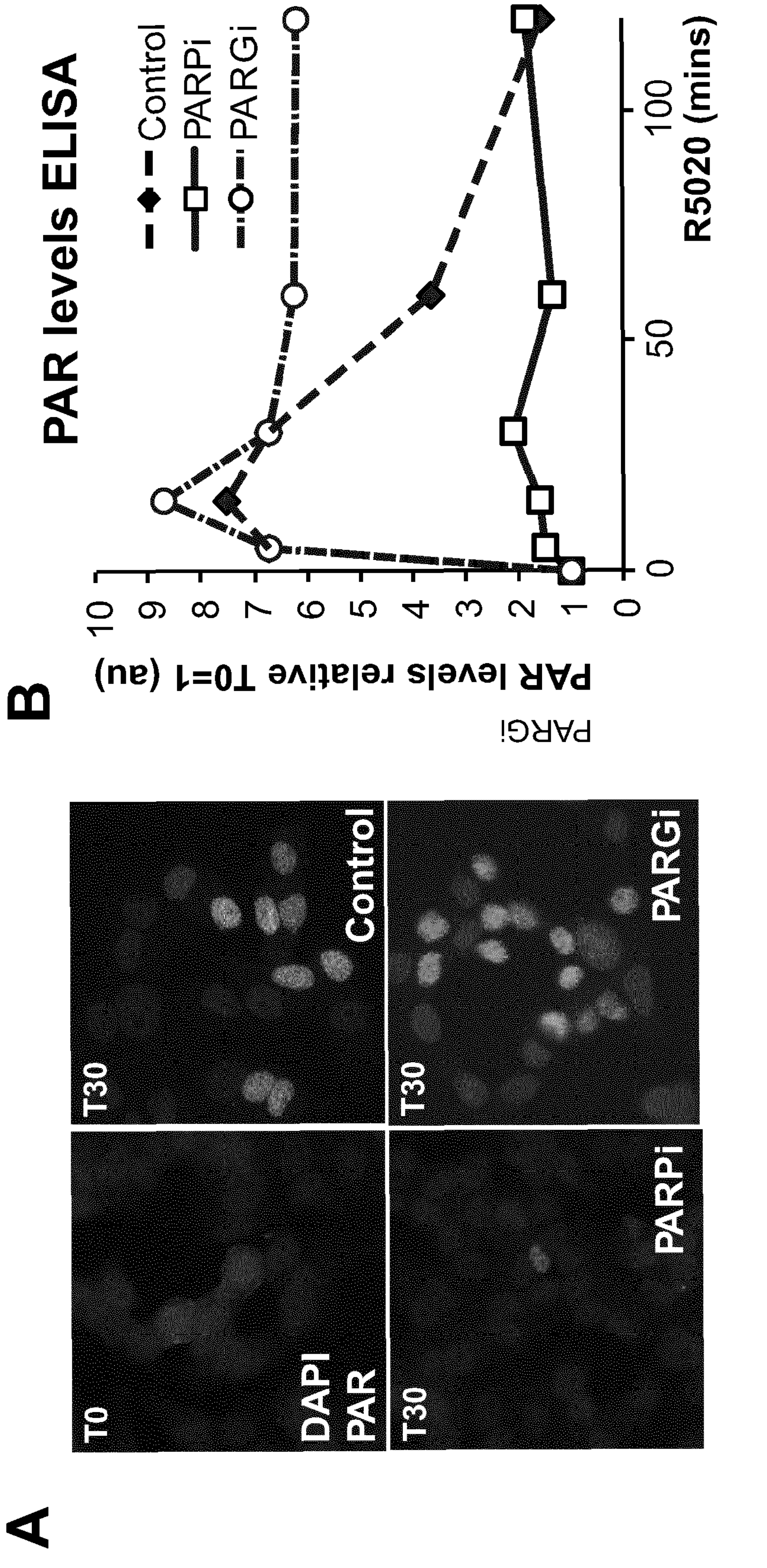
A
T0
DAPI
PAR
T30
PARPi
T30
Control
T30
PARGi
B
PAR levels ELISA
Control
PARPi
PARGi
PAR levels relative T0=1 (au)
10
9
8
7
6
5
4
3
2
1
0
0
50
100
R5020 (mins)

FIG. 5

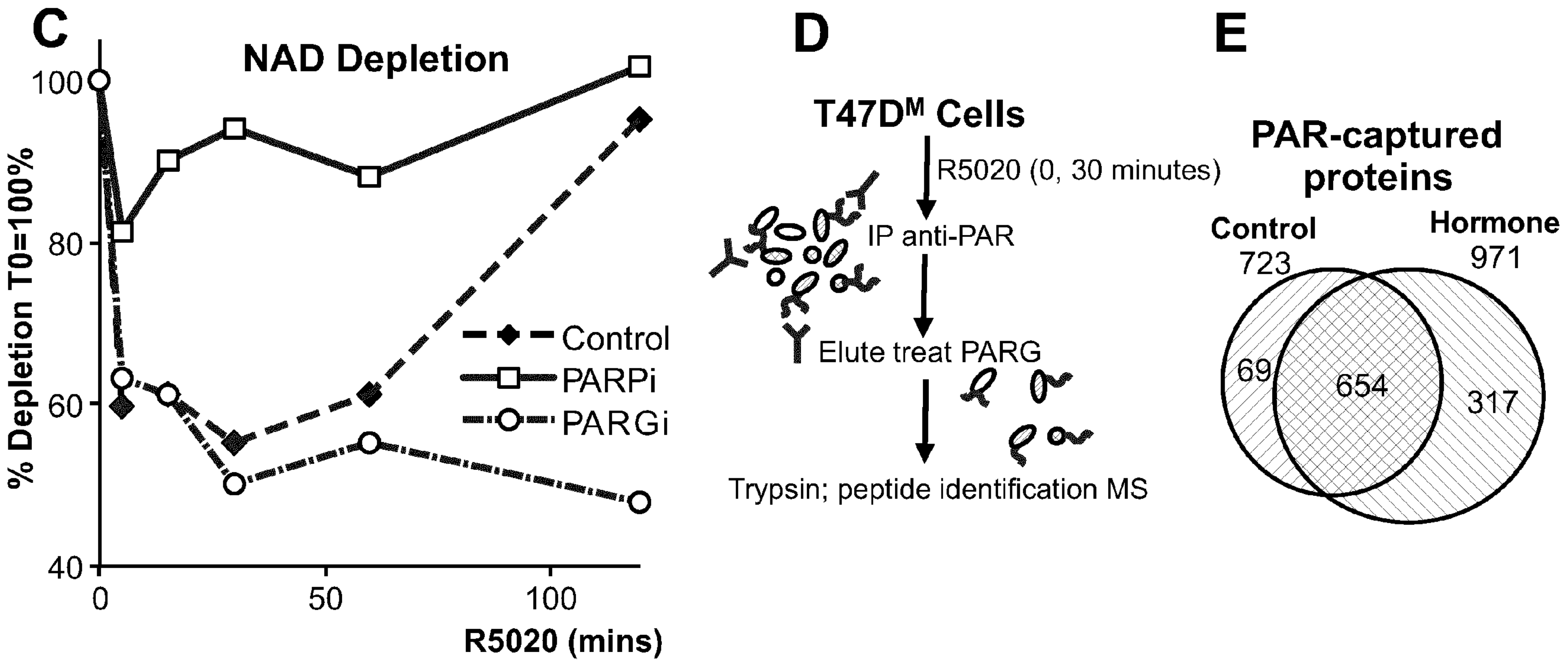
C
NAD Depletion
% Depletion T0=100%
100
80
60
40
0
50
100
R5020 (mins)
Control
PARPi
PARGi
D
T47D^M Cells
R5020 (0, 30 minutes)
IP anti-PAR
Elute treat PARG
Trypsin; peptide identification MS
E
PAR-captured proteins
Control
723
Hormone
971
69
654
317

FIG. 5 (cont. 1)

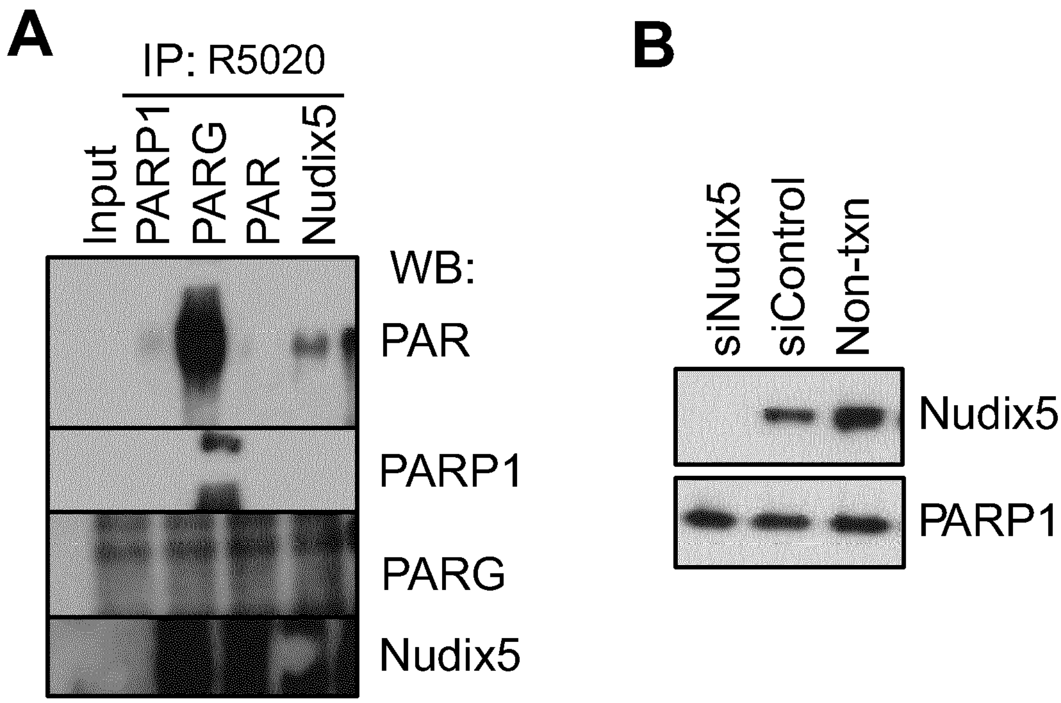
A
IP: R5020
Input
PARP1
PARG
PAR
Nudix5
WB:
PAR
PARP1
PARG
Nudix5
B
siNudix5
siControl
Non-txn
Nudix5
PARP1

FIG. 6

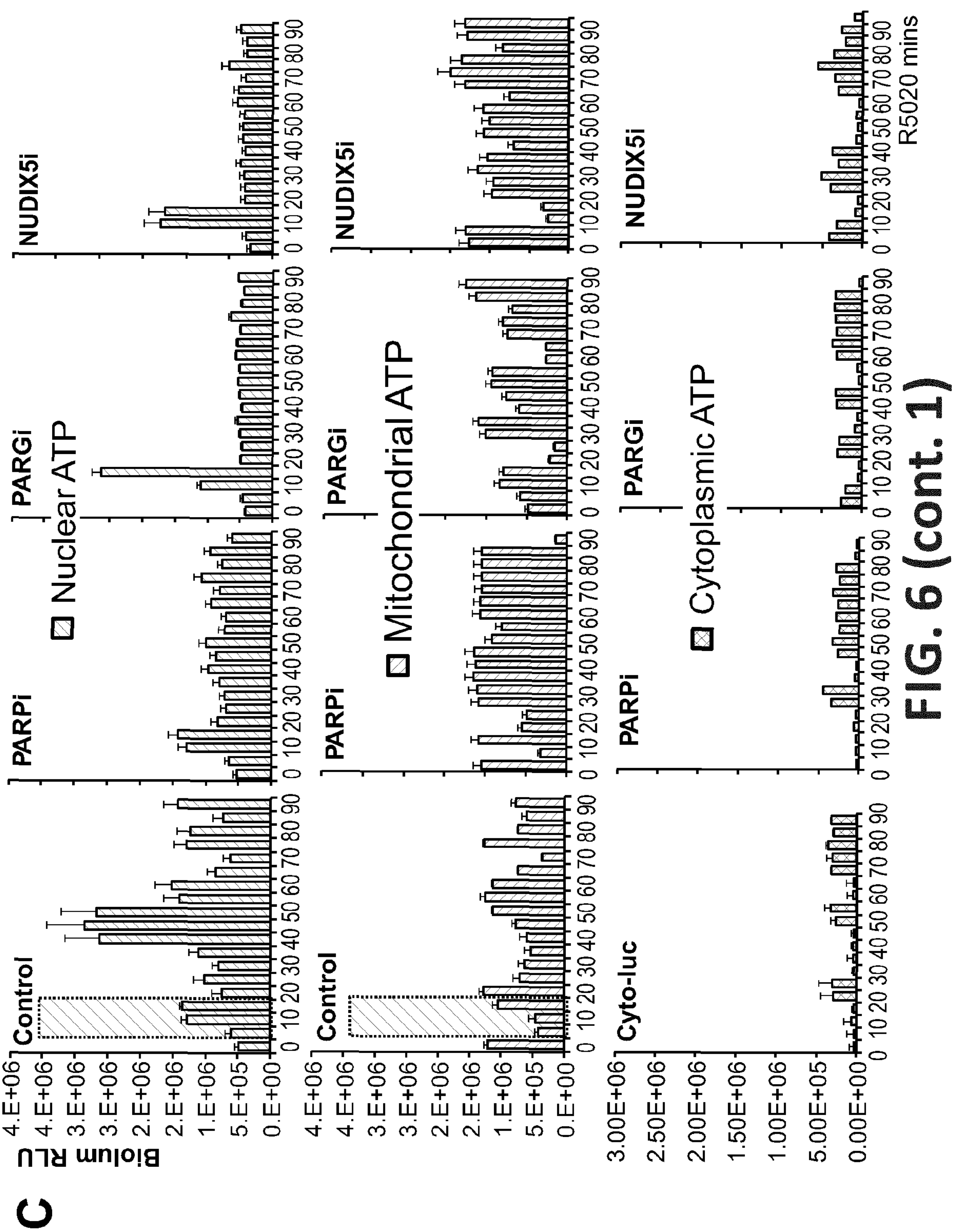
C
Biolum RLU
Control
PARPi
PARGi
NUDIX5i
Cyto-luc
Nuclear ATP
Mitochondrial ATP
Cytoplasmic ATP
R5020 mins

FIG. 6 (cont. 1)

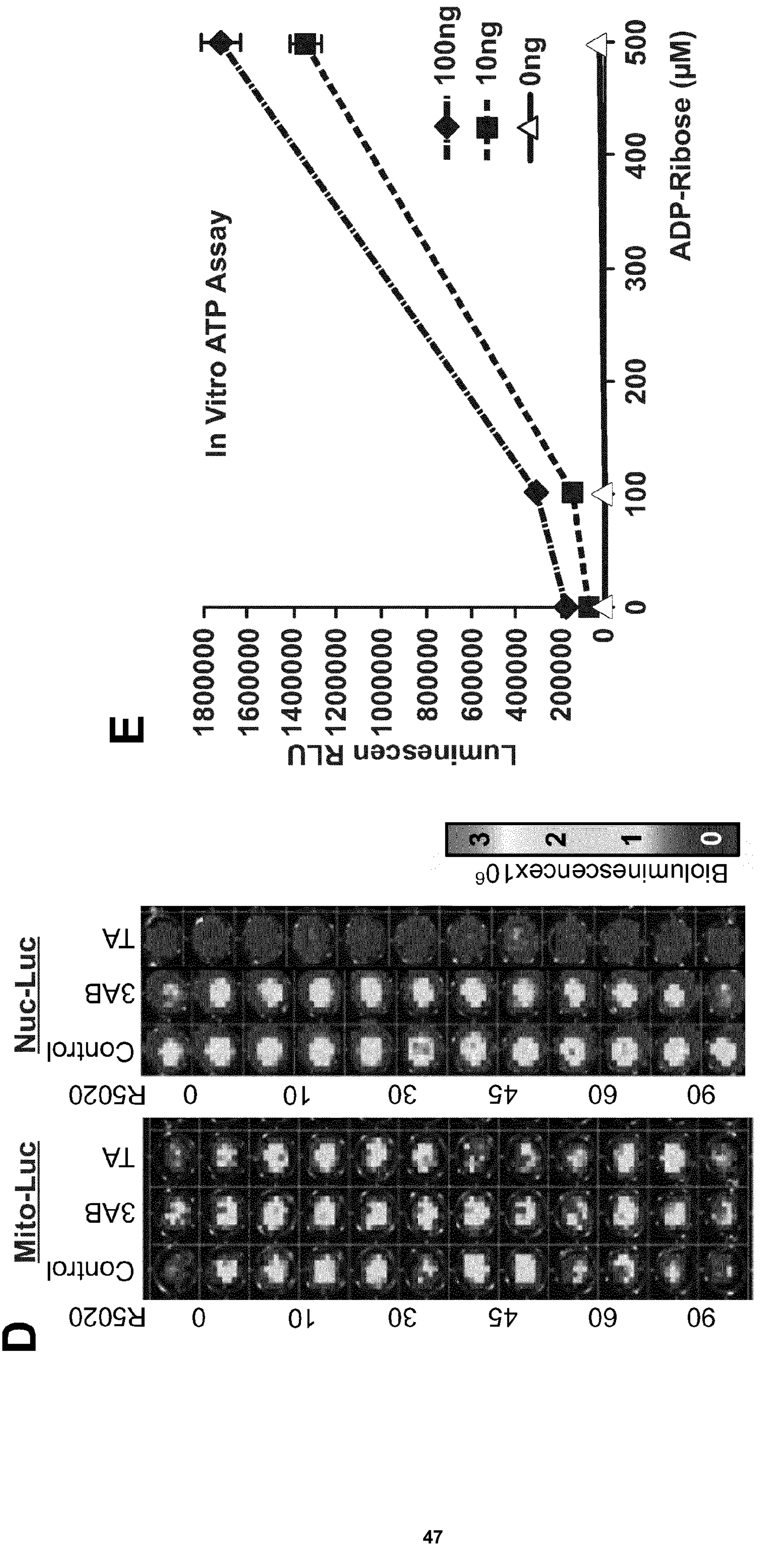
D
Mito-Luc
Nuc-Luc
R5020
Control
3AB
TA
0
10
30
45
60
90
Bioluminescencex10^6
3
2
1
0
E
In Vitro ATP Assay
Luminescen RLU
18000000
16000000
14000000
12000000
10000000
8000000
6000000
4000000
2000000
0
0
100
200
300
400
500
ADP-Ribose (μM)
100ng
10ng
0ng

FIG. 6 (cont. 2)

A

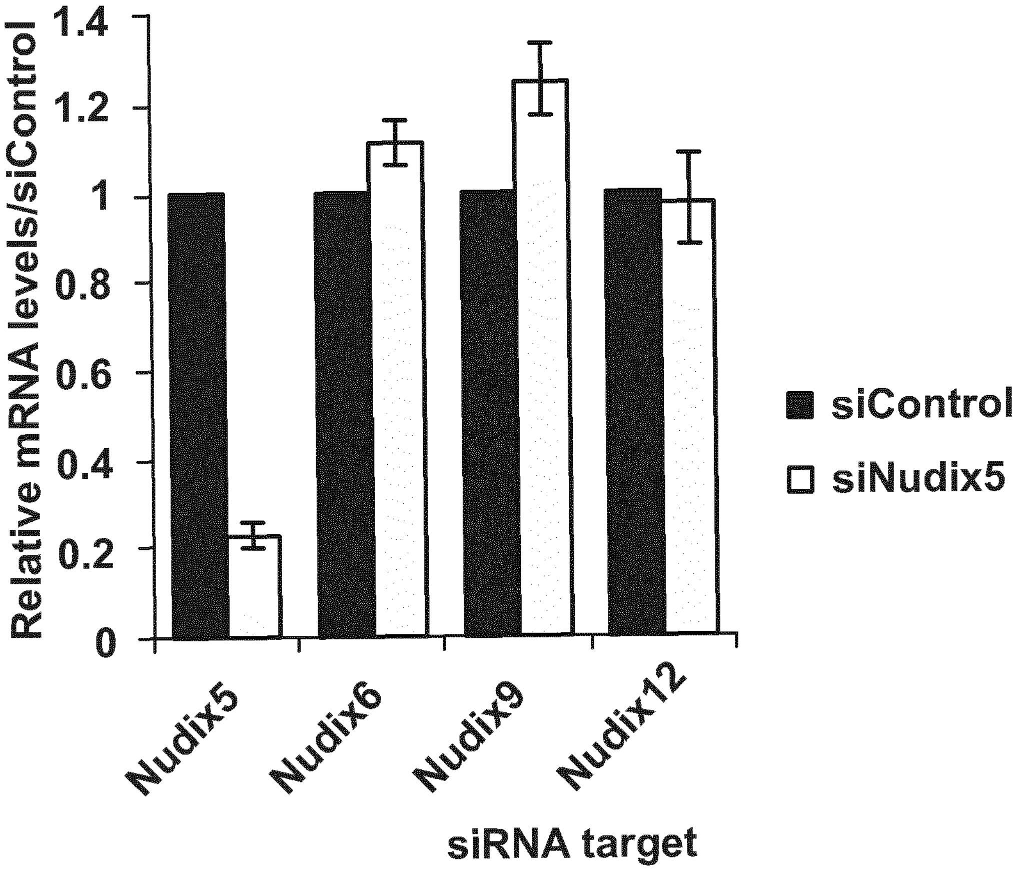
Relative mRNA levels/siControl
1.4
1.2
1
0.8
0.6
0.4
0.2
0
Nudix5
Nudix6
Nudix9
Nudix12
siRNA target
siControl
siNudix5

FIG. 7

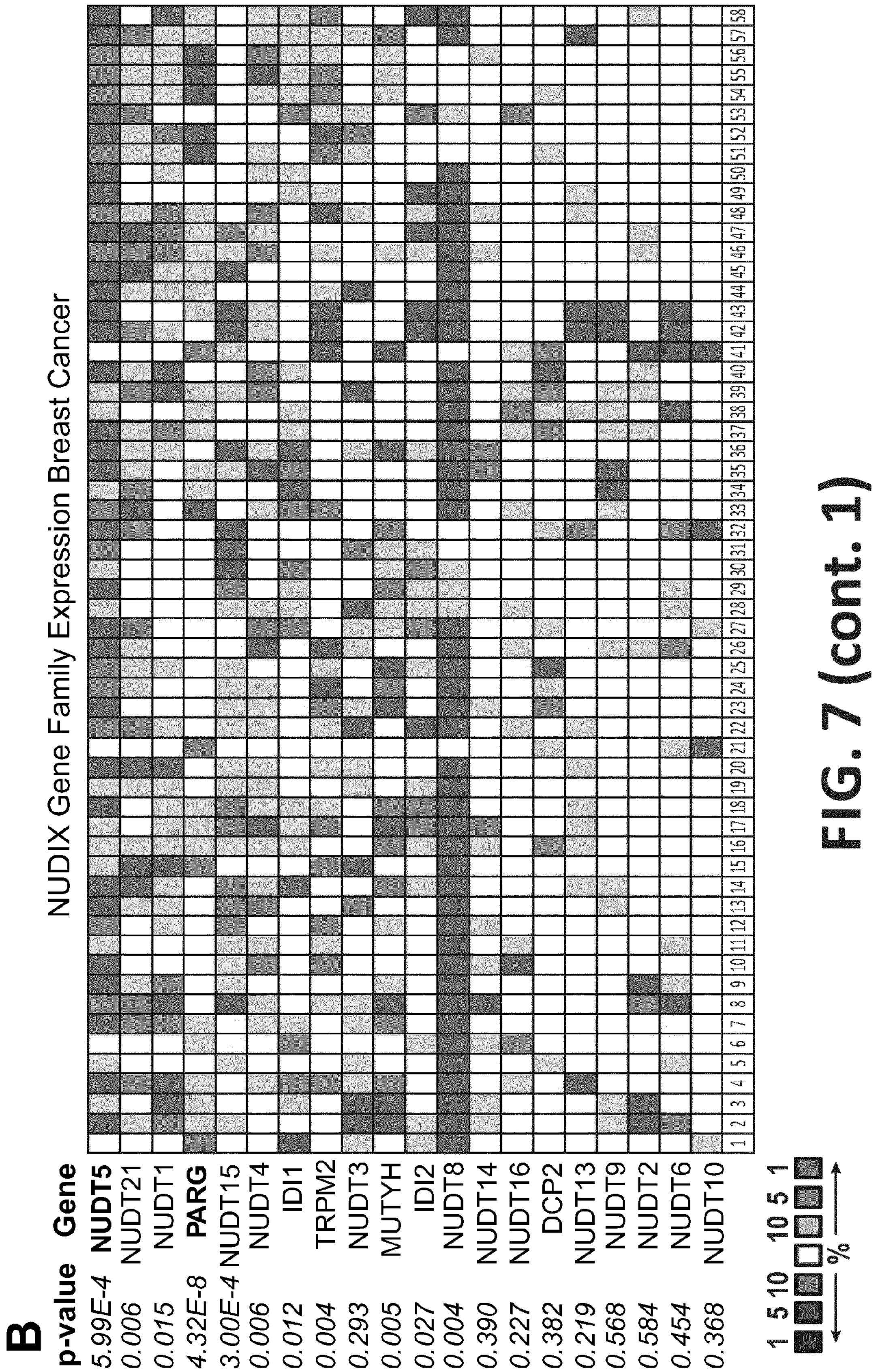
B
NUDIX Gene Family Expression Breast Cancer
p-value Gene
5.99E-4 NUDT5
0.006 NUDT21
0.015 NUDT1
4.32E-8 PARG
3.00E-4 NUDT15
0.006 NUDT4
0.012 IDI1
0.004 TRPM2
0.293 NUDT3
0.005 MUTYH
0.027 IDI2
0.004 NUDT8
0.390 NUDT14
0.227 NUDT16
0.382 DCP2
0.219 NUDT13
0.568 NUDT9
0.584 NUDT2
0.454 NUDT6
0.368 NUDT10
1 2 3 4 5 6 7 8 9 10 11 12 13 14 15 16 17 18 19 20 21 22 23 24 25 26 27 28 29 30 31 32 33 34 35 36 37 38 39 40 41 42 43 44 45 46 47 48 49 50 51 52 53 54 55 56 57 58
1 5 10 10 5 1
%

FIG. 7 (cont. 1)

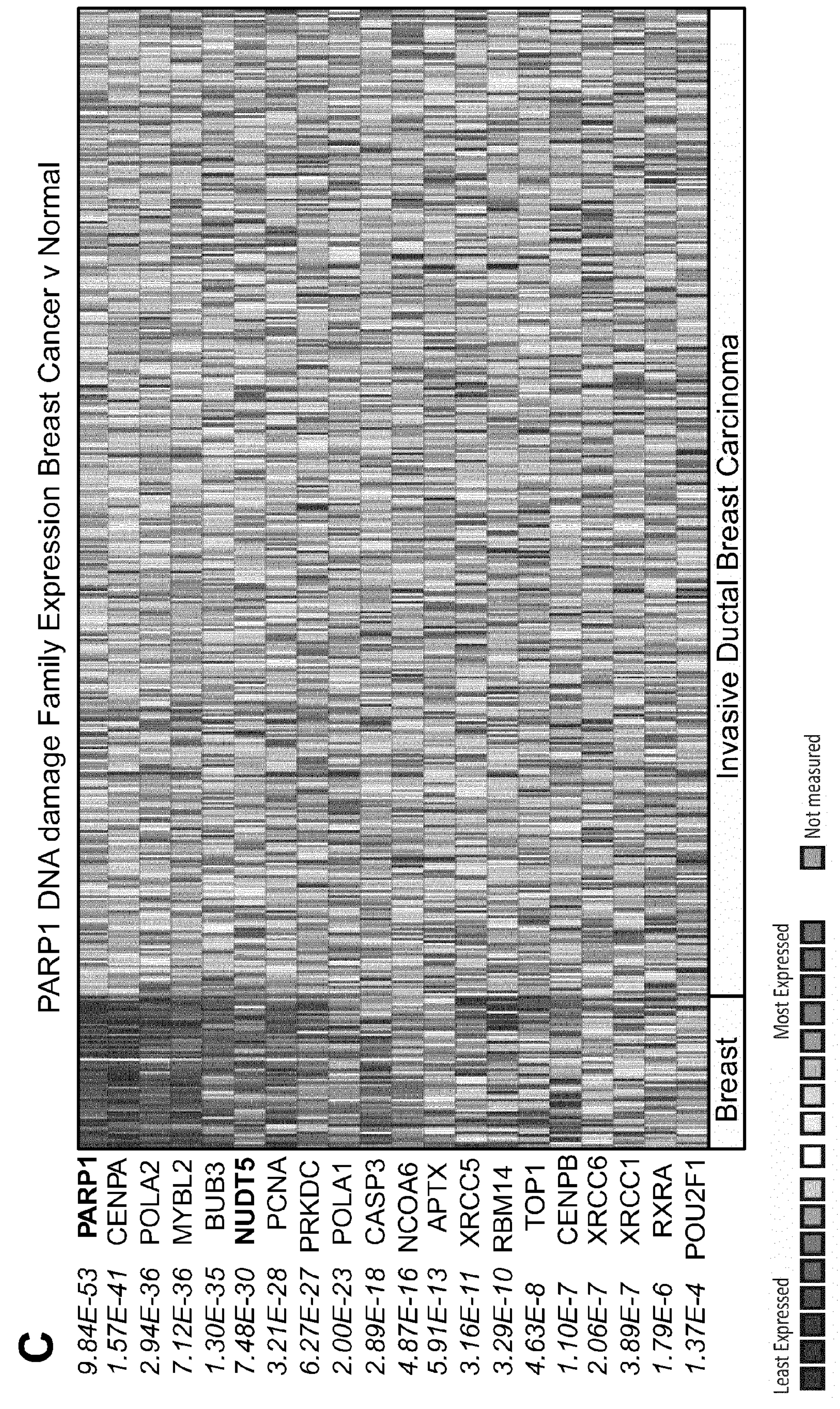
C
PARP1 DNA damage Family Expression Breast Cancer v Normal
9.84E-53 PARP1
1.57E-41 CENPA
2.94E-36 POLA2
7.12E-36 MYBL2
1.30E-35 BUB3
7.48E-30 NUDT5
3.21E-28 PCNA
6.27E-27 PRKDC
2.00E-23 POLA1
2.89E-18 CASP3
4.87E-16 NCOA6
5.91E-13 APTX
3.16E-11 XRCC5
3.29E-10 RBM14
4.63E-8 TOP1
1.10E-7 CENPB
2.06E-7 XRCC6
3.89E-7 XRCC1
1.79E-6 RXRA
1.37E-4 POU2F1
Breast
Invasive Ductal Breast Carcinoma
Least Expressed
Most Expressed
Not measured

FIG. 7 (cont. 2)

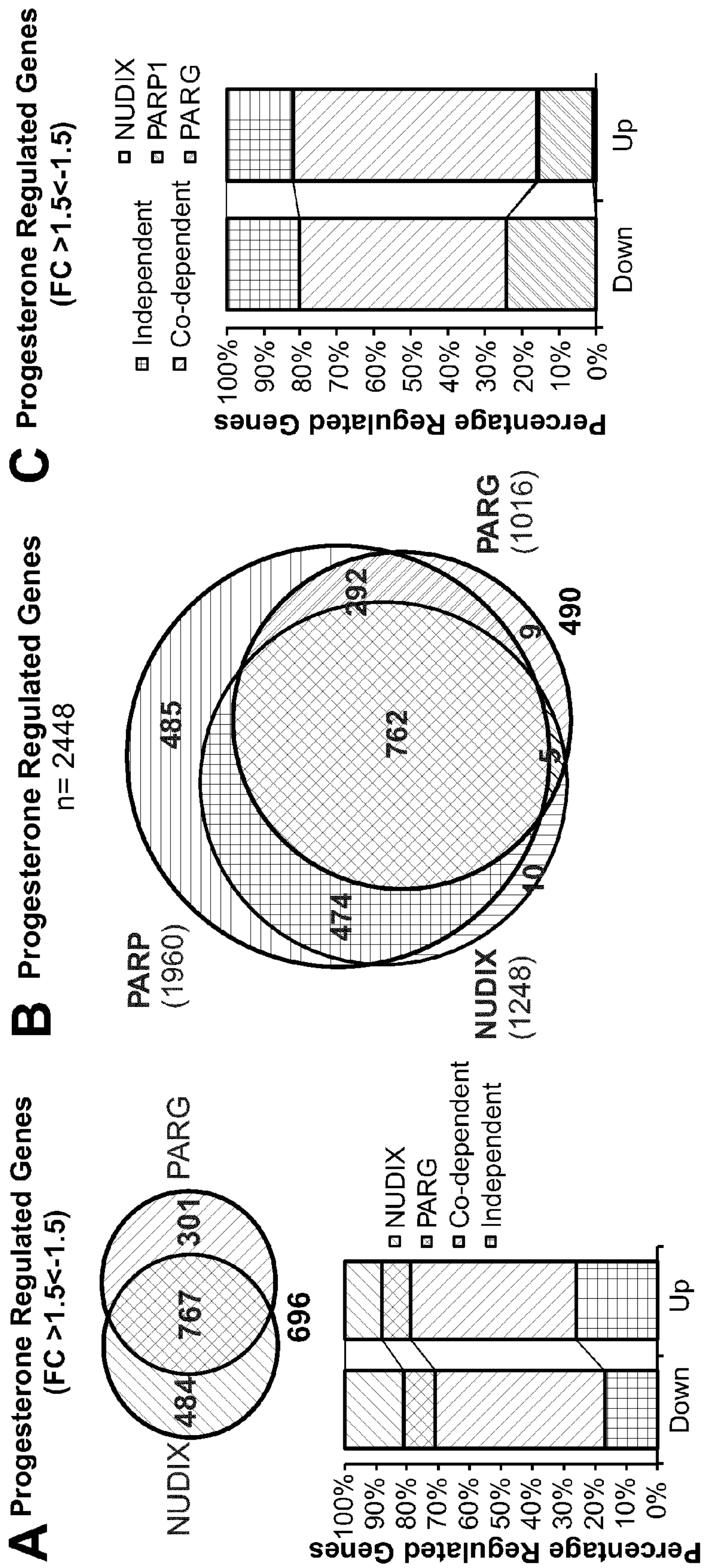
A
Progesterone Regulated Genes
(FC >1.5<-1.5)
NUDIX
PARG
484
767
301
696
Percentage Regulated Genes
100%
90%
80%
70%
60%
50%
40%
30%
20%
10%
0%
Down
Up
NUDIX
PARG
Co-dependent
Independent
B
Progesterone Regulated Genes
n= 2448
PARP
(1960)
NUDIX
(1248)
PARG
(1016)
485
474
762
292
10
5
9
490
C
Progesterone Regulated Genes
(FC >1.5<-1.5)
Independent
Co-dependent
NUDIX
PARP1
PARG
Percentage Regulated Genes
100%
90%
80%
70%
60%
50%
40%
30%
20%
10%
0%
Down
Up

FIG. 8

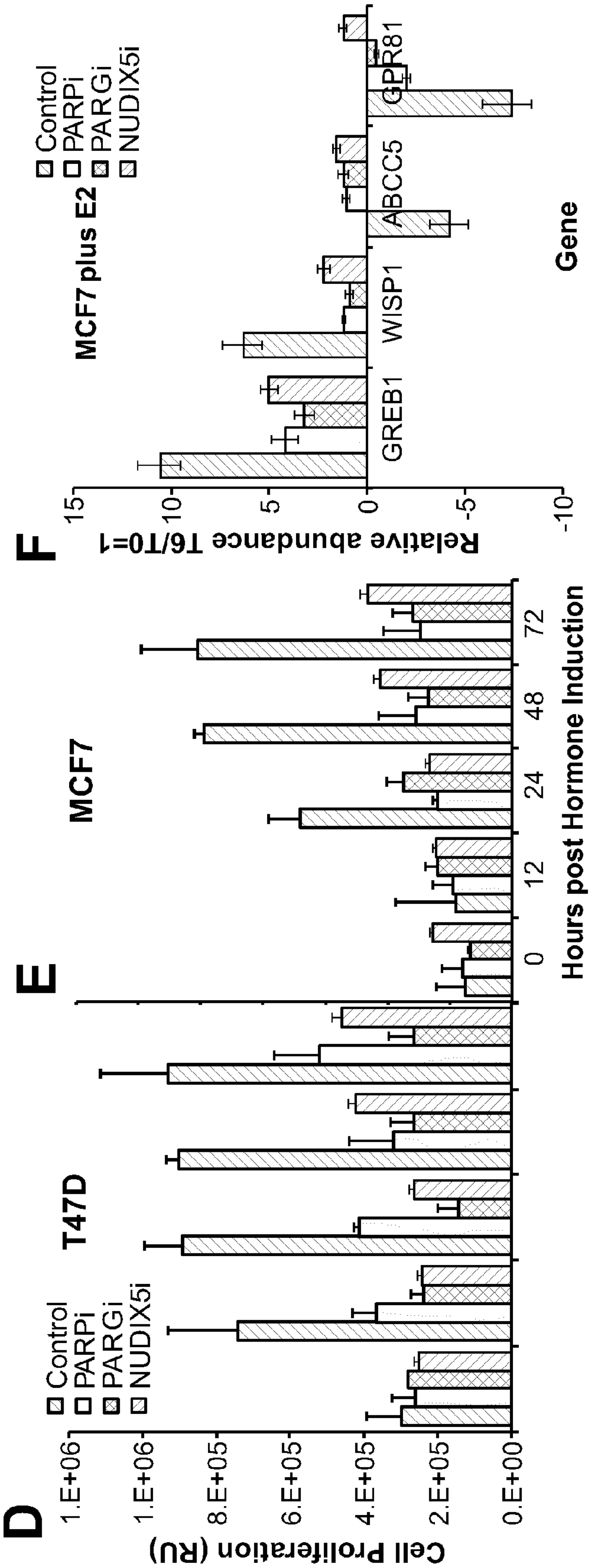

D
Cell Proliferation (RU)
1.E+06
1.E+06
8.E+05
6.E+05
4.E+05
2.E+05
0.E+00
Control
PARPi
PARGi
NUDIX5i
T47D
E
MCF7
0
12
24
48
72
Hours post Hormone Induction
F
MCF7 plus E2
Control
PARPi
PARGi
NUDIX5i
Relative abundance T6/T0=1
15
10
5
0
-5
-10
GREB1
WISP1
ABCC5
GPR81
Gene


FIG. 8 (cont. 1)

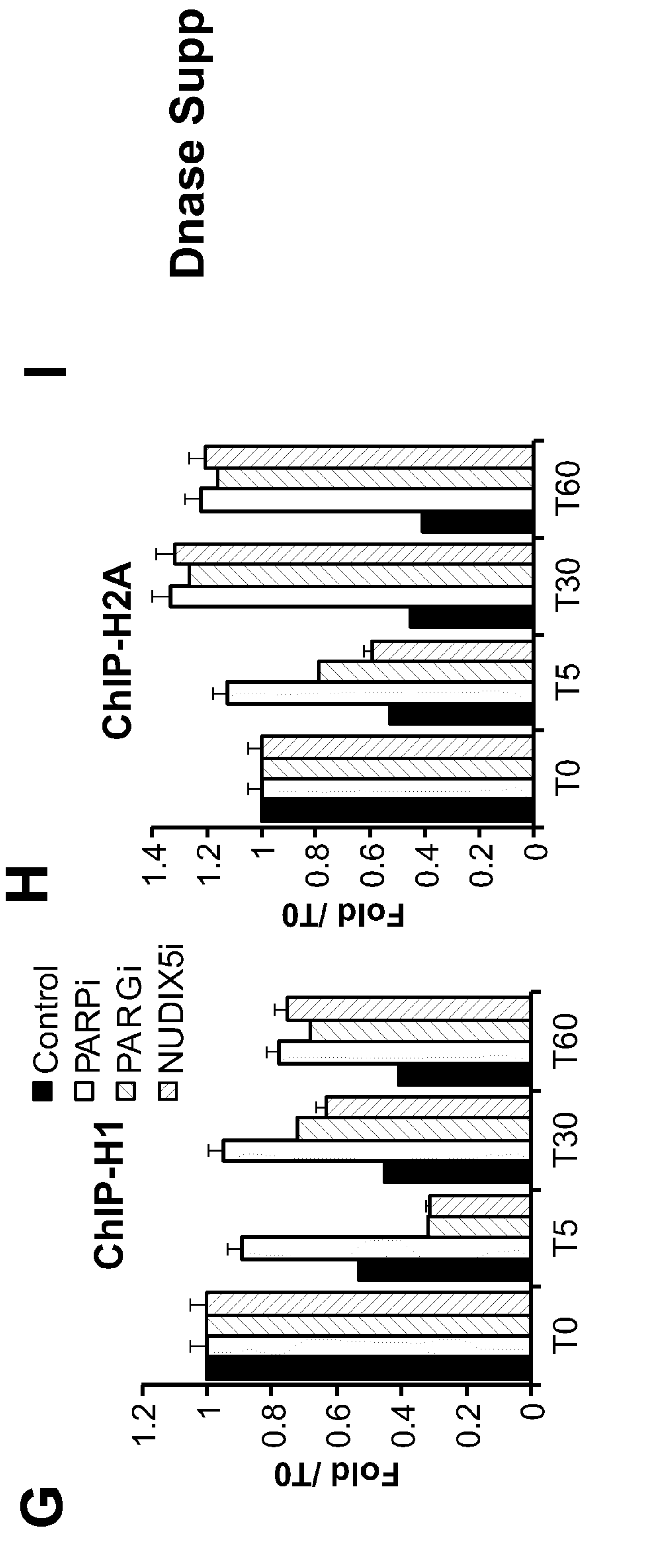
G
ChIP-H1
Control
PARPi
PARGi
NUDIX5i
Fold /T0
1.2
1
0.8
0.6
0.4
0.2
0
T0
T5
T30
T60
H
ChIP-H2A
Fold /T0
1.4
1.2
1
0.8
0.6
0.4
0.2
0
T0
T5
T30
T60
I
Dnase Supp

FIG. 8 (cont. 2)

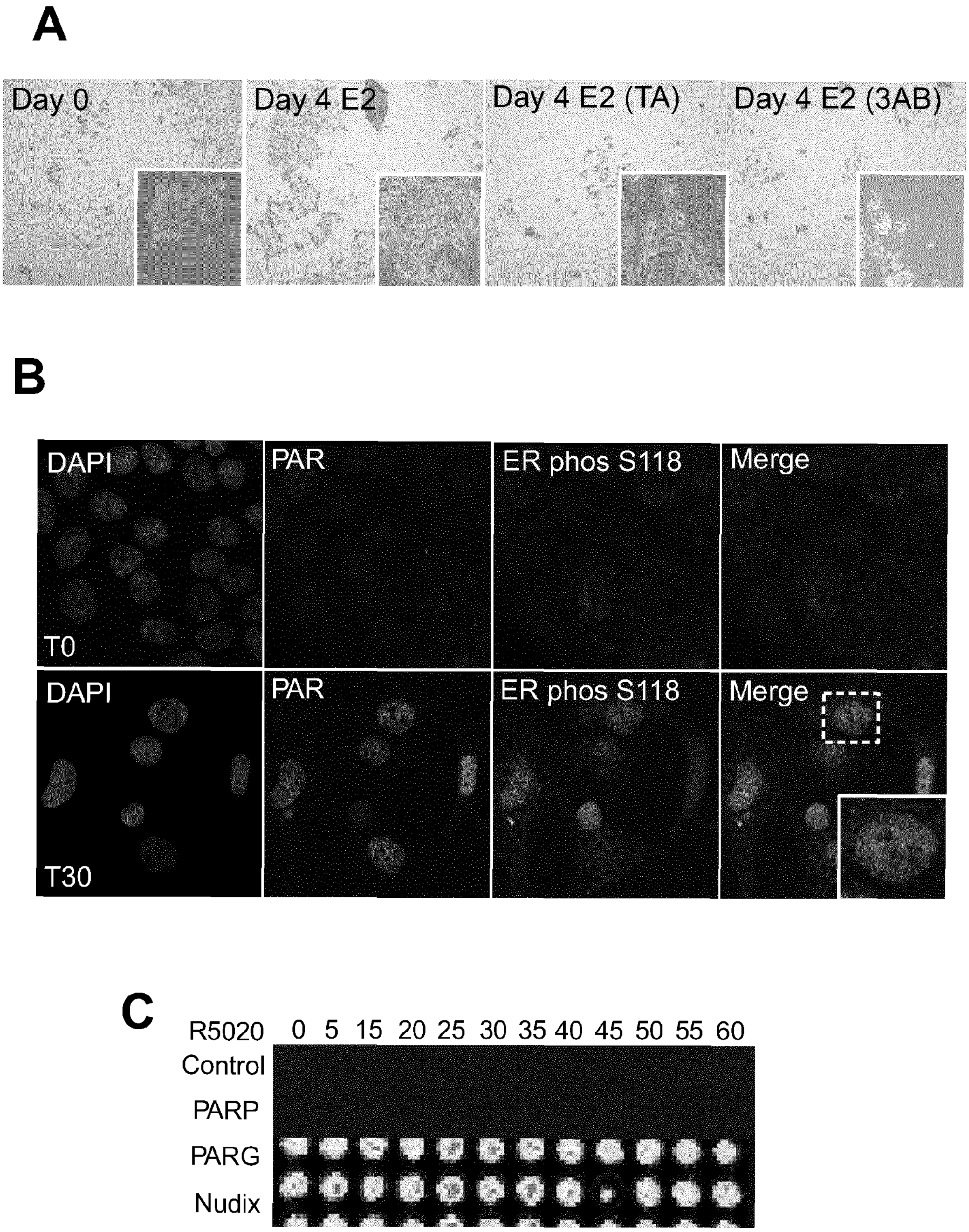
A
Day 0
Day 4 E2
Day 4 E2 (TA)
Day 4 E2 (3AB)
B
DAPI
PAR
ER phos S118
Merge
T0
DAPI
PAR
ER phos S118
Merge
T30
C
R5020 0 5 15 20 25 30 35 40 45 50 55 60
Control
PARP
PARG
Nudix

FIG. 9

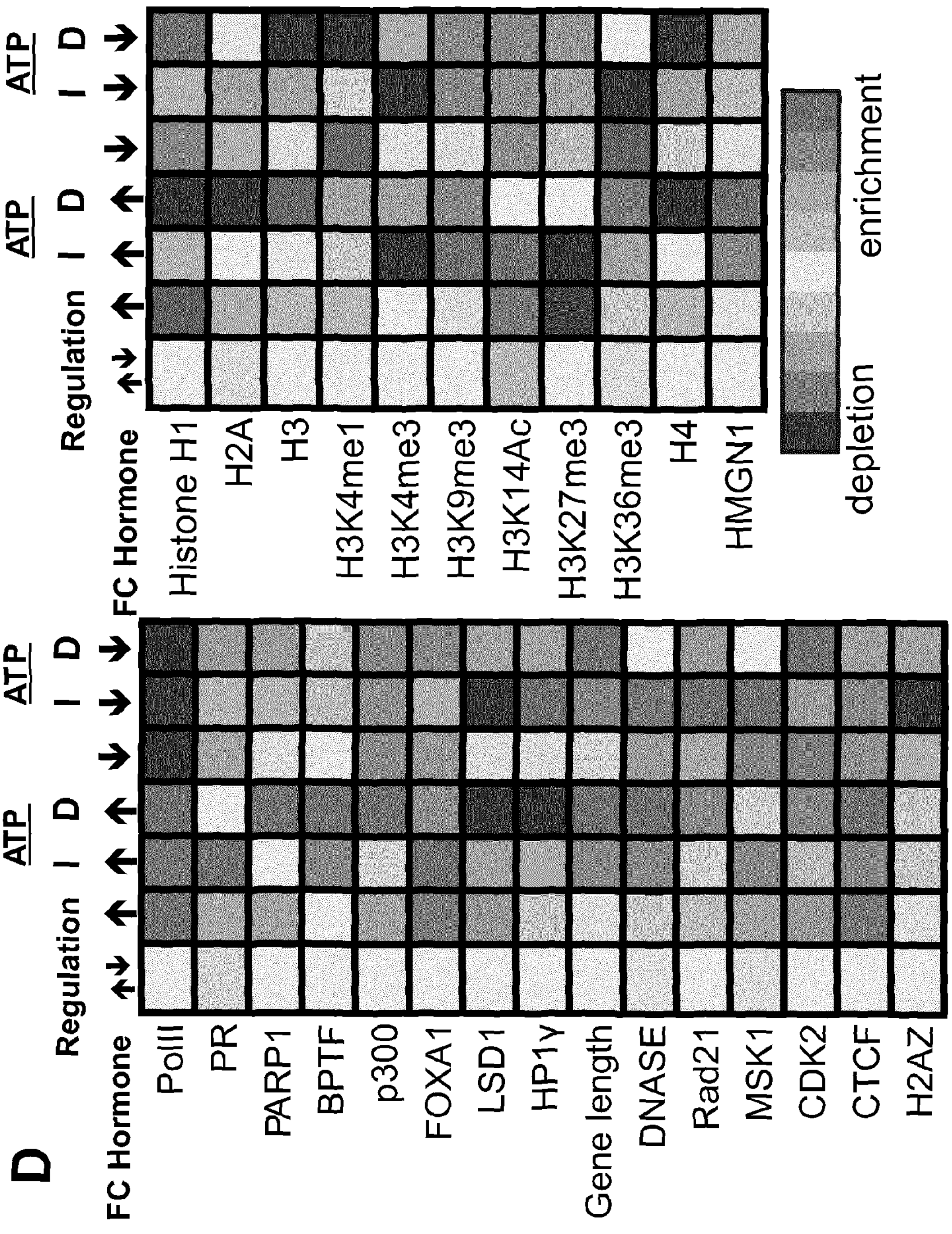
D
FC Hormone
Regulation
ATP
I
D
ATP
I
D
PolII
PR
PARP1
BPTF
p300
FOXA1
LSD1
HP1γ
Gene length
DNASE
Rad21
MSK1
CDK2
CTCF
H2AZ
FC Hormone
Regulation
ATP
I
D
ATP
I
D
Histone H1
H2A
H3
H3K4me1
H3K4me3
H3K9me3
H3K14Ac
H3K27me3
H3K36me3
H4
HMGN1
depletion
enrichment

FIG. 9 (cont. 1)

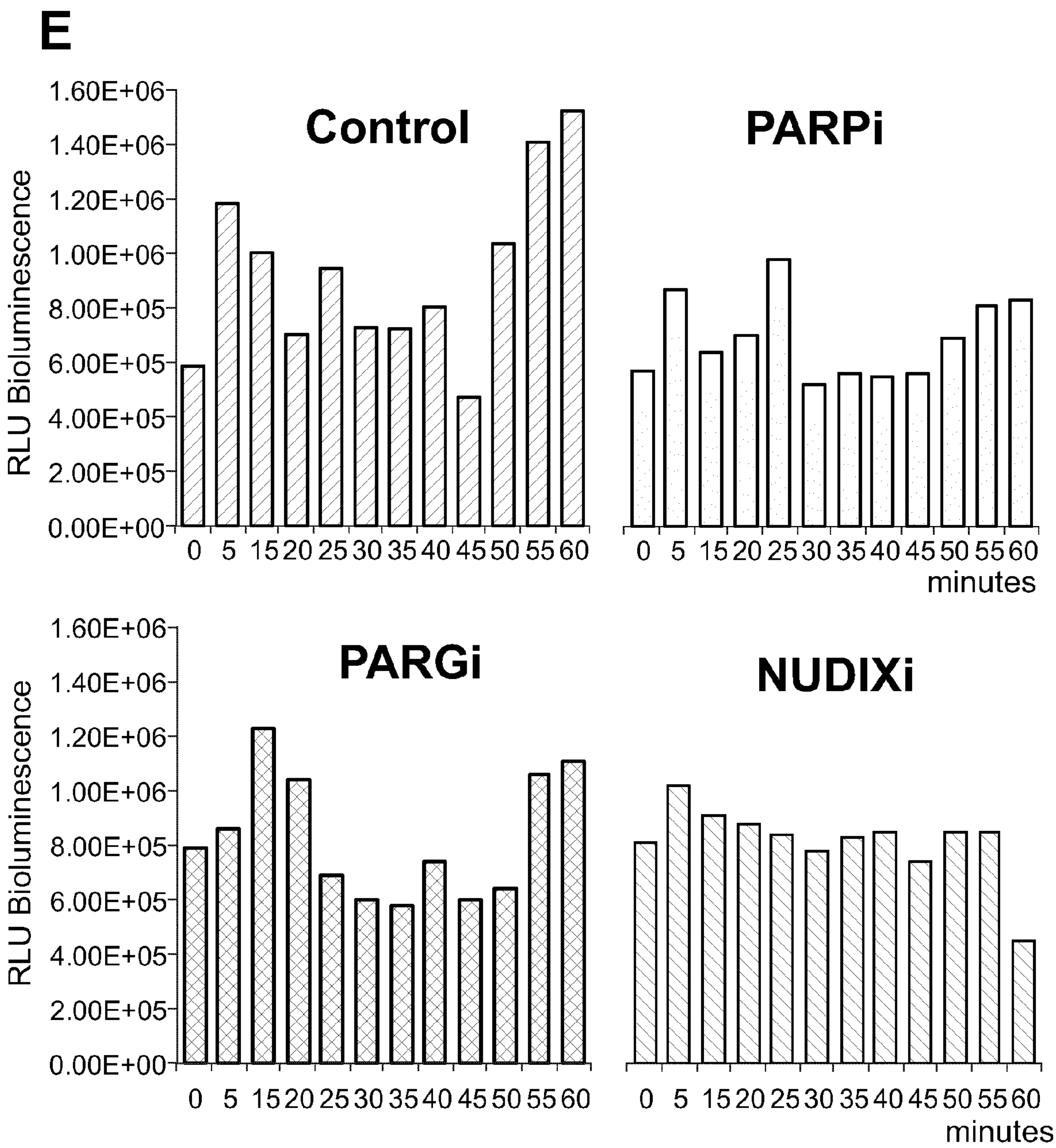
E
Control
PARPi
PARGi
NUDIXi
RLU Bioluminescence
1.60E+06
1.40E+06
1.20E+06
1.00E+06
8.00E+05
6.00E+05
4.00E+05
2.00E+05
0.00E+00
0 5 15 20 25 30 35 40 45 50 55 60
minutes

FIG. 9 (cont. 2)

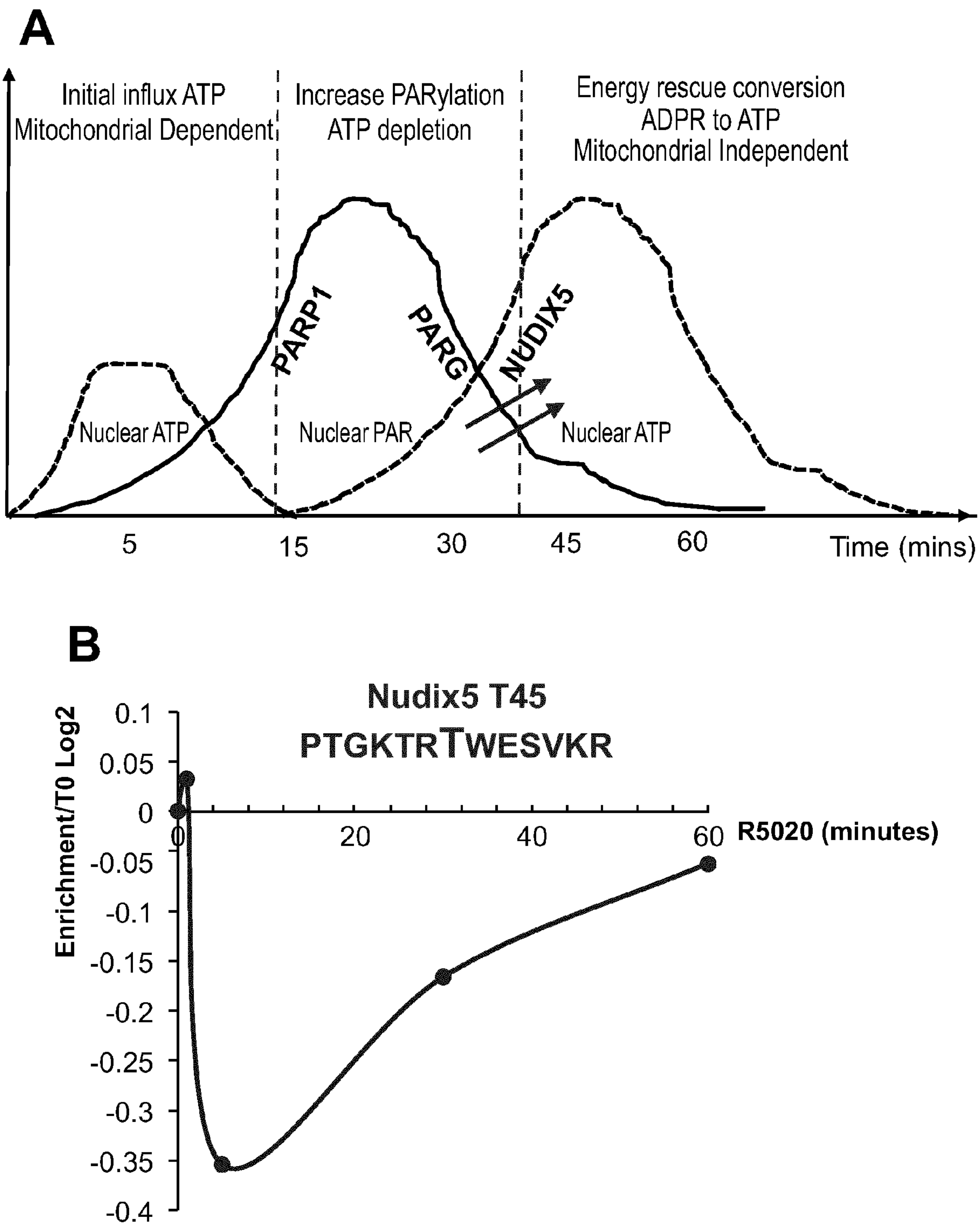
A
Initial influx ATP
Mitochondrial Dependent
Increase PARylation
ATP depletion
Energy rescue conversion
ADPR to ATP
Mitochondrial Independent
PARP1
PARG
NUDIX5
Nuclear ATP
Nuclear PAR
Nuclear ATP
5
15
30
45
60
Time (mins)
B
Nudix5 T45
PTGKTRTWESVKR
Enrichment/T0 Log2
0.1
0.05
0
-0.05
-0.1
-0.15
-0.2
-0.25
-0.3
-0.35
-0.4
0
20
40
60
R5020 (minutes)

FIG. 10

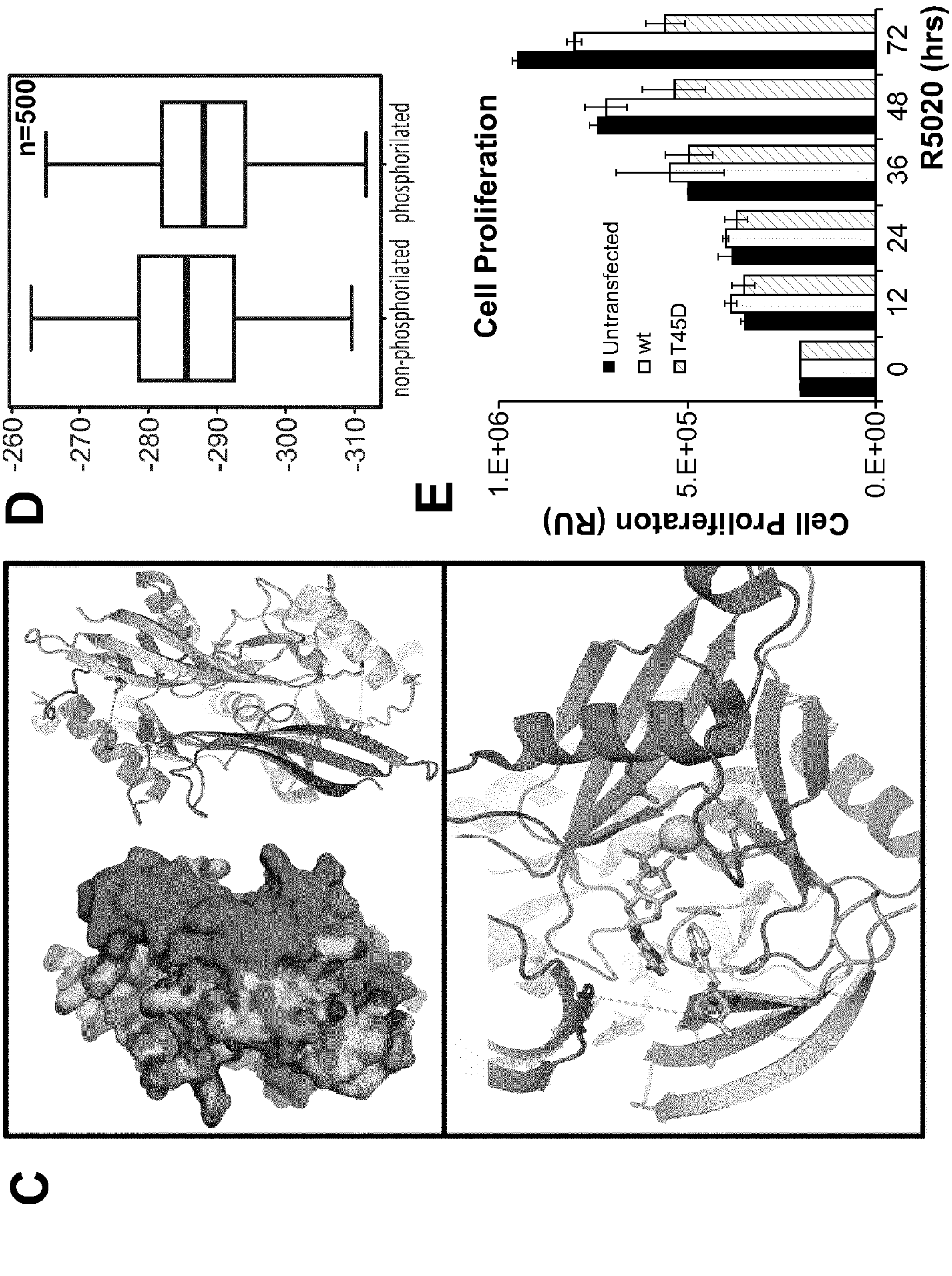
C
D
-260
-270
-280
-290
-300
-310
n=500
non-phosphorilated
phosphorilated
E
Cell Proliferation
Untransfected
wt
T45D
1.E+06
5.E+05
0.E+00
Cell Proliferaton (RU)
0
12
24
36
48
72
R5020 (hrs)

FIG. 10 (cont. 1)

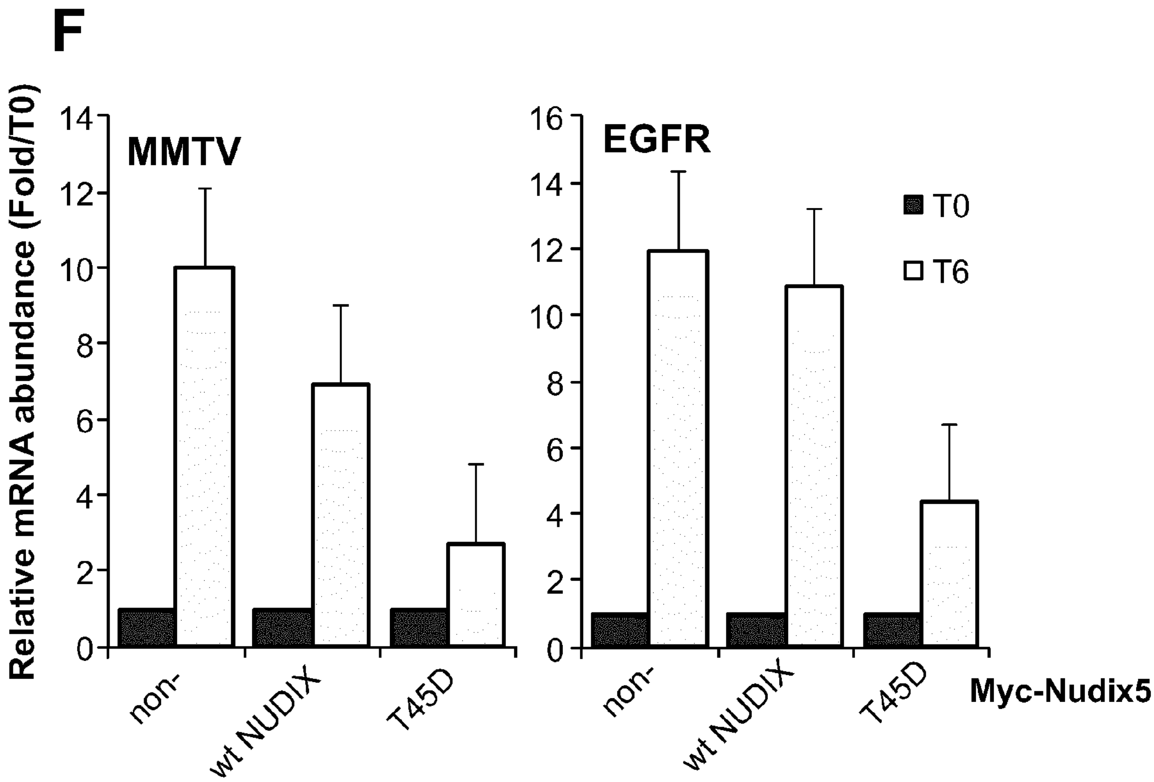
F
Relative mRNA abundance (Fold/T0)
MMTV
EGFR
0
2
4
6
8
10
12
14
16
T0
T6
non-
wt NUDIX
T45D
Myc-Nudix5

FIG. 10 (cont. 2)

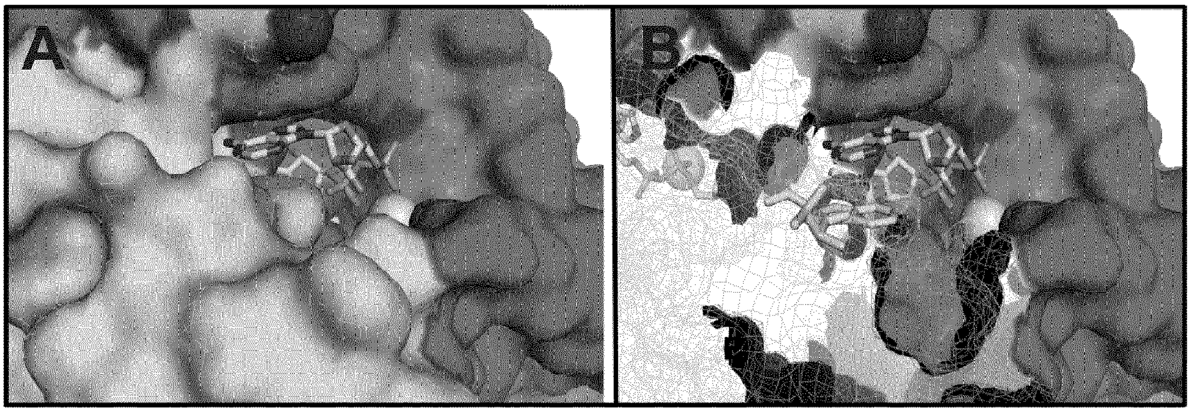


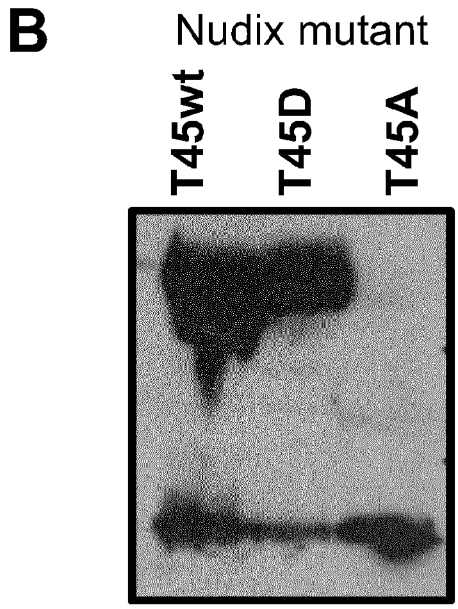


FIG. 11

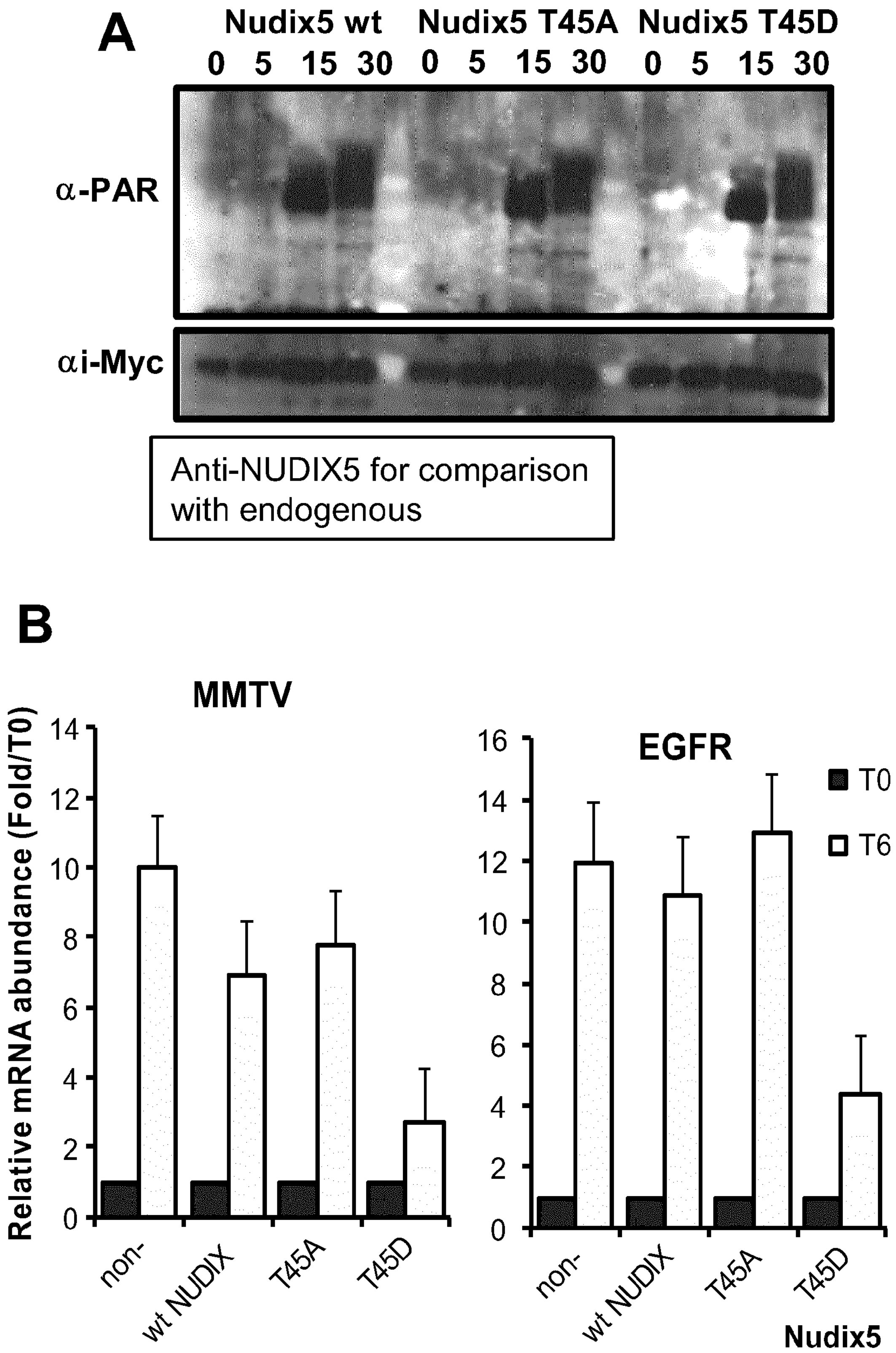
A
Nudix5 wt
Nudix5 T45A
Nudix5 T45D
0 5 15 30 0 5 15 30 0 5 15 30
α-PAR
αi-Myc
Anti-NUDIX5 for comparison with endogenous
B
MMTV
Relative mRNA abundance (Fold/T0)
14
12
10
8
6
4
2
0
non-
wt NUDIX
T45A
T45D
EGFR
16
14
12
10
8
6
4
2
0
T0
T6
Nudix5

FIG. 12

C

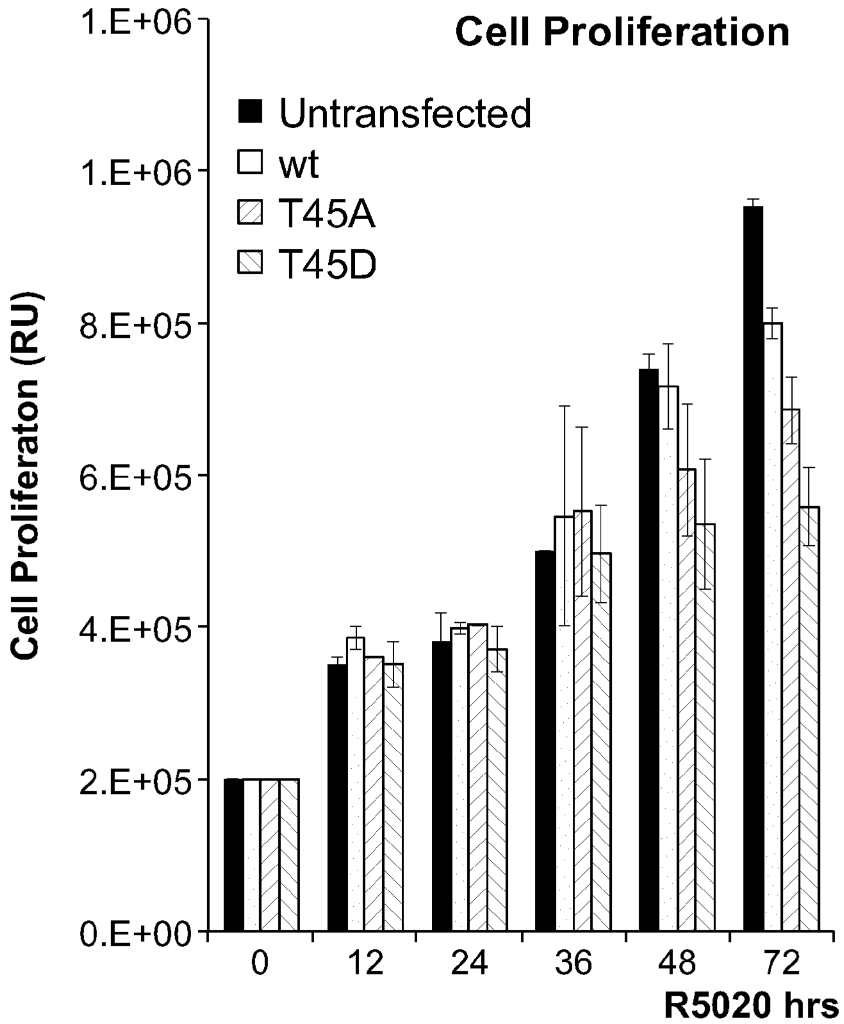


D

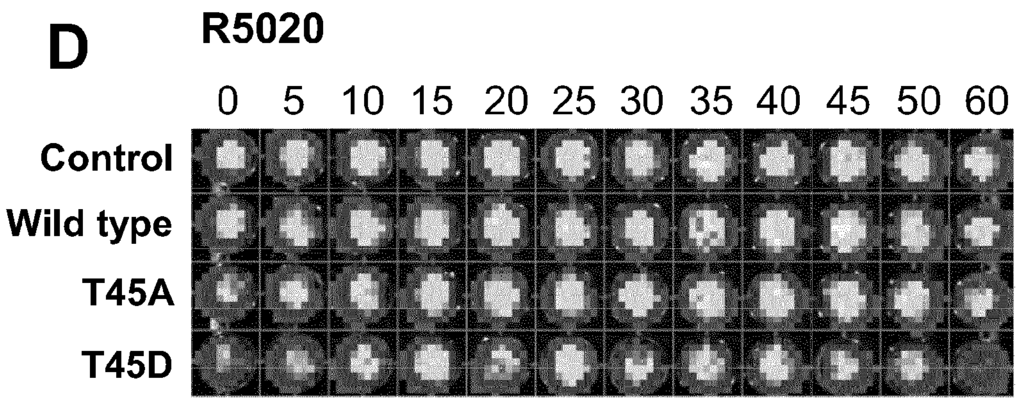


FIG. 12 (cont. 1)

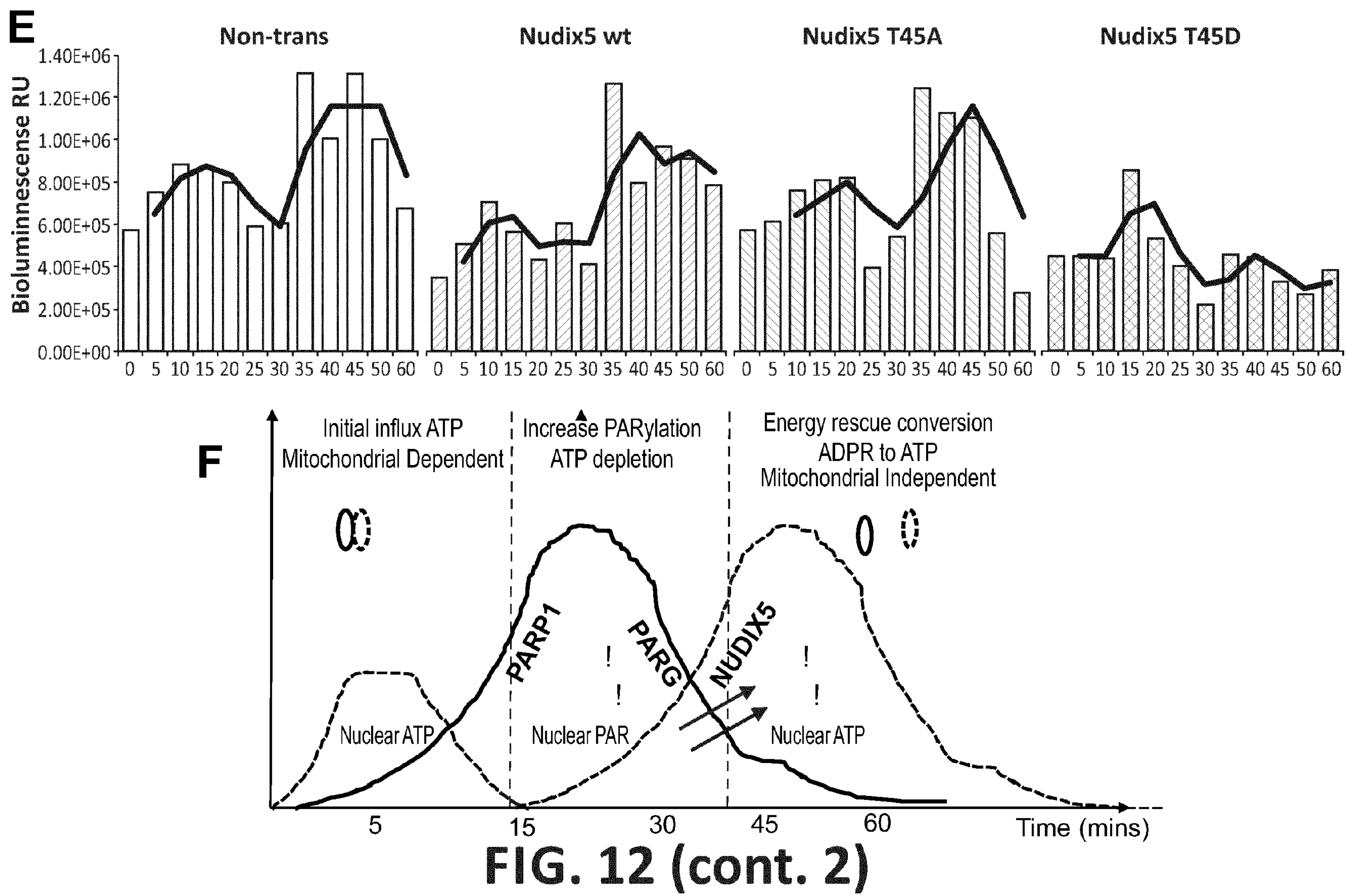
E
Non-trans
Nudix5 wt
Nudix5 T45A
Nudix5 T45D
Bioluminnescense RU
1.40E+06
1.20E+06
1.00E+06
8.00E+05
6.00E+05
4.00E+05
2.00E+05
0.00E+00
0 5 10 15 20 25 30 35 40 45 50 60
F
Initial influx ATP
Mitochondrial Dependent
Increase PARylation
ATP depletion
Energy rescue conversion
ADPR to ATP
Mitochondrial Independent
PARP1
PARG
NUDIX5
Nuclear ATP
Nuclear PAR
Nuclear ATP
5
15
30
45
60
Time (mins)

FIG. 12 (cont. 2)

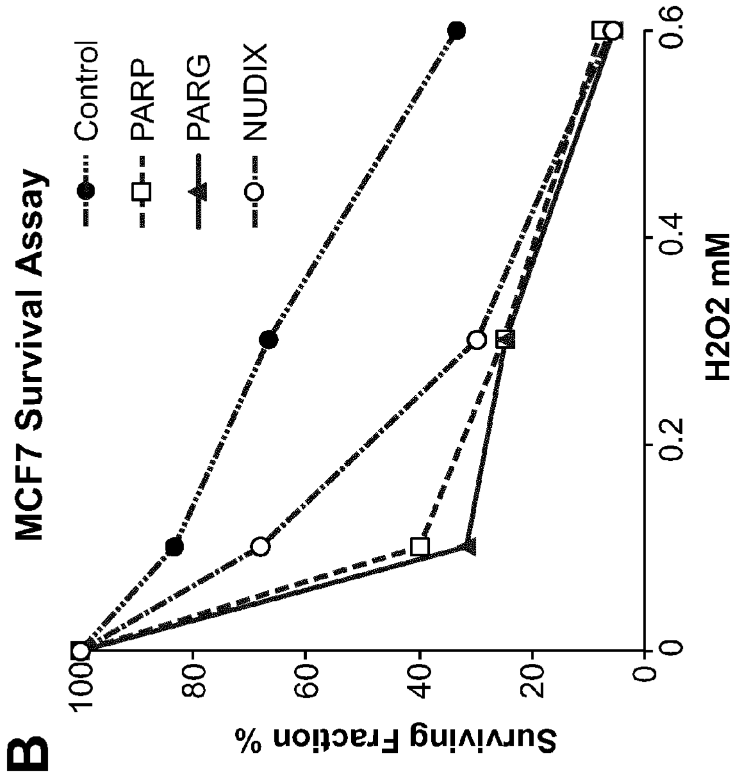
B
MCF7 Survival Assay
Control
PARP
PARG
NUDIX
Surviving Fraction %
100
80
60
40
20
0
0
0.2
0.4
0.6
H2O2 mM

FIG. 13

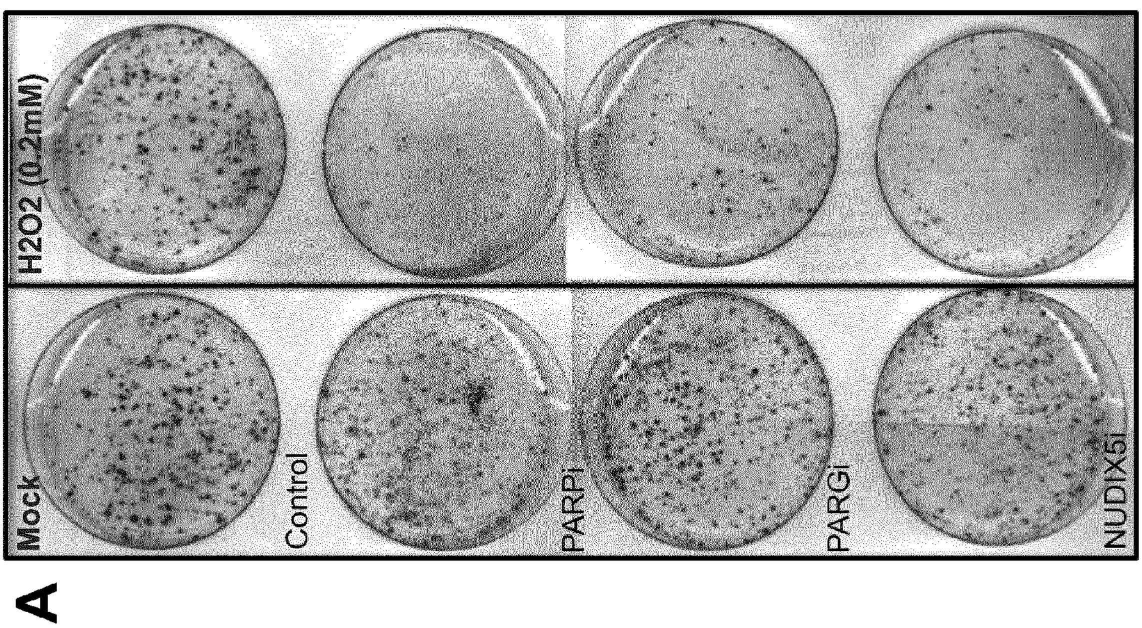
A
Mock
H2O2 (0.2mM)
Control
PARPi
PARGi
NUDIX5i

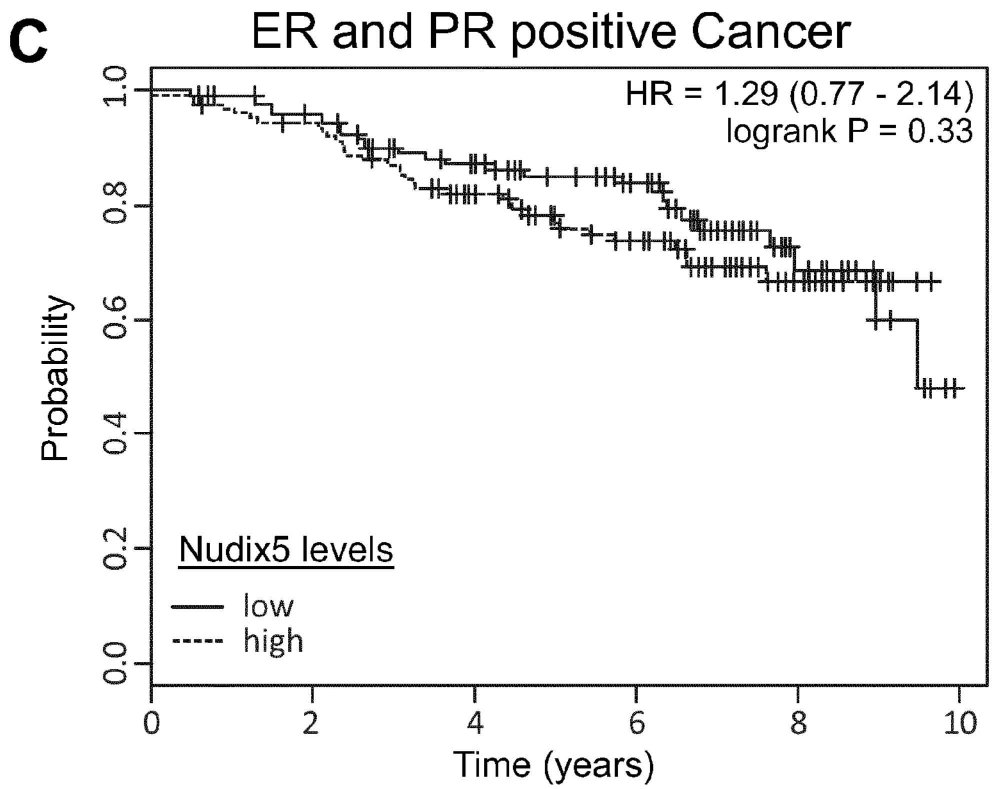
C
ER and PR positive Cancer
HR = 1.29 (0.77 - 2.14)
logrank P = 0.33
Probability
0.0
0.2
0.4
0.6
0.8
1.0
Nudix5 levels
low
high
0
2
4
6
8
10
Time (years)

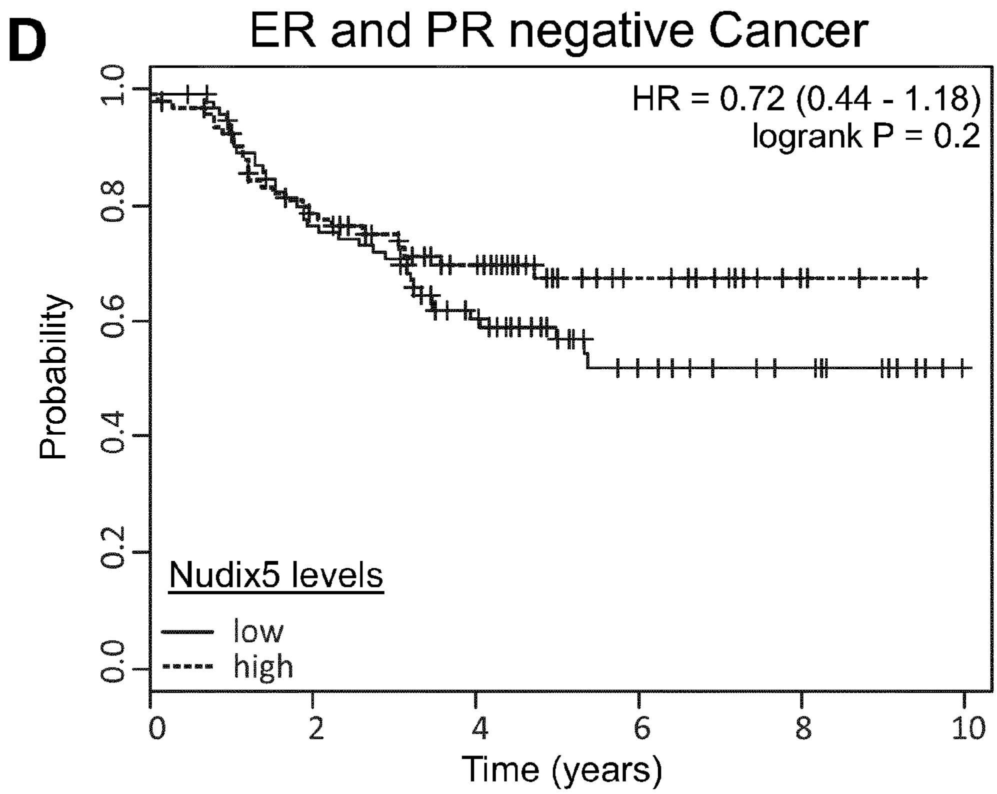
D
ER and PR negative Cancer
HR = 0.72 (0.44 - 1.18)
logrank P = 0.2
Probability
0.0
0.2
0.4
0.6
0.8
1.0
Nudix5 levels
low
high
0
2
4
6
8
10
Time (years)

FIG. 13 (cont. 1)

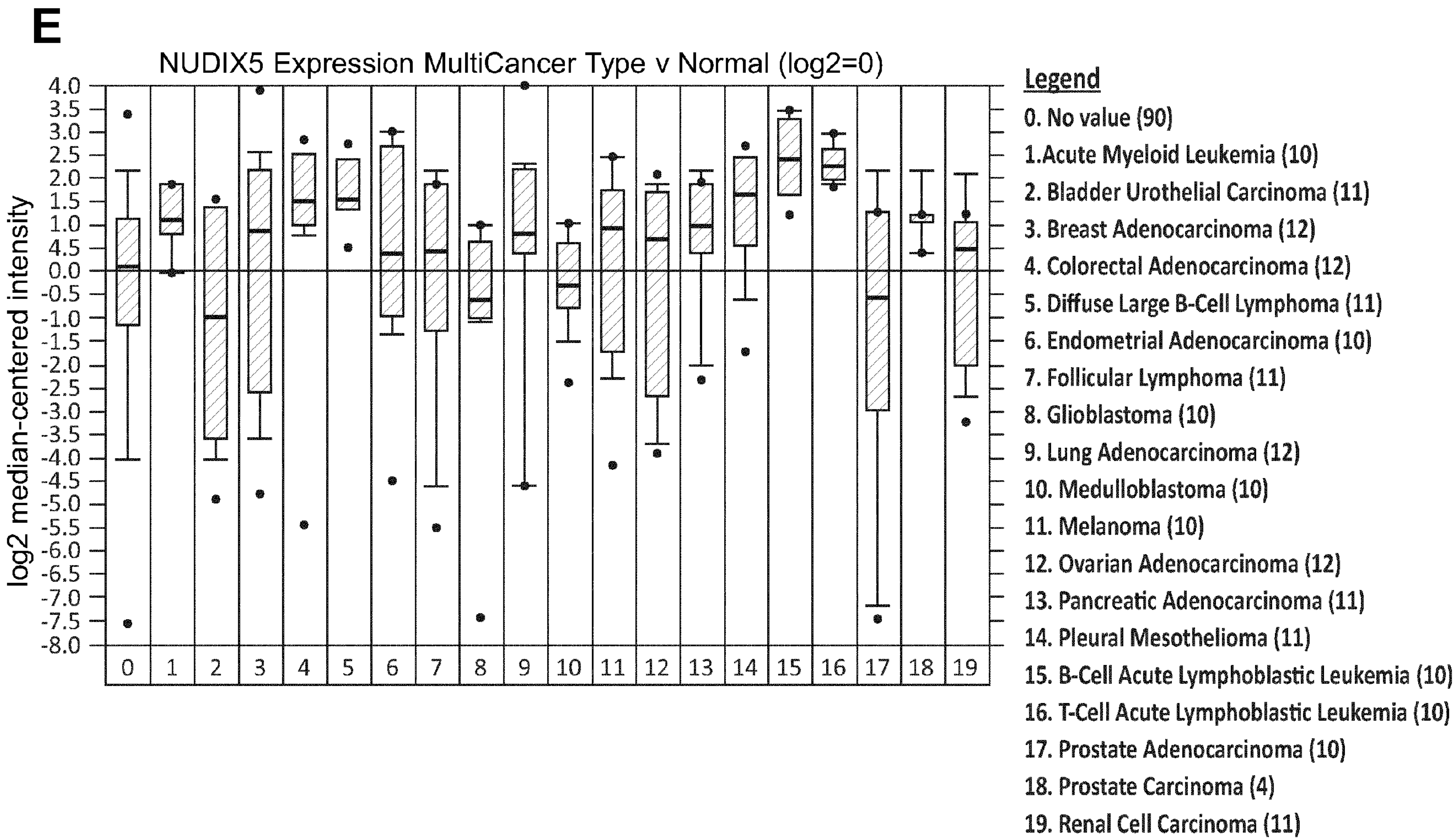
E
NUDIX5 Expression MultiCancer Type v Normal (log2=0)
log2 median-centered intensity
4.0
3.5
3.0
2.5
2.0
1.5
1.0
4.5
0.0
-0.5
-1.0
-1.5
-2.0
-2.5
-3.0
-3.5
-4.0
-4.5
-5.0
-5.5
-6.0
-6.5
-7.0
-7.5
-8.0
0 1 2 3 4 5 6 7 8 9 10 11 12 13 14 15 16 17 18 19
Legend
0. No value (90)
1.Acute Myeloid Leukemia (10)
2. Bladder Urothelial Carcinoma (11)
3. Breast Adenocarcinoma (12)
4. Colorectal Adenocarcinoma (12)
5. Diffuse Large B-Cell Lymphoma (11)
6. Endometrial Adenocarcinoma (10)
7. Follicular Lymphoma (11)
8. Glioblastoma (10)
9. Lung Adenocarcinoma (12)
10. Medulloblastoma (10)
11. Melanoma (10)
12. Ovarian Adenocarcinoma (12)
13. Pancreatic Adenocarcinoma (11)
14. Pleural Mesothelioma (11)
15. B-Cell Acute Lymphoblastic Leukemia (10)
16. T-Cell Acute Lymphoblastic Leukemia (10)
17. Prostate Adenocarcinoma (10)
18. Prostate Carcinoma (4)
19. Renal Cell Carcinoma (11)

FIG. 13 (cont. 2)

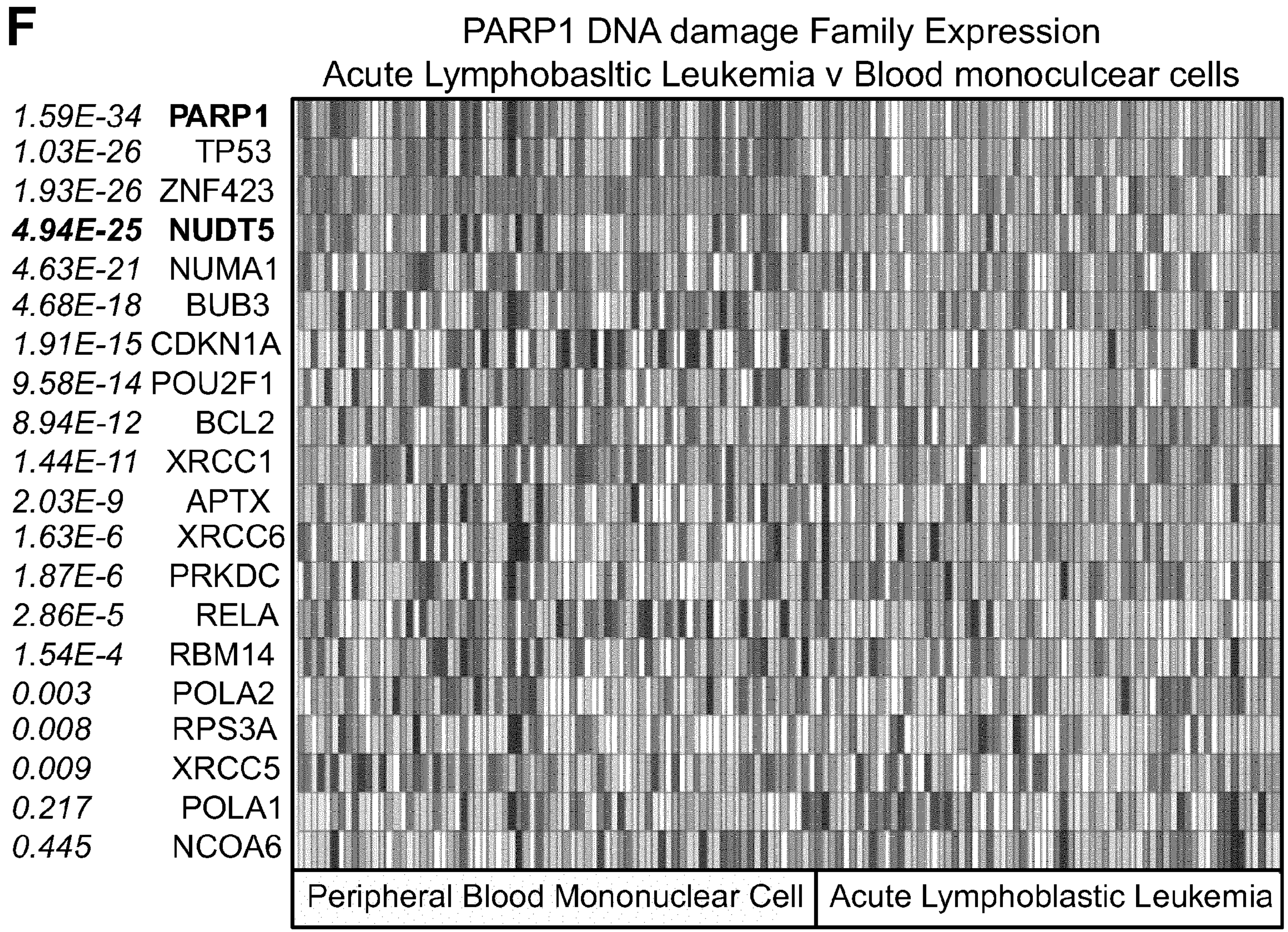
F
PARP1 DNA damage Family Expression
Acute Lymphobasltic Leukemia v Blood monoculcear cells
1.59E-34 PARP1
1.03E-26 TP53
1.93E-26 ZNF423
4.94E-25 NUDT5
4.63E-21 NUMA1
4.68E-18 BUB3
1.91E-15 CDKN1A
9.58E-14 POU2F1
8.94E-12 BCL2
1.44E-11 XRCC1
2.03E-9 APTX
1.63E-6 XRCC6
1.87E-6 PRKDC
2.86E-5 RELA
1.54E-4 RBM14
0.003 POLA2
0.008 RPS3A
0.009 XRCC5
0.217 POLA1
0.445 NCOA6
Peripheral Blood Mononuclear Cell
Acute Lymphoblastic Leukemia

FIG. 13 (cont. 3)

Europäisches Patentamt
European Patent Office
Office européen des brevets

# EUROPEAN SEARCH REPORT

Application Number
EP 14 38 2136

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X<br><br><br><br><br><br><br><br>A | L. FORMENTINI ET AL: "Poly(ADP-ribose) Catabolism Triggers AMP-dependent Mitochondrial Energy Failure", JOURNAL OF BIOLOGICAL CHEMISTRY, vol. 284, no. 26, 1 May 2009 (2009-05-01), pages 17668-17676, XP055140418, ISSN: 0021-9258, DOI: 10.1074/jbc.M109.002931<br>* abstract *<br>* page 17670, column 2, line 10 - page 17671, line 2, paragraph 1 *<br>----- | 2,5-7,14<br><br><br><br><br><br><br><br>1,3,4, 8-13,15 | INV.<br>C12N9/14<br>C12N15/55<br>C12N15/113 |
| X<br><br><br><br><br><br><br><br><br><br><br><br><br>A | TANUMA ET AL: "Evidence for a novel metabolic pathway of (ADP-ribose)N: Pyrophosphorolysis of ADP-ribose in HeLa S3 cell nuclei", BIOCHEMICAL AND BIOPHYSICAL RESEARCH COMMUNICATIONS, ACADEMIC PRESS INC. ORLANDO, FL, US, vol. 163, no. 2, 15 September 1989 (1989-09-15), pages 1047-1055, XP024769833, ISSN: 0006-291X, DOI: 10.1016/0006-291X(89)92327-9 [retrieved on 1989-09-15]<br>* the whole document *<br>-----<br>-/-- | 14<br><br><br><br><br><br><br><br><br><br><br><br><br>1-13,15 | TECHNICAL FIELDS SEARCHED (IPC)<br>C12N |

The present search report has been drawn up for all claims

2

| Place of search | Date of completion of the search | Examiner |
|---|---|---|
| The Hague | 18 September 2014 | Wiame, Ilse |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 38 2136

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LI-QUN ZHANG ET AL: "Lowered Nudix type 5 (NUDT5) expression leads to cell cycle retardation in HeLa cells", MOLECULAR AND CELLULAR BIOCHEMISTRY, KLUWER ACADEMIC PUBLISHERS, BO, vol. 363, no. 1 - 2, 27 December 2011 (2011-12-27), pages 377-384, XP035022052, ISSN: 1573-4919, DOI: 10.1007/S11010-011-1190-X * abstract * * page 378, column 1, last paragraph - column 2, paragraph 1 * ----- | 2,5-7 | |
| A | HELGE GAD ET AL: "MTH1 inhibition eradicates cancer by preventing sanitation of the dNTP pool", NATURE, vol. 508, no. 7495, 2 April 2014 (2014-04-02), pages 215-221, XP055140550, ISSN: 0028-0836, DOI: 10.1038/nature13181 * abstract * * page 218, column 1, last paragraph; figure 5a * ----- | 1-11 | TECHNICAL FIELDS SEARCHED (IPC) |
| A,D | R. H. G. WRIGHT ET AL: "CDK2-dependent activation of PARP-1 is required for hormonal gene regulation in breast cancer cells", GENES & DEVELOPMENT, vol. 26, no. 17, 1 September 2012 (2012-09-01), pages 1972-1983, XP055141153, ISSN: 0890-9369, DOI: 10.1101/gad.193193.112 * abstract * ----- | 1-11 | |

The present search report has been drawn up for all claims

2

| Place of search | Date of completion of the search | Examiner |
|---|---|---|
| The Hague | 18 September 2014 | Wiame, Ilse |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100190845 A1
- US 5262165 A
- US 5948433 A
- US 6010715 A
- US 6071531 A
- US 5082927 A
- WO 2007115376 A
- US 5622958 A
- US 20090170860 A1
- WO 2007014226 A2
- US 6335162 B
- US 4806415 A


### Non-patent literature cited in the description


- **CAO X.** *Cancer Chemotherapy Pharmacol,* 2007, vol. 59, 495-505
- **LE A. et al.** *Proc Natl Acad Sci USA,* 2010, vol. 107, 2037-2042
- **SAMBROOK, J. et al.** Molecular cloning: A Laboratory Manual. Cold Spring Harbor Laboratory Press, 2001, vol. 1-3
- **ROJO, M.G. et al.** *Folia Histochem. Cytobiol.,* 2009, vol. 47, 349-54
- **MULRANE, L. et al.** *Expert Rev. Mol. Diagn.,* 2008, vol. 8, 707-25
- **DOHERTY ; DOUDNA.** *Annu. Ref. Biophys. Biomolstruct.,* 2000, vol. 30, 457-75
- **KOHLER ; MILSTEIN et al.** *Nature,* 1975, vol. 256, 495
- **E.W. MARTIN.** Remington's Pharmaceutical Sciences. 2005
- Modified-Release Drug Delivery Technology. Marcel Dekker, Inc, 2002
- Handbook of Pharmaceutical Controlled Release Technology. Marcel Dekker, Inc, 2000
- Pharmaceutical tablet coating. **JOHNSON, J. L.** Coatings Technology Handbook. Marcel Dekker, Inc, 2001
- **CARSTENSEN, T.** Coating Tablets in Advanced Pharmaceutical Solids. Marcel Dekker, Inc, 2001, 455-468
- Nonviral Vectors for Gene Therapy. Academic Press, 1999
- Remington's Pharmaceutical Science. Mack Publishing Co, 1985
- **GOODMAN ; GILMAN'S.** The Pharmaceutical Basis of Therapeutics. Pergamon Press, 1990
- Tratado de Farmacia Galénica. **C. FAULÍ ; TRILLO.** Luzán 5, S.A. de Ediciones. 1993
- Remington's Pharmaceutical Sciences. Williams & Wilkins PA, 2000
- The Physician's Desk Reference. Medical Economics Company, Inc, 1995
- THE PHYSICIAN'S DESK REFERENCE. Drug Facts and Comparisons, Inc, 1993
- **STIRPE et al.** *Bio/Technology,* 1992, vol. 10, 405-12
- **PASTAN et al.** *Annu. Rev. Biochem.,* vol. 61, 331-54
- **BRINKMANN ; PASTAN.** *Biochim. et Biophys. Acta,* 1994, vol. 1198, 27-45
- **SANDVIG ; VAN DEURS.** *Physiol. Rev.,* 1996, vol. 76, 949-66
- **ARMSTRONG.** *J. Infect. Dis.,* 1995, vol. 171, 1042-5
- **KIM et al.** *Microbiol. Immunol.,* 1997, vol. 41, 805-8
- **SKINNER et al.** *Microb. Pathog.,* 1998, vol. 24, 117-22
- **MESRI et al.** *J. Biol. Chem.,* 1993, vol. 268, 4852-62
- **OGAWA et al.** *Science,* 1999, vol. 283, 2097-100
- **SMARDA et al.** *Folia Microbiol (Praha),* 1998, vol. 43, 563-82
- **WOOL et al.** *Trends Biochem. Sci.,* 1992, vol. 17, 266-69
- **ISLAM et al.** *J. Med. Chem.,* 1991, vol. 34, 2954-61
- **SKIBO et al.** *J. Med. Chem.,* 1994, vol. 37, 78-92
- **BEHROOZI et al.** *Biochemistry,* 1996, vol. 35, 1568-74
- **HELISSEY et al.** *Anticancer Drug Res.,* 1996, vol. 11, 527-51
- **UNNO et al.** *Chem. Pharm. Bull.,* 1997, vol. 45, 125-33
- **UNNO et al.** *Bioorg. Med. Chem.,* 1997, vol. 5, 903-19
- **UNNO et al.** *Bioorg. Med. Chem.,* 1997, vol. 5, 883-901
- **XU et al.** *Biochemistry,* 1998, vol. 37, 1890-7

- **LEE et al.** *Biochem. Mol. Biol. Int.,* 1996, vol. 40, 151-7
- **ROUTIER et al.** *Bioconjug. Chem.,* 1997, vol. 8, 789-92
- **SHAH G. et al.** *Methods in Molecular Biology.,* 2011, vol. 780, 3-34
- **PUTT KS et al.** *Anal Biochem.,* 01 March 2004, vol. 326 (1), 78-86
- **KUMMAR et al.** *MNC Medicine,* 2012, vol. 10, 25
- **DAVAR D. et al.** *Curr Med Chem,* 2012, vol. 19 (23), 3907-3921
- **FATHERS C. et al.** *Cell Cycle,* 01 March 2012, vol. 11 (5), 990-997
- **FORMENTININ L. et al.** *Br J Pharmacol.,* December 2008, vol. 155 (8), 1235-1249
- **SHIRATO M et al.** *Biochem Biophys Res Commun,* 06 April 2007, vol. 355 (2), 451-6
- **PUTT KS et al.** *Analytical Biochemistry,* 2004, vol. 326, 78-86
- **CARVALHO M. et al.** *Int J Biochem Cell Biol.,* 2007, vol. 39 (2), 298-310
- **PESSETO Z.Y. et al.** *Breast Cancer Res Treat.,* July 2012, vol. 134 (2), 511-517
- **IBRAHIM Y. et al.** *Cancer Discovery,* November 2012, vol. 2, 1036
- **BERMAN et al.** *Nucleic Acids Res,* 2000, vol. 28, 235-242
- **MODELLER.** *Protein Sci,* 2000, vol. 9, 1753-1773
- **VICENT et al.** *PLoS Genet,* 2009, vol. 5, e1000567
- **WRIGHT et al.** *Genes Dev,* 2012, vol. 26, 1972-1983
- **IMAMURA et al.** *Proceedings of the National Academy of Sciences of the United States of America,* 2009, vol. 106, 15651-15656
- **KARDASH et al.** *Nature protocols,* 2011, vol. 6, 1835-1846
- **HAGEMAN et al.** *The Journal of biological chemistry,* 2007, vol. 282, 34334-34345
- **WRIGHT et al.** *2012 Genes & Dev,* 2012, vol. 26, 1972-1983
- **MCLENNAN et al.** *Cell Mol ife Sci,* 2006, vol. 63, 123-143
- **FRIZZEL et al.** *BCR,* 2009, vol. 11, 111
- **WRIGHT et al.** *Genes & Dev,* 2012, vol. 26, 1972-1983
- **VICENT et al.** *Genes & Dev,* 2011, vol. 25, 845-862
- **DI STEFANO et al.** *Nature,* 2014, vol. 506, 235-239
- **KRISHNAKUMAR et al.** *Science,* 2008, vol. 319, 819-821
- **OEI ; ZIEGLER.** *The Journal of biological chemistry,* 2000, vol. 275, 23234-23239
- **JU et al.** *Science,* 2006, vol. 312, 1798-1802

SEQUENCE LISTING

<110> FUNDACIÓ CENTRE DE REGULACIÓ GENÒMICA

<120> COMPOSITIONS FOR TREATING CANCER

<130> P10495EP00

<160> 4

<170> PatentIn version 3.5

<210> 1
<211> 46
<212> DNA
<213> Artificial Sequence

<220>
<223> Forward primer T45D

<400> 1
tgtacgtttc actgattccc aatctctagt tttaccagta ggatcc 46

<210> 2
<211> 45
<212> DNA
<213> Artificial Sequence

<220>
<223> Reverse primer T45D

<400> 2
gatcctactg gtaaaactag agattgggaa tcagtgaaac gtaca 45

<210> 3
<211> 23
<212> DNA
<213> Artificial Sequence

<220>
<223> Sequence motive AA(N19)TT

<220>
<221> misc_feature
<222> (3)..(21)
<223> n is a, c, g, or t

<400> 3
aannnnnnnn nnnnnnnnnn ntt 23

<210> 4
<211> 23
<212> DNA
<213> Artificial Sequence

<220>
<223> Sequence motive NA(N21)

```
<220>
<221>  misc_feature
<222>  (1)..(1)
<223>  n is a, c, g, or t

<220>
<221>  misc_feature
<222>  (3)..(23)
<223>  n is a, c, g, or t

<400>  4
nannnnnnnn nnnnnnnnnn nnn                                             23
```